# EUROPEAN PATENT APPLICATION

(11) **EP 2 236 511 A2**
(43) Date of publication of application: **06.10.2010**
(21) Application number: 08864305.1
(22) Date of filing: 19.12.2008
(51) Int. Cl.: C07D 471/04, C07D 487/04, A61K 31/43, A61K 31/519, A61P 25/00

(54) **LIGANDS OF ALPHA-ADRENOCEPTORS AND OF DOPAMINE, HISTAMINE, IMIDAZOLINE AND SEROTONIN RECEPTORS AND THE USE THEREOF**

(30) Priority: 21.12.2007 RU 2007147347; 21.12.2007 RU 2007147349; 21.12.2007 RU 2007147351; 21.12.2007 RU 2007147352; 21.12.2007 RU 2007147355; 21.12.2007 RU 2007147356; 21.12.2007 RU 2007147358; 21.12.2007 RU 2007147361; 21.12.2007 RU 2007147363; 21.12.2007 RU 2007147365; 21.12.2007 RU 2007147367; 21.12.2007 RU 2007147368; 21.12.2007 RU 2007147370; 21.12.2007 RU 2007147371; 21.12.2007 RU 2007147372; 21.12.2007 RU 2007147374; 21.12.2007 RU 2007147375; 21.12.2007 RU 2007147376; 24.09.2008 RU 2008137937
(71) Applicant: Alla Chem, LLC., Karson City NV 89701 (US); Ivashchenko, Andrey Alexandrovich, Moscow, 127576 (RU)
(72) Inventor: IVASHCHENKO, Andrey Alexandrovich, Moscow 127576 (RU); IVASHCHENKO, Alexander Vasilievich, Encinitas California 92024 (US); LAVROVSKY, Yan Vadimovich, San Antonio Texas 78230 (US); MITKIN, Oleg Dmitrievich, Khimki Moskovskaya obl. 141400 (RU); SAVCHUK, Nikolay Filippovich, Moscow 117420 (RU); TKACHENKO, Sergey Yevgenievich, Chernogolovka Moskovskaya obl. 142432 (RU); OKUN, Ilya Matusovich, San Diego California 92121 (US)
(74) Representative: Henrion, Oliver
(86) International application number: PCT/RU2008/000780
(87) International publication number: WO 2009/082268

(57) **Abstract**

The invention relates to novel ligands the broad spectrum of biological activity of which includes simultaneously α-adrenoceptors, dopamine receptors, histamine receptors, imidazoline receptors and serotonin receptors, among them serotonin 5-HT₇ receptors, which are compounds of general formula **1** in the form of free bases, geometrical isomers, racemic mixtures or individual optical isomers, pharmaceutically acceptable salts and/or hydrates, 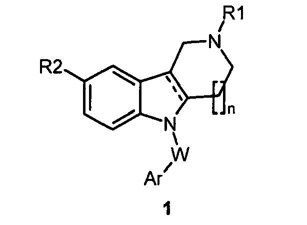 wherein: R1 is a substituent of amino group, selected from hydrogen, optionally substituted C₁-C₄ alkyl, acyl, heterocyclyl, alkoxycarbonyl, substituted sulfonyl; R2 is a substituent of cyclic system, selected from hydrogen, halogen, optionally substituted C₁-C₄ alkyl, CF₃, CN, alkoxy, alkoxycarbonyl, carboxyl, heterocyclyl or substituted sulfonyl; Ar is optionally substituted aryl not necessarily annalated with heterocyclyl, or optionally substituted aromatic heterocyclyl; W is optionally substituted (CH₂)ₘ group, optionally substituted CH=CH group, optionally substituted CH₂-CH=CH group, C≡C group, SO₂ group; n = 1, 2; m = 1, 2, 3; solid line accompanied by dotted line, i.e. (---) may represent single or double bond.

The invention also relates to active ingredients, pharmaceutical compositions comprising the said ligands as active ingredients; to novel medicaments useful for treatment of diseases and conditions of central nervous system (CNS) of humans and warm-blooded animals.

## Description

### Field of the invention

The invention relates to the novel ligands the broad spectrum of biological activity of which includes simultaneously α-adrenoceptors, dopamine receptors, histamine receptors, imidazoline receptors and serotonin receptors, among them serotonin 5-HT₇ receptors; to active ingredients, pharmaceutical compositions comprising the said ligands as active ingredients; to the novel medicines useful for treatment of diseases and conditions of the central nervous system (CNS) of humans and warm-blooded animals.

### Background of the invention

A great number of medicines and medicine candidates with a wide spectrum of receptor-specific activity for treatment of various diseases and conditions of CNS are known. For example, (3aR,10cR)-2-methyl-1,2,3,3a,4,5,6,10c-octahydropyrrolo[3,4-c]cabazole **A** is α_{1A}-adrenoceptor, α_{1B}- adrenoceptor and α_{1D}-adrenoceptors antagonist and α₂-adrenoceptor agonist. This compound is a non-opioid analgesic[WO 1999065911].

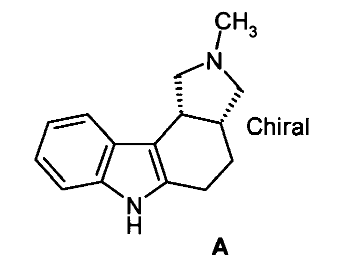

Antagonists of the central α₂-adrenoceptors intensify the releasing of noradrenaline by blocking the presynaptic α₂-receptors which serve for the negative control of this neurotransmitter releasing. Owing to their ability to increase noradrenaline concentration α₂-antagonists can be used for treatment and prophylaxis of depression. They are also potentially useful for treatment of Alzheimer's disease and memory disorders as it is known, that α₂-antagonists promote acetylcholine releasing [Tellez, et al. J. Neurochem. 1997, 68, 778-785].

Spectrum of receptor activity of Talipeksol dihydrochloride **B,** being in the market since 1996 as a medicine for Parkinson's disease treatment, along with agonistic activity to dopamine D₂ receptors and dopamine autoreceptos comprises agonistic activity to α₂-adrenoceptors [Boehringer Ingelheim, US 3804849].

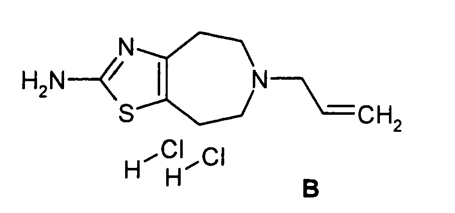

Spectrum of receptor activity of Pardoprunox **C**, which is on the phase III of clinical testing as a medicine for Parkinson's disease treatment, along with agonistic activity in relation to serotonine 5-HT_{1A} receptors and partial agonistic activity to dopamine D₂ receptors exhibits agonistic activity to α₁-adrenoceptors and antagonistic activity to α₂-adrenoceptors [Solvay, WO 2005107754].

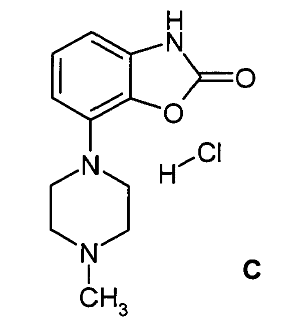

8-Chloro-11-piperazin-1-yl-5H-dibenzo[b,e][1,4]diazepine of ACADIA firm [WO 2006107948] is on the phase II of clinical testing as a medicine for Parkinson's disease treatment, its receptor-specific activity includes serotonin 5-HT_{2A} (inverse agonist), dopamine D₂ (partial agonist), dopamine D₃ (partial agonist) and muscarine M₁ (agonist) activity. γ-Carbolines of the general formula **D** display high activity in relation to serotonin receptors, such as 5-HT₁ and 5-HT₂, α₂-adrenoceptors and dopamine receptors. These compounds are useful for manufacturing of medicines for treatment of depressions, anxiety conditions and psychoses [Patent Janssen Pharm., US 6506768 B2, 2003]. 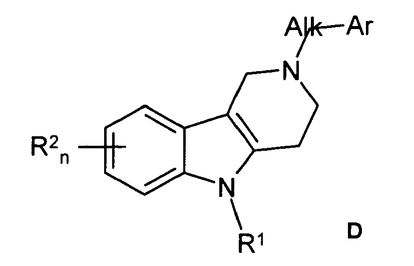 wherein R¹ represents hydrogen, optionally substituted C₁₋₆ alkyl, aryl; R²ₙ is one or more substituents which independently of each other represent halogen, hydroxy group, C₁₋₆ alkyl, C_{1-6 6} alkoxy or nitro group; n = 0, 1, 2 or 3; Alk is C₁₋₆ alkyldiyl; Ar represents optionally substituted azaheterocyclyl, optionally substituted phenyl or aryl.

The development of effective medicines for treatment of enxiety disorders (anxiolytics) has been conducting for many years. A considerable number of active ingredients, medicines and drug candidates for treatment of anxiety disorders is known the mechanism of action of which is based upon their receptor activity, selective or broad-ranging. As an example it is possible to mention the anxiolytics which are: antagonists of α_{2A}-adrenoceptors[WO 2002066484], antagonists of dopamine D₂ receptors [US 3816433, WO 2002043652, WO 2006096439, WO 2007058593], agonists of serotonin 5-HT_{1A} receptors [WO 2004002487, WO 2005094827, WO 2000006163, EP 0280269, US 5183819, EP 0170213, WO 2001034136, WO 2005115396 , WO 2004069339 , WO 2007047978, WO 2005117886, WO 2004069339, WO 2007047978], antagonists of serotonin 5-HT_{2A} receptors [US 4581171, WO 2006064754, EP 0066993, EP 0434021, WO 2001041766, WO 2001034136, EP 0374042, US 4737496, FR 2639942, WO 2007020337], agonists of serotonin 5-HT₆ receptors [WO 2003053433, WO 2005014000, WO 2003101990], antagonists of serotonin 5-HT₆ receptors [WO 2007108569, WO 2007054257, WO 2006091703, WO 2003035061, WO 2003072198, WO 2003011284, WO 2007098418].

For many years the development of effective remedies for treatment of depressions (antidepressants) has been carrying out. A large number of active ingredients, medicines and drug candidates for treatment of depressions is known the mechanism of action of which is based upon their receptor activity, selective or broad-ranging (adrenoceptor, dopamine, serotonin and others). Some known active ingredients for treatment of depression are shown in Table 1.

**Table 1. Active ingredients for treatment of depression.**

| Formula, name | Patent | Therapeutic group | Phase | mechanism of action |
|---|---|---|---|---|
| 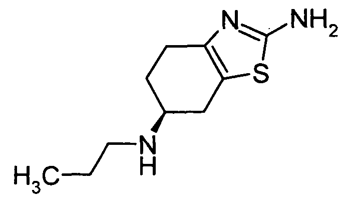 | Boehringer Ingelheim, WO 2007075095 | Antidepressant and others. | Launched - 1997 | Dopamine D₃ agonist |
| 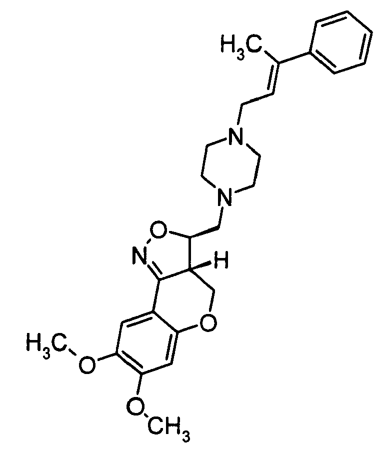 | Johnson & Johnson, WO 2002066484 | Antidepressant | Preclinical | α_{2A(2D)}-adrenoceptor antagonist, α_{2C}-adrenoceptor antagonist, inhibitor of serotonin transporter (SERT). |
| 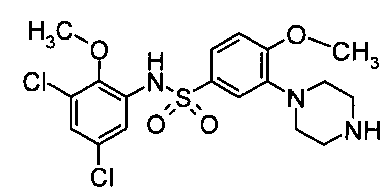 | Glaxo Smith Kline, WO 2003072198 | Antidepressant and others | Preclinica I | 5-HT₆ antagonist |
| 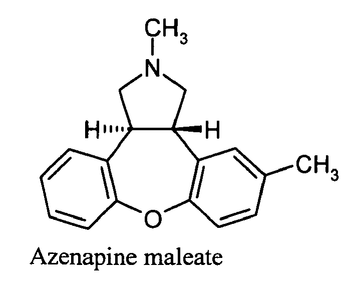 | Organone, Pfizer WO 2006040314 | Bipolar disorders and others. | Phase III | Dopamine D₁ antagonist, dopamine D₂ antagonist, 5-HT₂ antagonist |
| 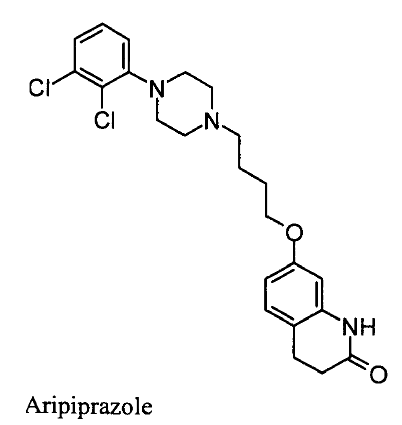 | Otsuka Pharm. WO 2004060374 | Antidepressant and others. | Launched - 2002 | Dopamine D₂ partial agonist, 5-HT_{1A} partial agonist, 5-HT₂ antagonist |
| 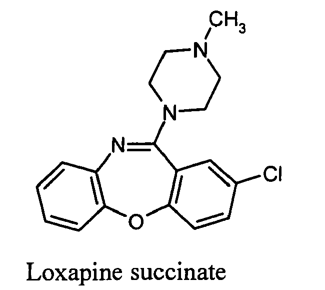 | Wyeth Pharmaceuti cals WO 2005023243 | Antidepressant and others. | Launched - 1975 | Dopamine D₂ antagonist, dopamine D₁ ligand, dopamine D₃ ligand, dopamine D₄ ligand, 5-HT₂ antagonist |

During the last years the considerable attention of investigators has been focused on the development of 5-HT₇ receptor antagonists [5-HT₇, Neurogenesis and antidepressants:a promising therapeutic axis for treating depression. Clinical and Experimental Pharmacology and Physiology (2007) 34, 546-551]. On their bases novel antidepressants have been prepared, for example, of formula E [WO 2006018308, WO 2006018309].

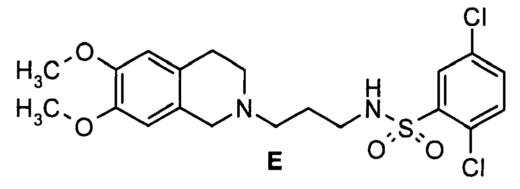

The development of effective remedies for treatment of mental disorders (antipsychotics) has been carrying out intensively for many years. A great number of active ingredients, medicines and drug candidates for treatment of mental disorders is known the mechanism of action of which is based upon their receptor activity, selective or broad-ranging f (adrenoceptor, dopamine, serotonin and others). Some known active ingredients for treatment of mental disorders are shown in Table 2.

**Table 2. Active ingredients for treatment of mental disorders.**

| Formula, name | Patent | Therapeutic group | Phase | Mechanism of action |
|---|---|---|---|---|
| 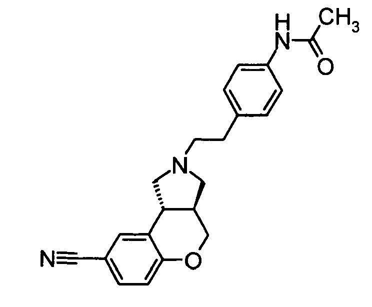 | Servier (Originator) EP 0887350 | Antipsychotic | Phase I | Dopamine D₃ antagonist |
| 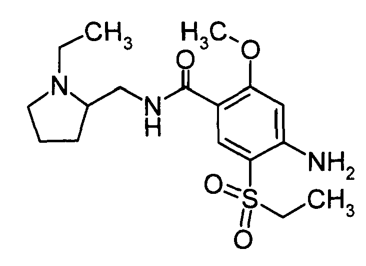 | Glaxo Smith Kline, SnofiAventis, WO 2006048560 | Antipsychotic | Launche d-1986 | Dopamine D₂ antagonist, dopamine D₃ antagonist |
| 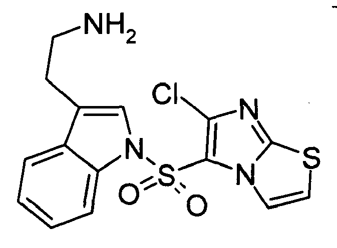 | Wyeth Pharmaceuticals WO 2003053433 | Antipsychotic and others. | Phase I | Serotonin 5-HT₆ antagonist |
| 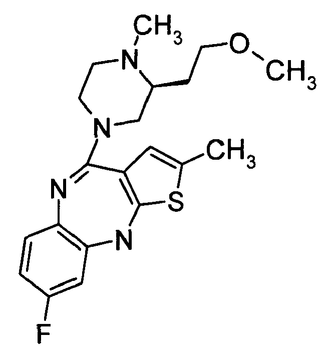 | Lilly WO 2004014895 | Antipsychotic | Preclinic al | Dopamine D₂ antagonist, serotonin 5-HT_{2A} antagonist, 5-HT₆ antagonist |
| 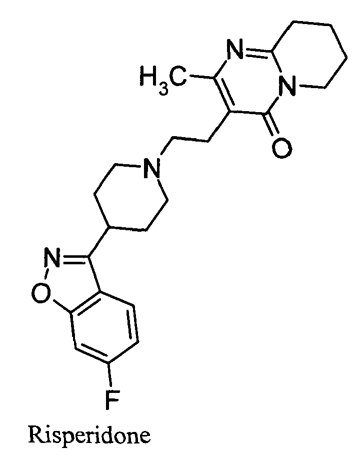 | Johnson & Johnson WO 2000023057 | Antipsychotic and others. | Launched - 1993 | Dopamine D₂ antagonist, 5-HT_{2A} antagonist |
| 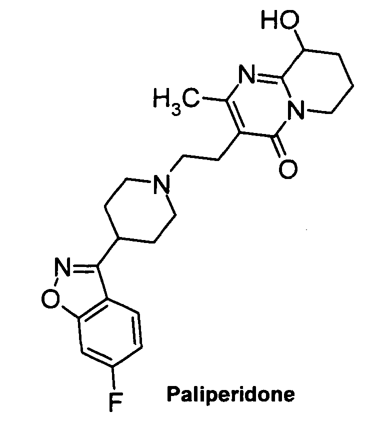 | Johnson & Johnson Janssen WO 2007044234 | Antipsychotic | Launched - 1997 | Dopamine D₂ antagonist, 5-HT₂ antagonist |
| 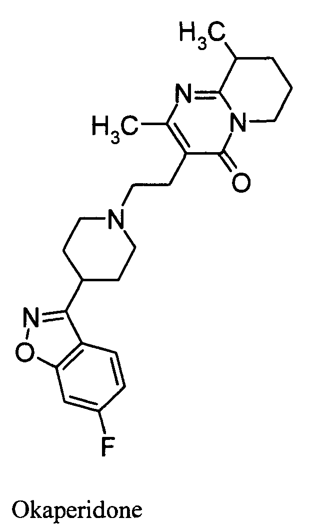 | Janssen Evotec EP 0196132, EP 0453042 | Antipsychotic | Phase II | Dopamine D₂ antagonist, 5-HT_{2A} antagonist |
| 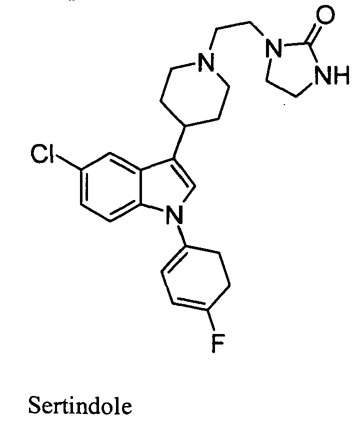 | Shionogi, Lundbeck, Abbot, WO 1997039752 | Antipsychotic | Launche d-2006 | Dopamine D₂ antagonist, 5-HT_{2A} antagonist |
| 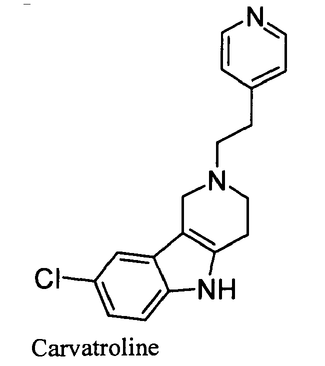 | Wyeth Pharmaceuti cals US 4636563 | Antipsychotic | Phase I | Dopamine D₂ antagonist, 5-HT_{2A} antagonist |
| 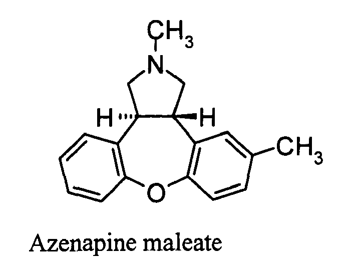 | Organon, Pfizer WO 2006040314 | Antipsychot ic and others. | Phase III | Dopamine D₁ antagonist, dopamine D₂ antagonist, 5-HT₂ antagonist |
| 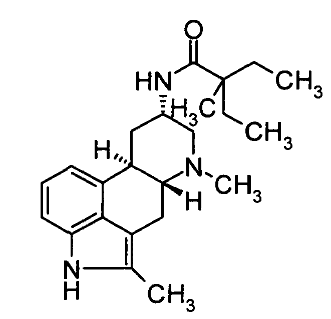 | Novartis GB 2206115 | Antipsychot ic | Phase II | Dopamine D₂ partial agonist, Dopamine D₁ antagonist, 5-HT_{1A} partial agonist |
| 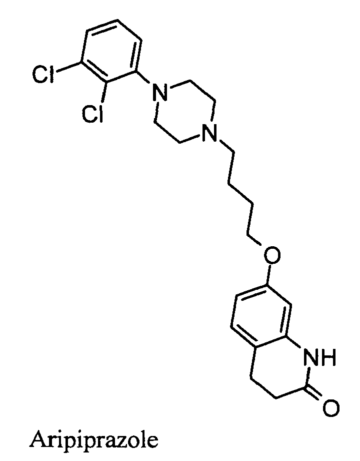 | Otsuka - et al. WO 2004060374 | Autism, Antipsychot ic and others. | Launche d-2006 | Dopamine D₂ partial agonist, 5-HT_{1A}partial agonist, 5-HT₂ antagonist |
| 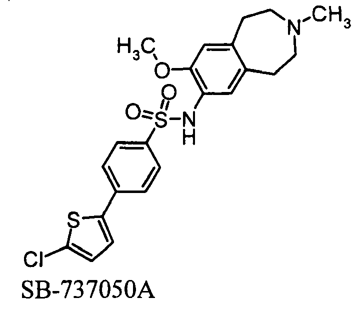 | Glaxo Group Ltd. WO 2003068752 | Antipsychot ic | Phase II | Dopamine D₂ antagonist, Dopamine D₃ antagonist, 5-HT₆ antagonist, 5-HT_{2A} antagonist, 5-HT_{2C} antagonist |
| 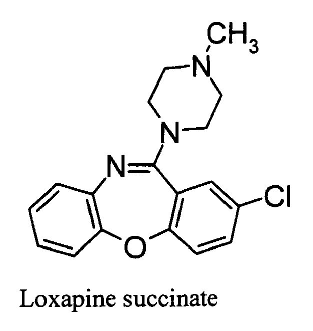 | Wyeth Pharmaceuti cals WO 2005023243 | Antipsychot ic and others. | Launched - 1975 | Dopamine D₂ antagonist, Dopamine D₁ ligand, Dopamine D₃ ligand, Dopamine D₄ ligand, 5-HT₂ antagonist |

The development of effective remedies for treatment of neurodegenerative diseases (ND) and cognitive disorders (CD) has been carrying out intensively for many years. It is known the whole number of medicines and drug candidates for treatment of ND and CD the mechanism of action of which is based upon their ability to interact with one or several receptors, among them: α_{1A}-, α_{1B}-, α_{1D}- and α_{2A}-adrenoceptors, dopamine D₁, D_{2L}, D_{2S}, D₃, D_{4.2}, D_{4.4} and D_{4.7} receptores, serotonin 5-HT_{1A}, 5-HT_{1B}, 5-HT_{2A}, 5-HT_{2B}, 5-HT_{2C}, 5-HT₆ and 5-HT₇ receptors and the others. As an example of such active ingredients dopamine D₁ receptor agonists for treatment of Parkison's disease could be mehtioned [Novartis Patent DE 3402392; DarPharma, Inc. and others. WO 2006012640; GlaxoSmithKline Patent WO 1996039136; Patent MDRNA, Inc. WO 2002024202; Patent of Maruko JP 1988033377]. One of the most perspective therapeutic axis in the treatment of Alzheimer's disease and other neurodegenerative diseases is based upon application of effective ingredients active towards serotonin 5-HT₆ receptors [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. At mammals these receptors are found in the central nervous system exclusively, and mainly in the regions of brain responsible for training and memory. [Ge'rard C., Martres M.-P., Lefe'vre K., Miquel M.-C., Verge' D., Lanfumey L., Doucet E., Hamon M., El Mestikawy S. Immuno-localisation of serotonin 5-HT6 receptor-like material in the rat central nervous system. Brain Research. 1997; 746:207-219]. Moreover, it has been shown [Dawson L.A., Nguyen H.Q., Li P. The 5-HT(6) receptor antagonist SB-271046 selectively enhances excitatory neurotransmission in the rat frontal cortex and hippocampus. Neuropsychopharmacology. 2001; 25:662-668], that 5-HT₆ receptors are the modulators of several neuromediator systems, including holinergic, noradrenergic, glutamatergic and dopaminergic.Taking into account the fundamental role of these systems in the normal cognitive processes and their disfunction at neurodegeneration, an exclusive role of 5-HT₆ receptors in the formation of normal or "pathological" memory becomes obvious. It has been shown in a large number of current publications, that blocking of 5-HT₆ receptors leads to considerable strengthening of memory consolidation in various animal models of training-storing-reproduction [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100. Riemer C., Borroni E., Levet-Trafit B., Martin J.R., Poli S., Porter R.H., Bos M. Influence of the 5-HT6 receptor on acetylcholine release in the cortex: pharmacological characterization of 4-(2-bromo-6-pyrrolidin-1-ylpyridine-4-sulfonyl)phenylamine, a potent and selective 5-HT6 receptor antagonist. J. Med. Chem. 2003; 46:1273-1276. King M.V., Woolley M.L., Topham LA., Sleight A.J., Marsden C.A., Fone K.C. 5-HT6 receptor antagonists reverse delay-dependent deficits in novel object discrimination by enhancing consolidation an effect sensitive to NMDA receptor antagonism. Neuropharmacology 2004; 47:195-204]. It has also been shown a considerable improvement of cognitive functions in aged rats in the model of Morris Water Maze under the action of 5-HT₆ receptor antagonists. [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100]. Recently more fundamental understanding of 5-HT₆ receptors role in cognitive processes and more accurate conceptions concerning possible pharmacophoric properties of their antagonists were achieved. [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. This resulted in preparation of highly affine selective ligands ("molecular tools"), and after that, drug candidates. Now a considerable number of ligands of serotonin 5-NT receptors are at different phases of clinical investigation as potential ingredients for treatment of various CNS diseases [http://integrity.prous.com].

The authors of the invention have disclosed novel ligands with extremely wide spectrum of biological activity including simultaneously α-adrenoceptors, dopamine, histamine, imidazoline and serotonin receptors, among them 5-NT₇ receptors. The authors have shown the possibility of their use as active ingredients of pharmaceutical compositions and remedies useful for prophylaxis and treatment of various pathological conditions and diseases of CNS.

The spectrum of receptor-specific agonistic and/or antagonistic activity of novel ligands includes α_{1A}, α_{1B}, α_{1D} and α_{2A} adrenoceptors, dopamine D₁, D_{2S}, D₃ and D₄.₂ receptors, histamine H₁ and H₂ receptors, imidazoline I₂ receptors, serotonin 5-HT_{1A}, 5-HT_{1B}, 5-HT_{2A}, 5-HT_{2B}, 5-HT_{2C}, 5-HT₆ and 5-HT₇ receptors

### Disclosure of the invention

In the context of the present invention, the terms are generally defined as follows:
**"Agonists"** means ligands being combined with receptors of definite type actively promote their specific signalling and by that cause the biological answer of the cell.
"**Azaheterocycle**" means an aromatic or nonaromatic mono- or polycyclic system with at least one nitrogen atom. Azaheterocycle may have one or more "cyclic system substituents".
**"Alkenyl"** means an aliphatic straight- or branched- hydrocarbon chain with 2 - 7 carbon atoms including C=C double bond. "Branched" means that one or several lower alkyl substituents, such as methyl, ethyl or propyl are attached to the straight alkenyl chain. Alkyl substituent may have one or more substituents such as: halogen, alkenyloxy, cycloalkyl, cyano; hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkoxycarbonyl or G¹G²N-, G¹G²NC(=O)-, G¹G²NSO₂-, where G¹ and G² independently of each other represent hydrogen atom, alkyl, aryl, aralkyl, heteroaralkyl, heterocyclyl or heteroaryl, or G¹ and G² together with the N-atom they are attached to, form through G¹ and G² 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, benzyloxycarbonylmethyl, and pyridylmethyloxycarbonylmethyl. The preferred alkenyl groups are ethenyl, propenyl, n-butenyl, iso-butenyl, 3-methylbuten-2-yl, n-pentenyl and cyclohexylbutenyl.
**"Alkyl"** means an aliphatic hydrocarbon straight or branched chain with 1-12 carbon atoms. Branched means that the alkyl chain has one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NC(=S)-, G¹G²NSO₂-, where G¹ and G² independently of each other represent hydrogen atom, alkyl, aryl, aralkyl, heteroaralkyl, heterocyclyl or heteroaryl, or G¹ and G² together with the N-atom, they are attached to, form through G¹ and G² 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G²NC(=O)-, annelated arylheterocyclenyl, annelated arylheterocyclyl.
**"Alkynyl"** means an aliphatic straight- or branched- hydrocarbon chain with 2 - 7 carbon atoms including C=C triple bond. "Branched" means that one or more lower alkyl substituents, such as methyl, ethyl or propyl are attached to the straight alkynyl chain. Alkynyl group may have one or more substituents, such as: halogen, alkenyloxy, cycloalkyl, cyano; hydroxy, alkoxy, carboxy, alkynyloxy, aryl, aralkoxy, aryloxy, aryloxycarbonyl, alkylthio, heteroaralkyloxy, heterocyclyl, heterocyclylalkyloxy, alkoxycarbonyl and others. The preferred alkynyl groups are ethynyl, propynyl, n-butynyl.
**"Alkoxy"** means alkyl-O-group, wherein alkyl is defined in this section. The preferred alkoxy groups are methoxy, ethoxy, n-propoxy, iso-propoxy and n-butoxy.
**"Alkoxycarbonyl"** means alkyl-O-C(=O)-group, wherein alkyl is defined in this section. The preferred alkoxycarbonyl groups are methoxycarbonyl, ethoxycarbonyl, n-butoxycarbonyl, isopropyloxycarbonyl, benzyloxycarbonyl and phenethyloxycarbonyl.
**"Amino group"**means G¹G²N-group substituted or not by "amino group substituent" G¹ and G², the meanings of which are defined in this section, for example, amino (NH₂), methylamino, diethylamino, benzylamino or phenethylamino.
**"Anxiolytic"** (tranquilizer) means a medicine intended for treatment of anxious disorders.
**"Antagonists"** mean ligands being binded with definite receptors do not cause active cellular responce. Antagonists prevent linkage of agonists with receptors and by that block the specific signalling.
**"Antidepressant"** means a medicine intended for treatment of depression.
**"Antipsychotic"** means a remedy intended for treatment of psychotic diseases.
**"Aryl"** means aromatic mono- or polycyclic system with 6 - 14 carbon atoms, preferably from 6 to 10 C-atoms. Aryl may have one or more "cyclic system substituents" of the same or different structure. The representatives of aryl groups are phenyl or naphthyl, substituted phenyl or substituted naphthyl. Aryl could be annelated with nonaromatic cyclic system or heterocycle. **"Aryloxy"** means aryl-O-group, wherein the meaning of aryl is defined in this section. The representatives of aryloxy groups are phenoxy- and 2- naphthyloxy.
**"Aryloxycarbonyl"** means aryl-O-C(=O)-group,wherein the meaning of aryl is defined in this section. The representatives of aryloxycarbonyl groups are phenoxycarbonyl and 2-naphthoxycarbonyl
**"Arylsulfinyl"** means aryl-SO-group, wherein the meaning of aryl is defined in this section.
**"Arylsulfonyl"** means aryl-SO₂-group, wherein the meaning of aryl is defined in this section.
**"Arylthio"** means aryl-S-group, wherein the meaning of aryl is defined in this section. The representatives of arylthio groups are phenylthio- and 2-naphthylthio-.
**"Aroylamino"** means aroyl-NH-group, wherein the meaning of aroyl is defined in this section.
**"Aroyl"** means aryl-C(=O)-group, wherein the meaning of aryl is defined in this section. The representatives of aroyl groups are benzoyl-, 1- and 2-naphthoyl-.
**"Acyl"** means H-C(=O)-, alkyl-C(=O)-, cycloalkyl-C(=O), heterocyclyl-C(=O)-, heterocyclylalkyl-C(=O)-, aryl-C(=O)-, arylalkyl-C(=O)-, heteroaryl-C(=O)-, heteroarylalkyl-C(=O)-groups, wherein alkyl, cycloalkyl, heterocyclyl, heterocyclylalkyl, aryl, arylalkyl, heteroaryl, heteroarylalkyl are defined in this section.
**"Acylamino"means** acyl-NH-group, wherein the meaning of acyl is defined in this section.
**«Substances causing dependence»** mean medicines, drugs, the psychoactive and physiologically active substances, capable to cause addiction and dependability, such as, alcohol, nicotine, amphetamines or sympathomimetics with analogous effect, caffeine and its analogues, cannabinoids, cocaine and its analogues, hallucinogens, inhalant substances, opiates, phencyclidine and its analogues, anabolics, somnolent and sedative medicines, and also any other natural, semisynthetic, synthetic either biotechnological substances, or mixes thereof, capable to cause addiction and dependability.
**"1,2-Vinyl radical"** means -CH=CH-group with one or more "alkyl substituents" of the same or different structure, the meanings of which are defined in this section.
**"Halogen"** means fluorine, chlorine, bromine and iodine. Preference is given to fluorine, chlorine and bromine.
**"Heteroaryl"** means aromatic monocyclic or polycyclic system with 5 - 14 carbon atoms, preferably from 5 to 10, in which one or more carbon atoms are substituted by one or more heteroatoms, such as N, S or O. The prefix "aza", "oxa" or "thia" before "heteroaryl" means that atoms N, O or S are introduced in the cyclic system. N-Atom of heteroaryl cycle could be oxidized to N-oxide. Heteroaryl may have one or more "cyclic system sustituents" of the same or different structure. The representatives of heteroaryl radicals are pyrrolyl, furanyl, thienyl, pyridyl, pyrazinyl, pyrimidinyl, isooxazolyl, isothiazolyl, tetrazolyl, oxazolyl, thiazolyl, pyrazolyl, furazanyl, thriazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzoimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl and others.
**"Heterocycle"** means aromatic or nonaromatic saturated mono- or polycyclic system including at least one heteroatom. The preferred heteroatoms are N, O or S. Heterocycle may have one or more "cyclic system substituents" of the same or different structure.
**"Heterocyclyl"** means a radical derived of heterocycle.
**"Hydrate"** means stoichiometric or nonstoichiometric compositions of the compounds or their salts with water.
**"Hydroxyalkyl"** means HO-alkyl-group, in which alkyl is defined in this section.
**"Depression"** means big depression; the incidental, chronic and recurring form of the big depression; dysthymic disorder (dysthymia); cyclotymias; affective disorder; a syndrome of seasonal affective disorder; bipolar disorder, including bipolar disorders of I and II type; and also other depressive disorders and conditions. Depression also means the depressions accompanying Alzheimer's disease, vascular dementia; disorder of the mood induced by alcohol and substances; schizoaffective disorder of depressive type; disorder of adaptation. Except for that, depression includes a depression of oncologic patients; a depression at Parkinson's disease; depressions after a myocardial infarction; depressions of fruitless women; pediatric depression; postnatal depression; the depressions accompanying somatic, neuralgic and other diseases
**"Substituent"** means a chemical radical that is attached to a scaffold (fragment), for example, "alkyl substituent", "amino group substituent", "carbamoyl substituent", and "cyclic system substituent", the meanings of which are defined in this section.
**"Alkyl group substituent"** means a substituent attached to alkyl or alkenyl group, the meanings of which are defined in this section. It is selected from hydrogen, alkyl, halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NSO₂-, where G¹ and G² independently of each other represent "amino group substituent", the meaning of which are defined in this section, for example, hydrogen, alkyl, aryl, aralkyl, heteroaralkyl, heterocyclyl or heteroaryl, or G¹ and G² together with the N-atom they are attached to via G¹ and G² form 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl, and pyridylmethyloxycarbonylmethyl. The preferred "alkyl group substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G²NC(=O)-, annelated arylheterocyclenyl, annelated arylheterocyclyl. The meanings of "alkyl group substituents" are defined in this section.
**"Amino group substituent"** means a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl.
**"Cyclic system substituent"** means a substituent attached to an aromatic or nonaromatic cyclic system selected from hydrogen, alkyl, alkenyl, alkynyl, aryl, heteroaryl, aralkyl, heteroaralkyl, hydroxy, hydroxyalkyl, alkoxy, aryloxy, acyl, aroyl, halogen, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, alkylthio, heteroaralkylthio, cycloalkyl, heterocyclyl, heterocyclenyl, amidino, G¹G²N-, G¹G²N-alkyl, G¹G²NC(=O)- or G¹G²NSO₂-, where G¹ and G² independently of each other represent hydrogen, optionally substituted alkyl, optionally substituted aryl, optionally substituted aralkyl, optionally substituted heteroaralkyl or G¹G²N-substituent wherein one of G¹ and G² could be acyl or aroyl, the meaning of the second substituent is defined above, or "cyclic system substituent" is G¹G²NC(=O)- or G¹G²NSO₂- ,where G¹ and G² together with the N-atom they are attached to via G¹ and G² form 4-7-membered heterocyclyl or hetrocyclenyl. The preferred "cyclic system substituents" are alkoxycarbonyl, alkoxy, halogen, aralkoxy, alkyl, hydroxyl, aryloxy, nitro, cyano, alkylsulfonyl, heteroaryl or G¹G²N-. If the cyclic system is saturated of partly saturated, "cyclic system substituent" may have the meanings: methylene (CH₂=), oxo (O=) or thioxo (S=).
**"Inert substituent"** ("non-interfering substituent") means low- or non-reactive radical, including, but not limited to: C₁-C₇ alkyl, C₂-C₇ alkenyl, C₂-C₇ alkynyl, C₁-C₇ alkoxy, C₇-C₁₂ aralkyl, substituted by inert substituents aralkyl, C₇-C₁₂ heterocyclylalkyl, substituted by inert substituents heterocyclylalkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₂-C₁₀ alkylsulfinyl, C₂-C₁₀ alkylsulfonyl, (CH₂)ₘ-O-(C₁-C₇ alkyl), -(CH₂)ₘ-N(C₁-C₇ alkyl)ₙ, aryl; aryl substituted by halogen or inert substituent; alkoxy group substituted by inert substituent; fluoroalkyl, aryloxyalkyl, heterocyclyl; heterocyclyl substituted by inert substituents and nitroalkyl; where m and n are varied from 1 to 7. The preferred inert substituents are C₁-C₇ alkyl, C₂-C₇ alkenyl, C₂-C₇ alkynyl, C₁-C₇ alkoxy, C₇-C₁₂ aralkyl, C₇-C₁₂ alkaryl, C₃-C₁₀ cycloalkyl, C₃-C₁₀ cycloalkenyl, C₁-C₇ alkyl substituted by inert substituents, phenyl; phenyl substituted by inert substituents; (CH₂)ₘ-O-(C₁-C₇ alkyl), -(CH₂)m-N(C₁-C₇ alkyl)ₙ, aryl; aryl substituted by inert substituents, heterocyclyl and heterocyclyl substituted by inert substituents.
**"Carboxy"** means HOC(=O)- (carboxy) group.
**"Carboxyalkyl"** means HOC(=O)-alkyl group, wherein the meaning of alkyl is defined in this section.
**"Cognitive disorders" or disorders of cognitive functions"** mean disorder (weakening) of mental abilities including attentiveness, memory, mentality, cognition, education, verbal, mental, executive and creative abilities, time and space orientation; in particular, cognitive disorders associated with Alzheimer's disease, Parkinson's and Huntington's diseases, senile dementia; age-associated memory impairment (AAMI); dysmetabolic encephalopathy; psychogenous memory impairment; amnesia; amnesic disturbances; transit global amnesia; dissociative amnesia; vascular dementia; light or mild cognitive impairment (MCI); attention deficit hyperactivity disorder (AD/HD); cognitive impairments, accompanying psychotic diseases, epilepsy, delirium, autism, psychosis, Down's syndrome, bipolar disorders and depression; AIDS-associated dementia; dementias at hypothyroidism; dementia connected with alcohol, substances causing dependability and neurotoxins; dementia accompanying neurodegenerative diseases, for example, cerebellar degeneracy and amyotrophic lateral sclerosis; cognitive disturbances connected with cerebral crisis, infectious and oncological brain diseases as well as traumatic brain injury; cognitive functions damages associated with autoimmune and endocrine diseases, and others.
**"Drug substance"** means physiologically active compound of synthetic or other (biotechnological, vegetable, animal, microbal and others) origins exhibiting pharmacological activity which is an active ingredient of pharmaceutical composition employed in production and preparation of drug medicine.
**"Drug medicine"** - is a compound (or a mixture of compounds in the form of pharmaceutical composition) in the form of tablets, capsules, injections, ointments and other ready forms intended for restoration, improvement or modification of physiological functions at humans and animals, and for treatment and prophylaxis of diseases, diagnostics, anesthesia, contraception, cosmetology and others.
**"Ligands"** (from latin *ligo*) represent chemical compounds (small molecule, peptide, protein, inorganic ion, and others) capable to interact with receptors which convert this interaction into specific signal.
**"Methylene radical"** means -CH₂-group with one or two "alkyl substituents" of the same or different structure, the meanings of which are defined in this section.
**"Neuro-degenerative diseases"** means specific conditions and diseases, accompanied by damage and primary destruction of nervous cell populations in the certain areas of the central nervous system. Neuro-degenerative diseases include but are not limited by: Alzheimer's disease; Parkinson's disease; Huntington's disease (chorea); multiocular sclerosis; cerebellar degeneracy; amyotrophic lateral sclerosis; dementias with Lewy bodies; spinal muscular atrophy; peripherical neuropathy; spongy encephalitis (Creutzfeld-Jakob Disease); AIDS dementia; multi-infract dementia; frontotemporal dementias; leukoencephalopathy (spongy degeneration of white matter); chronic neuro-degenerative diseases; cerebral crisis; ischemic, reperfussion and hypoxic brain damage; epilepsy; cerebral ischemia; glaucoma; traumatic brain injury; Down's syndrome; encephalomyelitis; meningitis; encephalitis; neuroblastoma; schizophrenia; depression. Moreover, neuro-degenerative diseases include pathological states and disorders connected with hypoxia, substance abuse, causing dependability, under neurotoxins influence; infectious and oncological brain diseases as well as neuronal damages associated with autoimmune and endocrine diseases and others.
**"Optionally substituted radical"** means a radical without or with one or more substituents.
**"Lower alkyl"** means a straight or branched alkyl radical with 1 - 4 carbon atoms.
**"Nootrops"** or **"Nootropics"** (neurometabolic stimulats) are medicines which are taken for cognition enhancing.
**"Psychotic disorders"** are diseases or diseased conditions associated with mental disturbance and/or mentality frustration. "Psychotic disorders" include affective disorders (bipolar affective disorders, big depression, hypomania, minor depression, maniacal syndrome, Cotard's syndrome, cyclothymia, schizo-affective disorders and so on), intellectual-mnestic disorders; manias (hypomania, graphomania, cleptomania, compulsive shopping, mania of persecution, pornographomania, erotomania and so on); disorder of multiple personality, amentia, alcoholomania, deliration, delirium syndrome, hallucinosis, hallucinations, lucinatory effects, homicidomania, delirium; illusion, querulous paranoiaclinical lycanthropy, macropsia, antagonistic delusion, micropsia, narcomania; anorexia nervosa, oneiroid syndrome, paranoid, paranoia, paraphrenia, pseudohallucinations, psychosis, Cotard's syndrome, schizoaffective disorder, schizotypical disorder, schizophrenia, schizo-affective psychosis disorder, schizophrenomorphic disorder, Shrebera's syndrome, Daniel Paul's syndrome), phobias (agarophobia, arachnephobia, autophobia, verminophobia, hydrosophobia, hydrophobia, demophobia, zoophobia, carcinophobia, claustrophobia, climacophobia, xenophobia, misophobia, radiophobia, photophobia; skoliephobia, scotophobia, social phobia, tetraphobia, triskaidekaphobia, erotophobia); alcoholic psychosis, alcoholic palimpsest, allotriophagy, aphasia, graphomania, dissociative fugue state, dissociative disorders; dysphorias, internet-dependences, hypochondria, hysteria, kopophobia, delirium of persecution, melancholy, misanthropy, obsession, panic attacks, Asperger's syndrome, Capgras' syndrome, Munchausen's syndrome, Retta's syndrome, Fregoly's syndrome, syndrome of attention and hyperactivity deficit, obsessive-compulsive disorder, syndrome of chronic narcotization consequences, syndrome of psychic automatism, syndrome of infantile autism, madness, taphophilia, anxiety conditions, Hikikomory's syndrome, erotographomania and so on.
**"Psychotic diseases"** are all types of schizophrenia; schizo-effective psychosis; schizo-typical disorders; schizo-affective disorders, including bipolar and depressive types; delirious disorders including reference delusion, delusion of persecution, megalomania, delusion of jealosy, erotomania, and also hypochondriacal, somatic, mixed and not differentiated delirium; short-time psychotic disorders; induced psychotic frustration; induced by substances psychotic frustration; and other psychotic disorders.
**"Substance-related disorders"** mean the disorders caused by the use of substances (substance use disorder), such as medicines, alcohol, drugs (addiction, substance dependence), pathological craving (substance craving) and abusing (substance abuse); and the disorders induced by chemical substances, such as, an intoxication (substance intoxication), abstinency or withdrawal symptoms (substance withdrawal), delirium (substance-induced delirium), dementia (substance-induced persisting dementia), amnesic disorders (substance-induced persisting amnesic disorder), psychotic disorders (substance-induced psychotic disorder), mood disorders (substance-induced mood disorder), anxiety disorders (substance-induced anxiety disorder), sexual dysfunctions (substance-induced sexual dysfunction), sleep disorders (substance-induced sleep disorder), persisting perception disorders (substance-induced persisting perception disorder, flashbacks).
**"Receptors"** (from latin *recipere*) represent biological macromolecules located either on cytoplasmic cell membrane or intracellular, capable specifically interact with restricted number of physiologically active compounds (ligands) and transform the signal about this interaction into definite cellular response.
**"Serotonin (5-hydroxytriptamine) receptors (5-HT)"** are GPCR receptors positively bound to adenylate cyclase.
**"Anxiety disorders"** means generalized (inconcrete) anxiety; acute uncontrolled anxiety; panic disorder; phobia, for example, agoraphobia (acute fear of crowded place) or social (acute fear of humiliation at presence of other people) or any other phobia (acute fear of particular subjects, animals or situations, in the form of phobia of height, of medical procedures, lifts, open space etc.); an obsessional condition (obsessive-compulsive disorder); posttraumatic stress disorder and acute stress disorder. Besides, anxiety disorders include anxiety conditions induced by alcohol or substances; anxiety under adaptation; as well as mixed forms of anxiety disorders and depression.
**"Cycloalkyl"** means nonaromatic mono- or polycyclic system with 3 - 10 carbon atoms. Cycloalkyl may have one or more "cyclic system substituents" of the same or different structure. The representatives of cycloalkyl groups are cyclopropyl, cyclobutyl, cyclopentyl, cyclohexyl, decalinyl, norbornyl, adamant-1-yl and others. Cycloalkyl could be annelated with aromatic cycle or heterocycle. The preferred "cyclic system substituents" are alkyl, aralkoxy, hydroxy or G¹G²N-group the meanings of which are defined in this section.
**"Schizophrenia"** means all known types, forms and variants of the disease, including: simple, hebephrenic, paranoid, hypertoxic (pyretic), catatonic, schizo-affective, residual or not differentiated schizophrenia and/or the forms of schizophrenia defined in classification of the American Psychiatric Association (American Psychiatric Association; in: Diagnostic and Statistical Manual of Mental Disorders, IV Edition, Washington D.C. 2000) or in the International classification (*International Statistical Classification of Diseases and Related Health Problems*) or any other known forms.
**"Pharmaceutical composition"** means a composition comprising an active ingredient (or some of them) and at least one of the components selected from a group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and the way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and the mixtures of thereof as well. Protection against the effect of microorganisms can be provided by various antibacterial and antifungal agents, such as, for example, parabens, chlorobutanole, sorbic acid, and similar compounds. A composition may also contain isotonic agents, such as, for example, sugar, sodium chloride, and similar compounds. A prolonged effect of the composition may be achieved by agents slowing down absorption of the active ingredient, for example, aluminum monostearate and gelatine. Examples of suitable carriers, solvents, diluents and delivery agents include water, ethanol, polyalcohols and their mixtures, natural oils (such as olive oil) and for injection-grade organic esters (such as ethyl oleate). Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and high molecular weight polyethylene glycol. A pharmaceutical composition for peroral, sublingval, transdermal, intramuscular, intravenous, subcutaneous, local or rectal administration of the active ingredient, alone or in combination with another active compound may be administered to humans and animals in a standard administration form, or in a mixture with traditional pharmaceutical carriers. Suitable standard administration forms include peroral forms such as tablets, gelatin capsules, pills, powders, granules, chewing-gums and peroral solutions or suspensions, for example, therapeutic kit; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. The salts could be prepared *in situ* in the processes of synthesis, isolation or purification of compounds or they could be prepared specially. In particular, bases' salts could be prepared starting from purified bases of the disclosed compounds and suitable organic or mineral acids. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of the properties of such salts is given in: Berge S.M., et al., "Pharmaceutical Salts" J.Pharm.Sci., 1977, 66: 1-19). Salts of the disclosed acids may be also prepared by the reaction of purified acids specifically with suitable bases; moreover, metal salts and amine salts may be synthesized too. Metal salts are salts of sodium, potassium, calcium, barium, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of the sufficient basicity to produce a stable salt and suitable for use for medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.
**"Fragment"** (scaffold) means a molecular frame typical for the group of compounds or compounds belonging to the combinatorial library.
**"1,2-Ethylene radical"** means -CH₂-CH₂-group containing one or more "alkyl substituents" of the same or different structure, the meanings of which are defined in this section.

The subject of the present invention is the novel ligands the spectrum of biological activity of which includes simultaneously α-adrenoceptors, dopamine receptors, histamine receptors, imidazoline receptors, and serotonin receptors, among them serotonin 5-HT₇ receptors, which are compounds of general formula **1**, in the form of free bases, geometrical isomers, racemic mixtures or individual optical isomers, and also in the form of pharmaceutically acceptable salts and/or hydrates thereof. 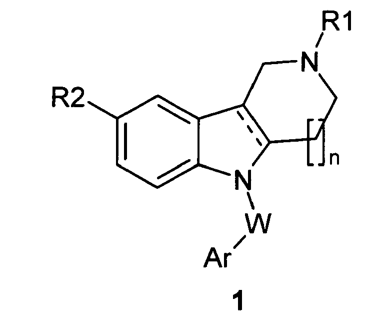 wherein:
R1 is amino group substituent selected from hydrogen, optionally substituted C₁-C₄ alkyl, acyl, heterocyclyl, alkoxycarbonyl, substituted sulfonyl;
R2 represents a substituent of cyclic system selected from hydrogen, halogen, optionally substituted C₁-C₄ alkyl, CF₃, CN, alkoxy, alkoxycarbonyl, carboxyl, heterocyclyl or substituted sulfonyl;
Ar is optionally substituted aryl or optionally substituted heterocyclyl;
W represents optionally substituted (CH₂)ₘ group, optionally substituted ethenyl group - CH=CH-, optionally substituted propenyl group -CH₂-CH=CH-, ethynyl group -C≡C-, or SO₂ group;
n = 1 or 2; and m=1, 2 or 3,
the solid line accompanied by dotted line, i.e. (---) represents single or double bond.

The spectrum of receptor-specific agonistic and/or antagonistic activity of novel ligands includes α_{1A}, α_{1B}, α_{1D} and α_{2A} adrenoceptors, dopamine D₁, D_{2S}, D₃ and D₄.₂ receptors, histamine H₁ and H₂ receptors, imidazoline I₂ receptors and serotonin 5-HT_{1A}, 5-HT_{1B}, 5-HT_{2A}, 5-HT_{2B}, 5-HT_{2C}, 5-HT₆ and 5-HT₇ receptors.

The more preferable ligands are substituted 1,2,3,4-tetrahydro-1H-pyrido[4,3-b]indoles of general formula **1.1** and 1,2,3,4,5,6-hexahydroazepino[4,3-b]indoles of general formula **1.2** and pharmaceutically acceptable salts thereof. 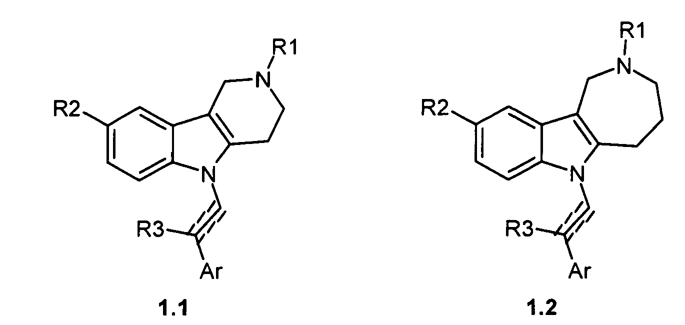 wherein: R1, R2 and Ar are as defined above; R3 is hydrogen, hydroxy group or alkyl group substituent; the solid line accompanied by two dotted lines, i.e. ( 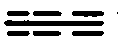 ) represents single, double or triple bond.

The more preferable ligands are substituted 1,2,3,4-tetrahydro-1H-pyrido[4,3-b]indoles of general formula **1.1.1** and 1,2,3,4,5,6-hexahydroazepino[4,3-b]indoles of general formula **1.2.1** and pharmaceutically acceptable salts thereof. 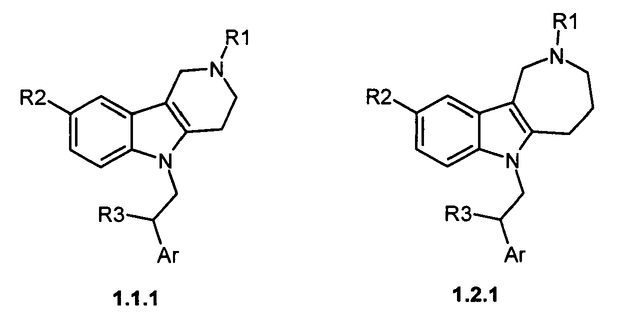 wherein: R1, R2, R3 and Ar are all as defined above.

The more preferable ligands are substituted 1,2,3,4-tetrahydro-1H-pyrido[4,3-b]indoles of general formula **1.1.2** and 1,2,3,4,5,6-hexahydroazepino[4,3-b]indoles of general formula **1.2.2** and pharmaceutically acceptable salts thereof. 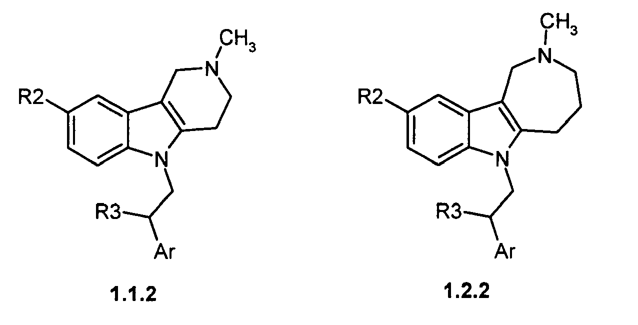 wherein: R2, R3 and Ar are as defined above.

Among the ligands of general formula **1.1.2** the more preferable ligands are substituted 1,2,3,4-tetrahydro-1H-pyrido[4,3-b]indoles of formulas **1.1.2(1), 1.1.2(2), 1.1.2(3), 1.1.2(4), 1.1.2(5)** and **1.1.2(6)** and pharmaceutically acceptable salts thereof. 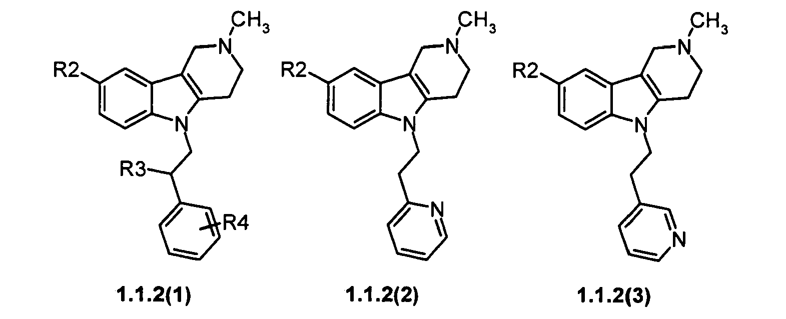 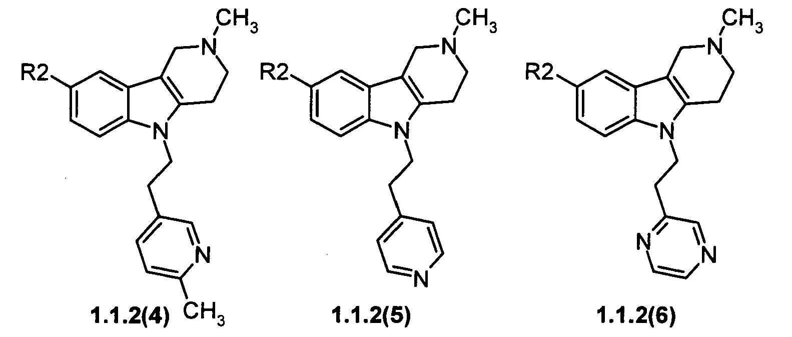 wherein: R2 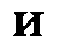 R3 are as defined above; R4 represents a substituent of cyclic system selected from hydrogen, halogen, optionally substituted C₁-C₄ alkyl, optionally substituted C₁-C₄ alkyloxy, CF₃, CN, substituted amino group.

Among the ligands of general formula **1.1.2** the more preferable ligands are 2-methyl-5-phenethyl-1,2,3,4-tetrahydro-1H-pyrido[4,3-b]indole **1.1.2(1-1),** 2,8-dimethyl-5-phenethyl-1,2,3,4-tetrahydro-1H-pyrido[4,3-b]indole **1.1.2(1-2),** 2,8-dimethyl-5-[2-(4-methylphenyl)-ethyl]-1,2,3,4-tetrahydro-1H-pyrido[4,3-b]indole **1.1.2(1-3),** 2-methyl-8-methoxy-5-phenethyl-1,2,3,4-tetrahydro-1H-pyrido[4,3-b]indole **1.1.2(1-4),** 2-methyl-5-(2-hydroxy-2-phenyl)ethyl-8-fluoro-1,2,3,4-tetrahydro-1H-pyrido[4,3-b]indole, **1.1.2(1-6),** 2-methyl-8-trifluoromethyl-5-phenethyl-1,2,3,4-tetrahydro-1H-pyrido[4,3-b]indole **1.1.2(1-7),** 2,8-dimethyl-5-(2-hydroxy-2-phenyl)ethyl-1,2,3,4-tetrahydro-1H-pyrido[4,3-b]indole **1.1.2(1-10),** 2,8-dimethyl-5-[2-(4-N,N-dimethylaminophenyl)ethyl]-1,2,3,4-tetrahydro-1H-pyrido[4,3-b]indole **1.1.3(1-11),** 2,8-dimethyl-5-[2-(4-methoxyphenyl)ethyl]-1,2,3,4-tetrahydro-1H-pyrido[4,3-b]indole **1.1.2(1-13),** 2,8-dimethyl-5-[2-(4-fluorophenyl)ethyl]-1,2,3,4-tetrahydro-1H-pyrido[4,3-b]indole **1.1.2(1-15),** 2,8-dimethyl-5-[2-(4-trifluoromethylphenyl)ethyl]-1,2,3,4-tetrahydro-1H-pyrido[4,3-b]indole **1.1.2(1-17)** and 2-methyl-5-[2-(4-methylphenyl)ethyl]-8-fluoro-1,2,3,4-tetrahydro-1H-pyrido[4,3-b]indole **1.1.2(1-20)** and pharmaceutically acceptable salts thereof.

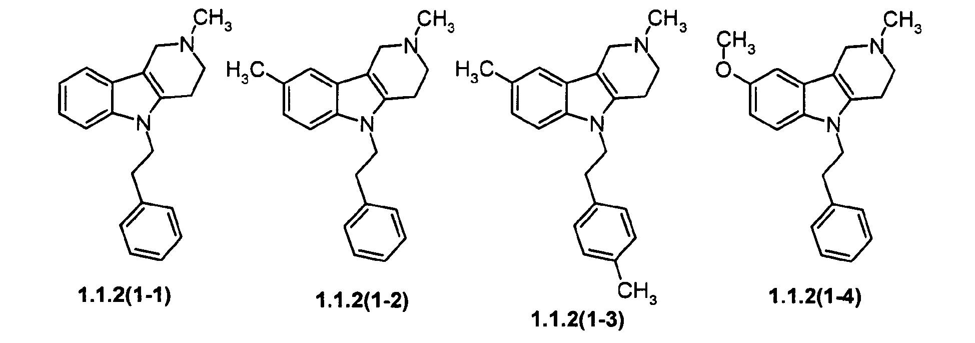

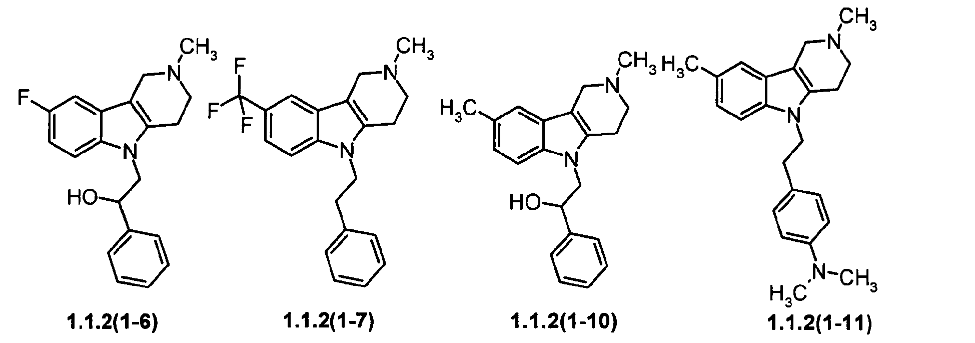

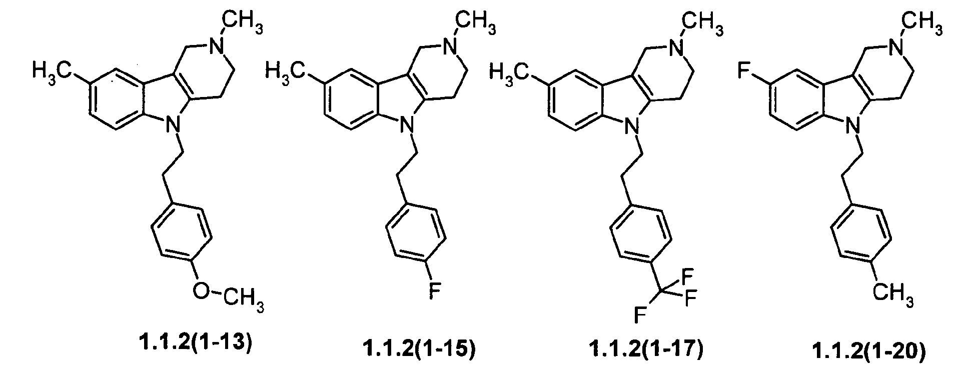

Among the ligands of general formulas **1.1.2(2), 1.1.2(3), 1.1.2(4), 1.1.2(5)** the more preferable ligands are 2,8-dimethyl-5-[2-(pyridin-2-yl)ethyl]-1,2,3,4-tetrahydro-1H-pyrido[4,3-b]indole **1.1.2(2-2),** 2,8-dimethyl-5-[2-(pyridin-3-yl)ethyl]-1,2,3,4-tetrahydro-1H-pyrido[4,3-b]indole **1.1.2(3-2),** 2,8-dimethyl-5-[2-(4-methylpyridin-3-yl)ethyl]-1,2,3,4-tetrahydro-1H-pyrido[4,3-b]indole **1.1.2(4-1)** and 2,8-dimethyl-5-[2-(pyridin-4-yl)ethyl]-1,2,3,4-tetrahydro-1H-pyrido[4,3-b]indole **1.1.2(5-1)** and pharmaceutically acceptable salts thereof.

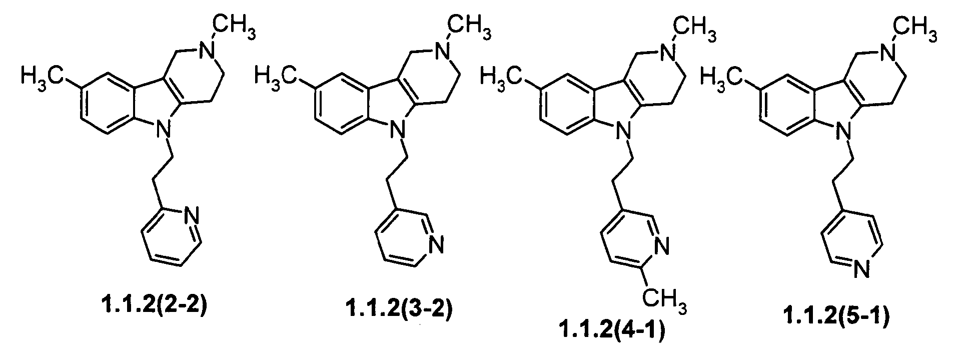

Among the ligands of the general formula **1.1.2(4)** the more preferable ligands are 2,8-dimethyl-5-[2-(4-methylpyridin-3-yl)ethyl]-1,2,3,4-tetrahydro-1H-pyrido[4,3-b]indole bis-methyl sulfonate **1.1.2(4-4)** and 2,8-dimethyl-5-[2-(4-methylpyridin-3-yl)ethyl]-1,2,3,4-tetrahydro-1H-pyrido[4,3-b]indole naphtalene-1,5-disulfonate **1.1.2(4-5).**

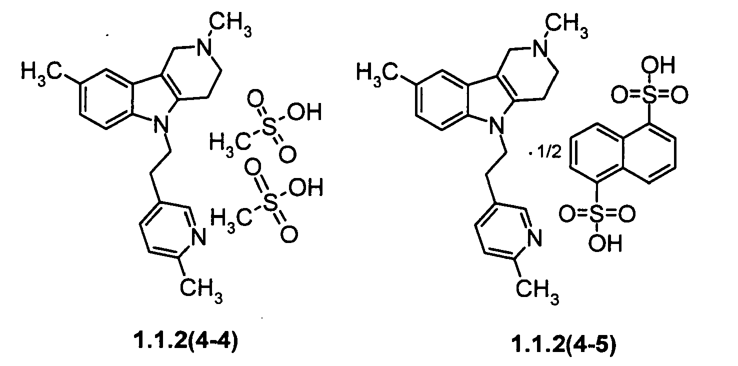

Among the ligands of general formula **1.2.2** the more preferable ligands are substituted 1,2,3,4,5,6-hexahydroazepino[4,3-b]indoles of general formulas **1.2.2(1), 1.2.2(2), 1.2.2(3), 1.2.2(4), 1.2.2(5)** and **1.2.2(6)** and pharmaceutically acceptable salts thereof. 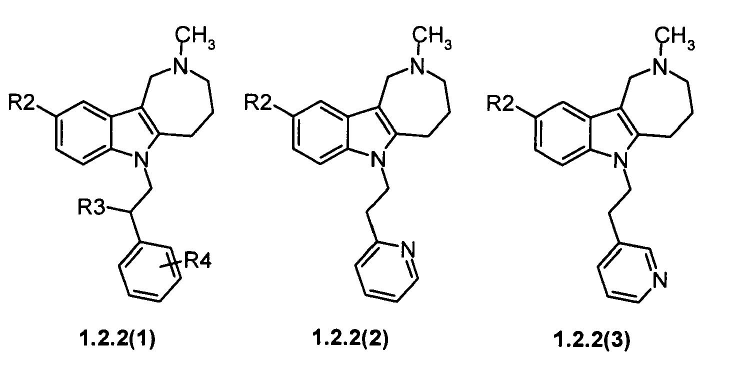 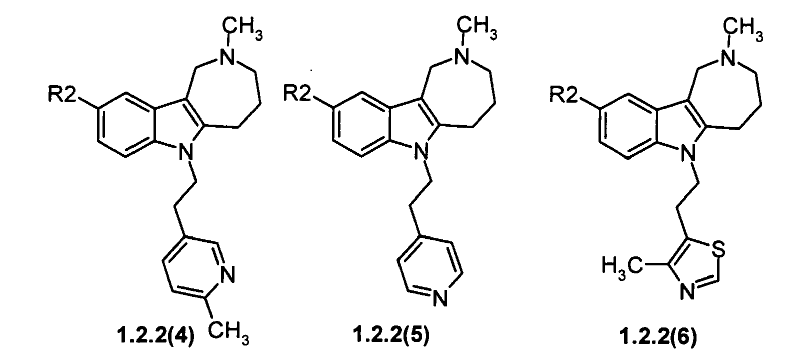 wherein: R2, R3 and R4 are all as defined above.

Among the ligands of general formula **1.2.2** the more preferable ligands are also 2-methyl-6-phenethyl-1,2,3,4,5,6-hexahydroazepino[4,3-b]indole **1.2.2(1-1),** 2,9-dimethyl-6-phenethyl-1,2,3,4,5,6-hexahydroazepino[4,3-b]indole **1.2.2(1-2),** 2-methyl-9-methoxy-6-phenethyl-1,2,3,4,5,6-hexahydroazepino[4,3-b]indole **1.2.2(1-4),** 2-methyl-6-phenethyl-9-fluoro-1,2,3,4,5,6-hexahydroazepino[4,3-b]indole **1.2.2(1-5),** 2-methyl-9-trifluoro-6-phenethyl-1,2,3,4,5,6-hexahydroazepino[4,3-b]indole **1.2.2(1-7),** 6-[(2-hydroxy-2-phenyl)ethyl]-2,9-dimethyl-1,2,3,4,5,6-hexahydroazepino[4,3-b]indole **1.2.2(1-8),** 2,9-dimethyl-6-[2-(4-methylphenyl)-ethyl]-1,2,3,4,5,6-hexahydroazepino[4,3-b]indole **1.2.2(1-9),** 2,9-dimethyl-6-[2-(4-methoxyphenyl)ethyl]-1,2,3,4,5,6-hexahydroazepino[4,3-b]indole **1.2.2(1-10),** 2,9-dimethyl-6-[2-(4-fluorophenyl)ethyl]-1,2,3,4,5,6-hexahydroazepino[4,3-b]indole **1.2.2(1-12),** 2,9-dimethyl-6-[2-(4-trifluoromethylphenyl)ethyl]-1,2,3,4,5,6-hexahydroazepino[4,3-b]indole **1.2.2(1-13)** and 2-methyl-6-[2-(4-methylphenyl)ethyl]-9-fluoro-1,2,3,4,5,6-hexahydroazepino[4,3-b]indole **1.2.2(1-15)** and pharmaceutically acceptable salts thereof.

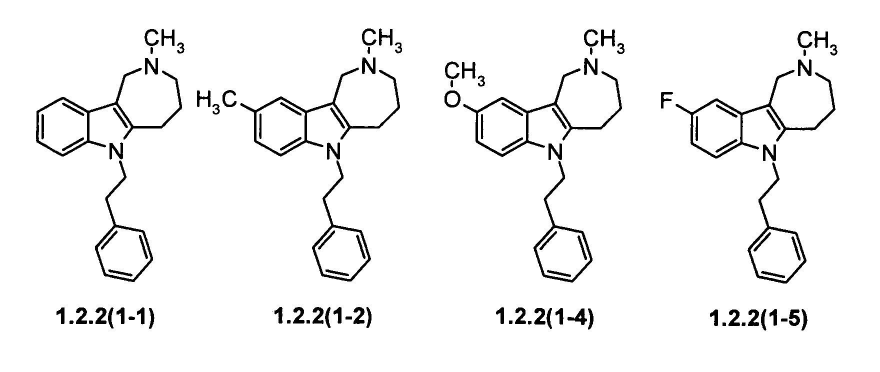

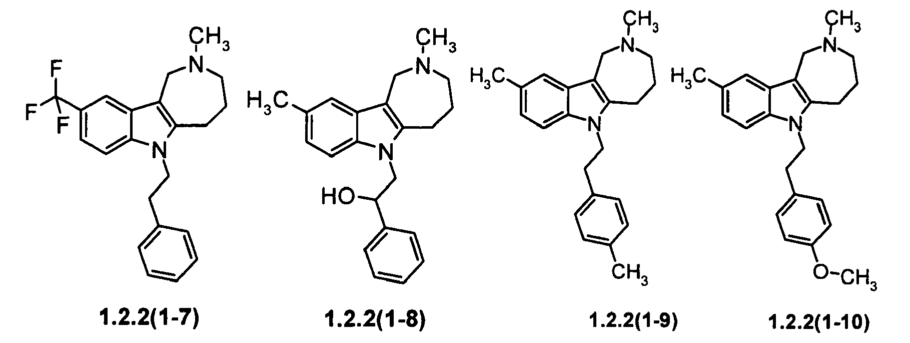

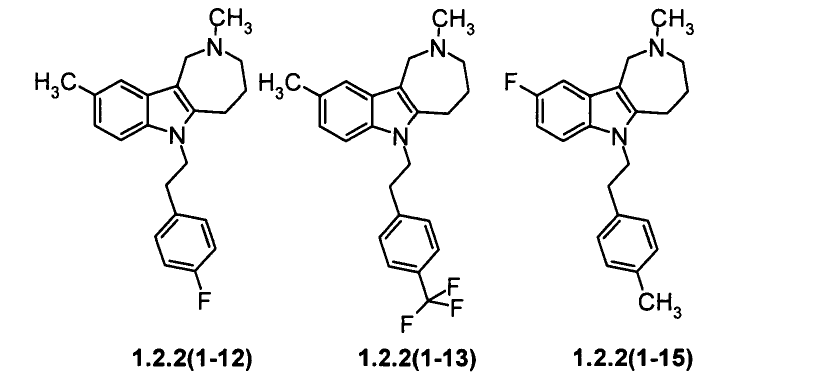

The more preferable ligands are also substituted 1,2,3,4-tetrahydro-1H-pyrido[4,3-b]indoles of general formula **1.3** and 1,2,3,4,5,6-hexahydroazepino[4,3-b]indoles of general formula **1.4** and pharmaceutically acceptable salts thereof. 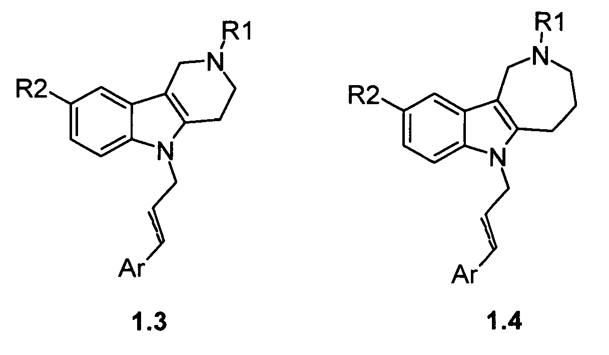 wherein: R1, R2, Ar and the solid line accompanied by dotted line, i.e. (---), are all as defined above.

The more preferable ligands are also substituted 1,2,3,4-tetrahydro-1H-pyrido[4,3-b]indoles of general formulas **1.3.1, 1.3.2, 1.3.3** and 1,2,3,4,5,6-hexahydroazepino[4,3-b]indoles of general formula **1.4.3** and pharmaceutically acceptable salts thereof. 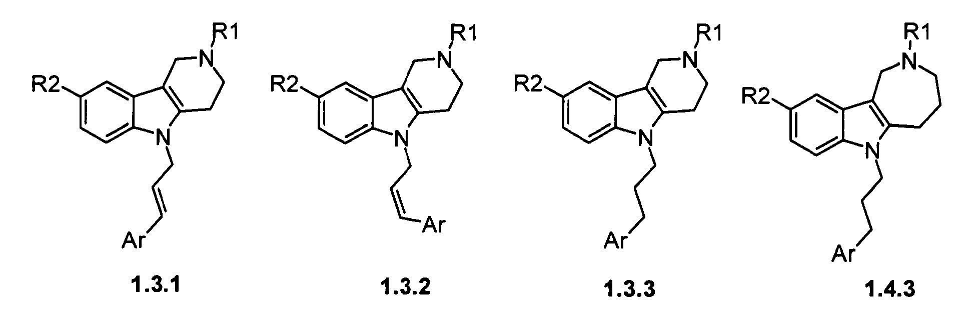 wherein: R1, R2 and Ar are all as defined above.

The more preferable ligands are also substituted 2,3,4,4a,5,9b-hexahydro-1H-pyrido[4,3-b]indoles of general formula **1.5** and 1,2,3,4,5,5a,6,10b-octahydroazepino[4,3-b]indoles of general formula **1.6** and pharmaceutically acceptable salts thereof. 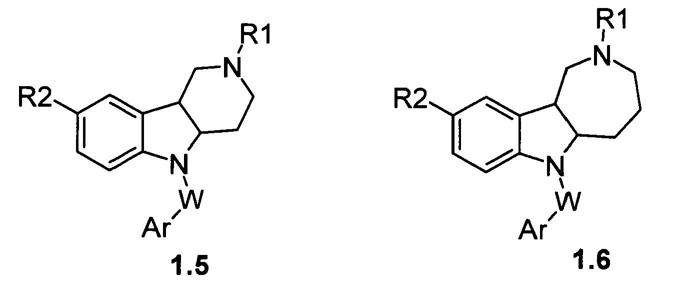 wherein: R1, R2, Ar and W are all as defined above.

The more preferable ligands are also substituted *cis*-2,3,4,4a,5,9b-hexahydro-1H-pyrido[4,3-b]indoles of general formula **1.5.1** and *cis*-1,2,3,4,5,5a,6,10b-octahydroazepino[4,3-b]indoles of general formula **1.6.1** and pharmaceutically acceptable salts thereof. 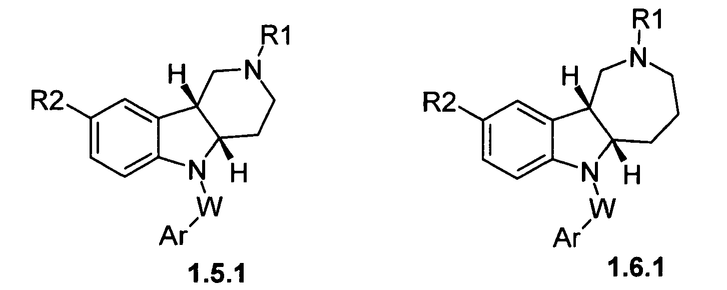 wherein: R1, R2, Ar and W are as defined above.

The more preferable ligands are also substituted *trans*-2,3,4,4a,5,9b-hexahydro-1H-pyrido[4,3-b]indoles of general formula **1.5.2** and *trans*-1,2,3,4,5,5a,6,10b-octahydroazepino[4,3-b]indoles of general formula **1.6.2** and pharmaceutically acceptable salts thereof. 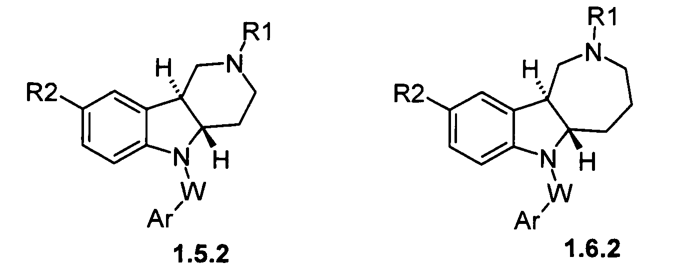 wherein: R1, R2, Ar and W are all as defined above.

The more preferable ligands are also substituted hydrogenated 1H-pyrido[4,3-b]indoles of general formula **1.7** and hydrogenated azepino[4,3-b]indoles of general formula **1.8** and pharmaceutically acceptable salts thereof. 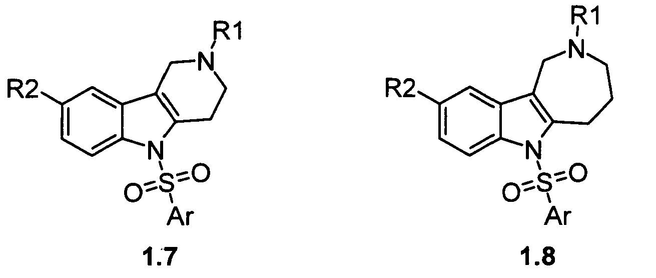 wherein: R1, R2 and Ar are all as defined above.

The more preferable ligands are also substituted hydrogenated 1H-pyrido[4,3-b]indoles of general formula **1.9** and hydrogenated azepino[4,3-b]indoles of general formula **1.10** and pharmaceutically acceptable salts thereof. 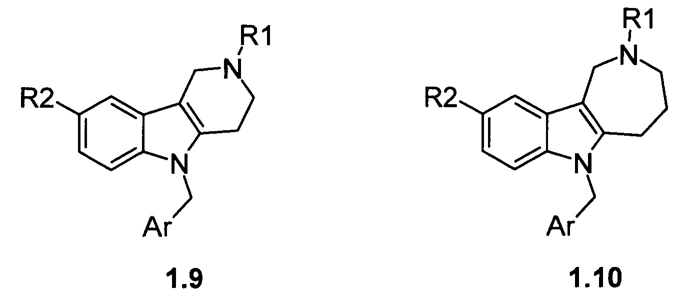 wherein: R1, R2 and Ar are all as defined above.

The more preferable ligands are also 5-benzyl-2-methyl-2,3,4,5-tetrahydro-1H-pyrido[4,3-b]indole hydrochloride **1.9(1)·HCl,** 5-benzyl-2-methyl-2,3,4,5-tetrahydro-1H-pyrido[4,3-b]indole methylsulfonate **1.9(1)·CH₃SO₃H,** bis-(5-benzyl-2-methyl-2,3,4,5-tetrahydro-1H-pyrido[4,3-b]indole) naphtalene-1,5-disulfonate **1.9(1)·1/2NDSA.**

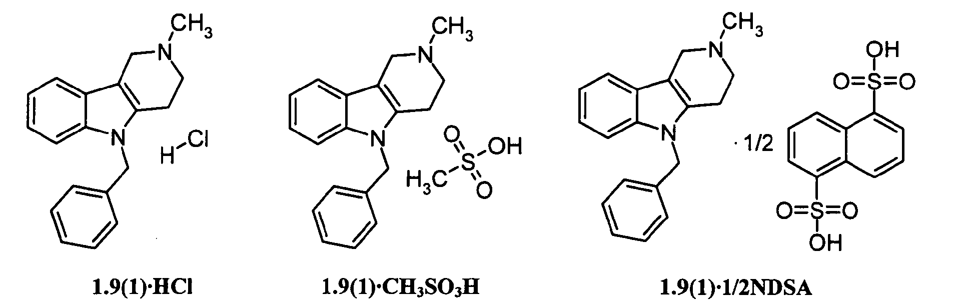

The subject of the present invention is an active ingredient for pharmaceutical compositions and medicines intended for prophylaxis and treatment of pathologic conditions and diseases of CNS pathogenesis of which is connected with hyper- or hypoactivation of alpha-adrenergic, dopamine, histamine, imidazoline, serotonin receptors, which are ligands represented by general formulas **1, 1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10** in the form of free bases and pharmaceutically acceptable salts, hydrates, solvates, geometrical isomers, racemic mixtures or individual optical isomers and pharmaceutically acceptable salts thereof.

The more preferable active ingredient is ingredient which is ligand represented by general formulas **1.1.2(1-1), 1.1.2(1-2), 1.1.2(1-3), 1.1.2(1-4), 1.1.2(1-6), 1.1.2(1-7), 1.1.2(1-10), 1.1.2(1-11), 1.1.2(1-13), 1.1.2(1-15), 1.1.2(1-17), 1.1.2(1-20), 1.1.2(2-2), 1.1.2(3-2), 1.1.2(4-1), 1.1.2(4-4), 1.1.2(4-5), 1.1.2(5-1), 1.2.2(1-1), 1.2.2(1-2), 1.2.2(1-4), 1.2.2(1-5), 1.2.2(1-7), 1.2.2(1-8), 1.2.2(1-9), 1.2.2(1-10), 1.2.2(1-12), 1.2.2(1-13), 1.2.2(1-15), 1.9(1)** or pharmaceutically acceptable salts thereof.

The subject of the present invention is pharmaceutical composition with a broad spectrum of biological activity towards various receptors, including alpha-adrenergic, dopamine, histamine, imidazoline, serotonin receptors, comprising effective amount of active ingredient of general formulas **1, 1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10** in the form of free bases and pharmaceutically acceptable salts, hydrates, solvates, geometrical isomers, racemic mixtures or individual optical isomers and/or hydrates thereof.

The more preferable pharmaceutical composition is pharmaceutical composition with a broad spectrum of receptor-specific activity for prophylaxis and treatment of various conditions and diseases of CNS in humans and animals comprising pharmaceutically effective amount of active ingredient of following formulas **1.1.2(1-1), 1.1.2(1-2), 1.1.2(1-3), 1.1.2(1-4), 1.1.2(1-6), 1.1.2(1-7), 1.1.2(1-10), 1.1.2(1-11), 1.1.2(1-13), 1.1.2(1-15), 1.1.2(1-17), 1.1.2(1-20), 1.1.2(2-2), 1.1.2(3-2), 1.1.2(4-1), 1.1.2(4-4), 1.1.2(4-5), 1.1.2(5-1), 1.2.2(1-1), 1.2.2(1-2), 1.2.2(1-4), 1.2.2(1-5), 1.2.2(1-7), 1.2.2(1-8), 1.2.2(1-9), 1.2.2(1-10), 1.2.2(1-12), 1.2.2(1-13), 1.2.2(1-15)** or pharmaceutically acceptable salts and/or hydrates thereof.

Pharmaceutical compositions may include pharmaceutically acceptable excipients. Pharmaceutically acceptable excipients mean diluents, auxiliary agents and/or carriers applied in the sphere of pharmaceutics. According to the invention pharmaceutical composition together with active ingredient of general formula **1** may include other active ingredients provided that they do not give rise to undesirable effects, for example, allergic reactions.

If needed, according to the present invention pharmaceutical compositions can be used in clinical practice in various forms prepared by mixing the compositions with traditional pharmaceutical carries, for example, peroral forms (such as, tablets, gelatinous capsules, pills, solutions or suspensions); forms for injections (such as, solutions or suspensions for injections, or a dry powder for injections which requires only addition of water for injections before utilization); local forms (such as, ointments or solutions).

The carriers used in pharmaceutical compositions, according to the present invention, represent carriers which are applied in the sphere of pharmaceutics for preparation of the commonly used forms including: binding agents, greasing agents, disintegrators, solvents, diluents, stabilizers, suspending agents, colorless agents, taste flavors are used for peroral forms; antiseptic agents, solubilizers, stabilizers are used in forms for injections, base materials, diluents, greasing agents, antiseptic agents are used in local forms.

The subject of the present invention is also method for preparation of pharmaceutical composition by mixing active ingredient with exicipient and/or solvent, **characterized in that** as active ingredient at least one ligand of general formula **1**, either racemate, or optical isomer, or geometrical isomers or acceptable salt and/or hydrate thereof in pharmacologically effective amount is used.

The subject of the present invention is also medicine in the form of tablet, capsule, intravenous, intranasal and transdermal formulation, muscular injection, syrup, suppository, aerosol, or pessary placed in pharmaceutically acceptable packing intended for treatment of CNS diseases and neurological disorders connected with hyperactivation (or hypoactivation) of alpha-adrenergic, dopamine, histamine, imidazoline and serotonin receptors.

Pathologic conditions and diseases of CNS pathogenesis of which is connected with hyper- or hypoactivation of alpha-adrenergic, dopamine, histamine, imidazoline, serotonin receptors, responsible for functional status of CNS are included, but not limited by hyperkinetic disorders, among them: imbecility, anxiety disorders, psychotic disorders and schizophrenia, depression, cognitive disorders or cognitive disfunctions, including Alzheimer's disease and Hungtigton's disease, neurodegenerative diseases, substance-related dependability, substance-related disorders, and others.

The preferred medicines include at least one active ingredient of general formulas **1,1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10** or recemate, or optical isomer, or geometrical isomer, or pharmaceutically acceptable salt and/or hydrate thereof.

The preferred medicines include at least one active ingredient of general formulas **1.1.2(1-1), 1.1.2(1-2), 1.1.2(1-3), 1.1.2(1-4), 1.1.2(1-6), 1.1.2(1-7), 1.1.2(1-10), 1.1.2(1-11), 1.1.2(1-13), 1.1.2(1-15), 1.1.2(1-17), 1.1.2(1-20), 1.1.2(2-2), 1.1.2(3-2), 1.1.2(4-1), 1.1.2(4-4), 1.1.2(4-5), 1.1.2(5-1), 1.2.2(1-1), 1.2.2(1-2), 1.2.2(1-4), 1.2.2(1-5), 1.2.2(1-7), 1.2.2(1-8), 1.2.2(1-9), 1.2.2(1-10), 1.2.2(1-12), 1.2.2(1-13), 1.2.2(1-15)** or pharmaceutically acceptable salt and/or hydrate thereof.

The subject of the present invention is also method for treating and prophylaxis of various CNS diseases and conditions, connected with hyperactivation (or hypoactivation) of alpha-adrenergic, dopamine, histamine, imidazoline and serotonin receptors in humans and other mammals, needed of such treatment, which is consisted in administering to the said mammals effective amount of active ingredient of general formulas **1, 1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10** or racemate, or optical isomer, or geometrical isomer, of pharmaceutically acceptable salt and/or hydrate, or pharmaceutical composition, or medicine comprising active ingredient mentioned above.

Besides, the clinical dose of pharmaceutical composition or medicine comprising ligands of general formula **1,** may be corrected depending on: therapeutic efficiency and bio-accessibility of active ingredients in organism, rate of their exchange and removal from organism, and also age, gender, and severity of the patient's symptoms. Thus, the daily intake for adults normally being 10∼500 mg, preferably 50∼300 mg. Accordingly, the above effective doses are to be taken into consideration while preparing medicament of the present invention from pharmaceutical composition in the form of dose units, each dose unit of the preparation containing 10∼500 mg of the compound of general formula **1,** preferably 50-300 mg. Following the instructions of a physitian or pharmacist, the preparations may be taken several times over specified periods of time (preferably, from one to six times).

The subject of the present invention is also 1-aryl-2-(1,2,3,4-tetrahydro-1H-pyrido[4,3-b]indol-5-yl)-ethanoles of general formula **1.1.1** and 1-aryl-2-(2,3,4,5-tetrahydro-1H-azepino[4,3-b]indol-6-yl)-ethanoles of general formula **1.2.1** 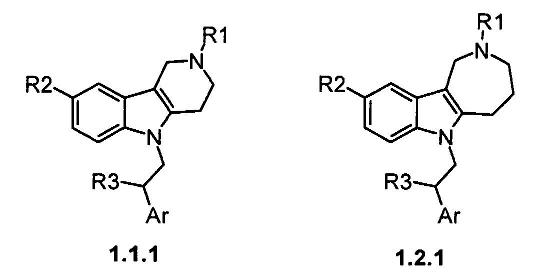 wherein: **R¹, R²,** and **Ar** are all as defined above, **R³ =** OH.

The subject of the present invention is also 2,8-dimethyl-5-[2-(4-methylpyridin-3-yl)ethyl]-1,2,3,4-tetrahydro-1H-pyrido[4,3-b]indole bis-methylsulfonate of the formula 1.1.2(4-**4)·2CH₃SO₃H** and 2,8-dimethyl-5-[2-(4-methylpyridin-3-yl)ethyl]-1,2,3,4-tetrahydro-1H-pyrido[4,3-b]indole naphtalene-1,5-disulfonate of the formula **1.1.2(4-5)·1/2NDSA.**

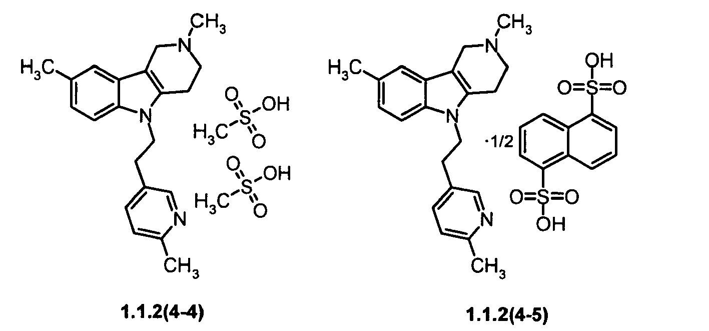

The subject of the present invention are also substituted 1,2,3,4-tetrahydro-1H-pyrido[4,3-b]indoles of general formula **1.3** and 1,2,3,4,5,6-hexahydroazepino[4,3-b]indoles of general formula **1.4** and pharmaceutically acceptable salts thereof, 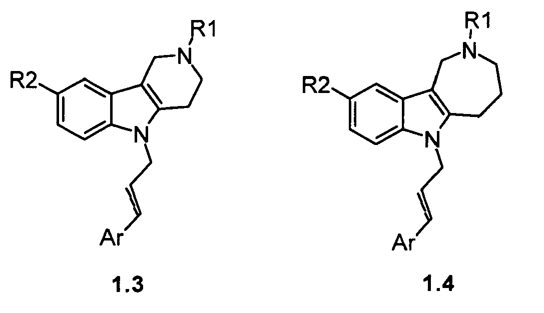 wherein: R1, R2 and Ar are all as defined above; the solid line accompanied by dotted line, i.e. (---) is single or double bond.

The more preferable substituted 1,2,3,4-tetrahydro-1H-pyrido[4,3-b]indoles and 1,2,3,4,5,6-hexahydroazepino[4,3-b]indoles are compounds of general formulas **1.3.1, 1.3.2, 1.3.3** and compounds of general formula **1.4.3** and pharmaceutically acceptable salts thereof, 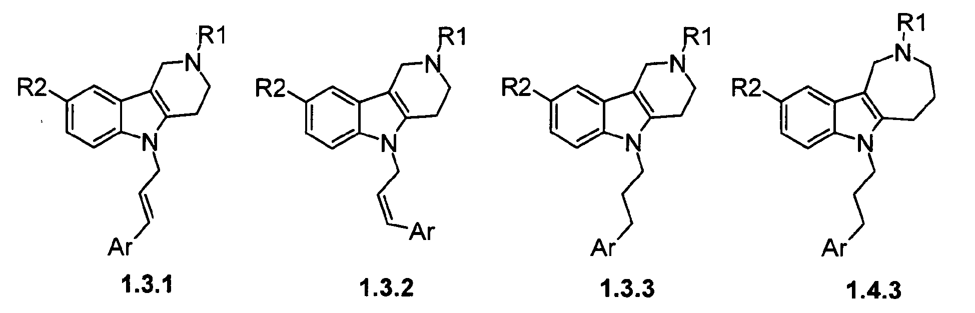 wherein: R1, R2 and Ar are all as defined above.

The subject of the present invention are substituted 6-sulfonyl-azepino[4,3-b]indoles of general formula **1.8** and pharmaceutically acceptable salts thereof, 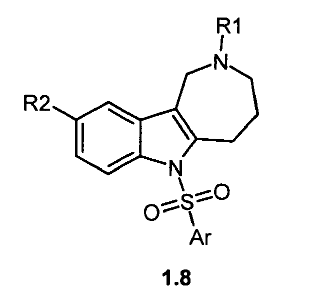 wherein: R1, R2 and Ar are all as defined above.

The subject of the present invention is also 5-benzyl-2-methyl-2,3,4,5-tetrahydro-1H-pyrido[4,3-b]indole methylsulfonate **1.9(1)·CH₃SO₃H.**

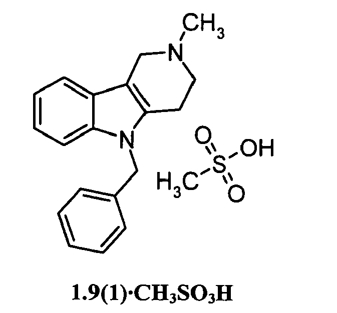

The ligands of general formula **1** biological activity of which includes simultaneously alpha-adrenergic, dopamine, histamine, imidazoline, serotonin receptors are represented in Table 3. These ligands are novel or known compounds. Synthesis of the known compounds of general formula 1 is described in many publications, for example, [Horlein, Ulrich; Hecht, Gerhard. Med. u. Chem., Abhandl. med.-chem. Forschungsstatten Farbenfabriken Bayer (1956), 5 267-80. Kost, J.Gen.Chem. USSR (Engl.Transl.), v. 33, 1963, p. 3538. Mashkovsky M.D. Medicines. Ed. 13. Kharkov: Torsing, 1998. v.l. p. 280-281. Bull Exp Biol Med. 2000, 129(6), 544-546, US 6187785 (2001), JP 09216882 (1997), RU 2140417 (1999), US 3502688 (1972), RU 2334747 (2008), WO2008/024029 (2008)].

The novel compounds of general formula **1** were prepared according to the methods known in the art described, for example, in the publications mentioned above.

Substituted 1,2,3,4-tetrahydro-1H-pyrido[4,3-b]indoles of general formula **1.1.1 (R3** = **H**) and 1,2,3,4,5,6-hexahydroazepino[4,3-b]indoles of general formula **1.2.1 (R3** = **H)** were prepared by catalytic hydrogenation of corresponding vinyl derivatives of general formula 2.

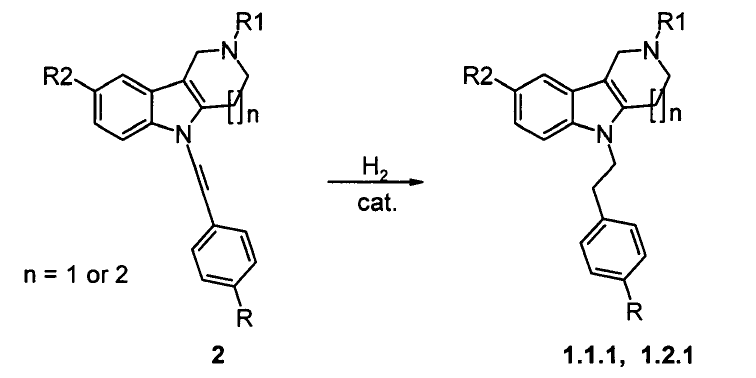

The subject of the present invention is method for preparation of hydroxy derivatives of 1,2,3,4-tetrahydro-1H-pyrido[4,3-b]indoles of general formula **1.1.1 (R3 = OH)** and 01,2,3,4,5,6-hexahydroazepino[4,3-b]indoles of general formula **1.2.1 (R3** = **OH)** consisting in interaction of compounds **3** with corresponding epoxides **4** in the presence of alkali.

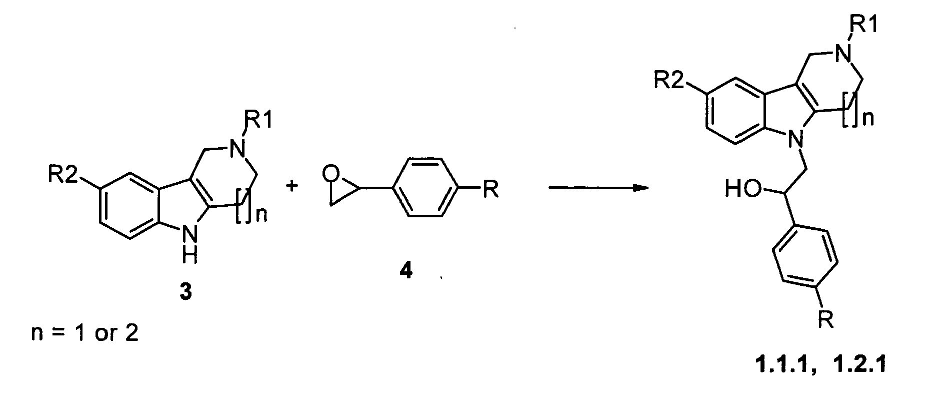

The subject of the present invention is also method for preparation of 2,8-dimethyl-5-[2-(4-methylpyridin-3-yl)ethyl]-1,2,3,4-tetrahydro-1H-pyrido[4,3-b]indole bis-methylsulfonate of formula **1.1.2(4-4)·2CH₃SO₃H** by interaction of 2,8-dimethyl-5-[2-(4-methylpyridin-3-yl)ethyl]-1,2,3,4-tetrahydro-1H-pyrido[4,3-b]indole of formula **1.1.2(4-1)** with methanesulfonic acid of formula **5.**

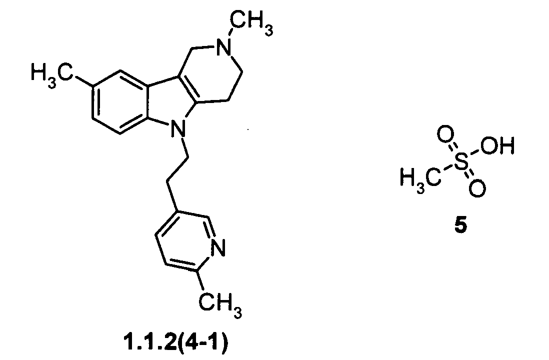

The subject of the present invention is also method for preparation of 2,8-dimethyl-5-[2-(4-methylpyridin-3-yl)ethyl]-1,2,3,4-tetrahydro-1H-pyrido[4,3-b]indole naphthalene-1,5-disulfonate of formula **1.1.2(4-5)·1/2NDSA** by interaction of 2,8-dimethyl-5-[2-(4-methylpyridin-3-yl)ethyl]-1,2,3,4-tetrahydro-1H-pyrido[4,3-b]indole dihydrochloride of formula **1.1.2(4-1)·2HCl** with naphthalene-1,5-disulfonate of formula **6.**

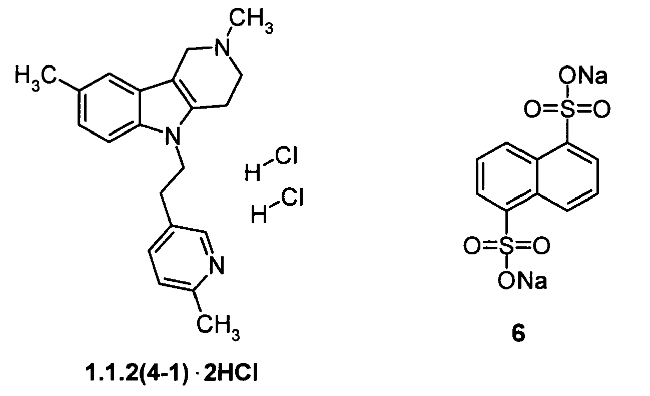

The subject of the present invention is also method for preparation of substituted 1,2,3,4-tetrahydro-1H-pyrido[4,3-b]indoles of general formula **1.3** and 1,2,3,4,5,6-hexahydroazepino[4,3-b]indoles of general formula **1.4** consisting in interaction of compounds **3** with alkenylchlorides **7** and subsequent reduction of obtained propylenes **1.3.1, 1.3.2, 1.4.1** and **1.4.2** with the formation of corresponding substituted propanes **1.3.3** and **1.4.3.**

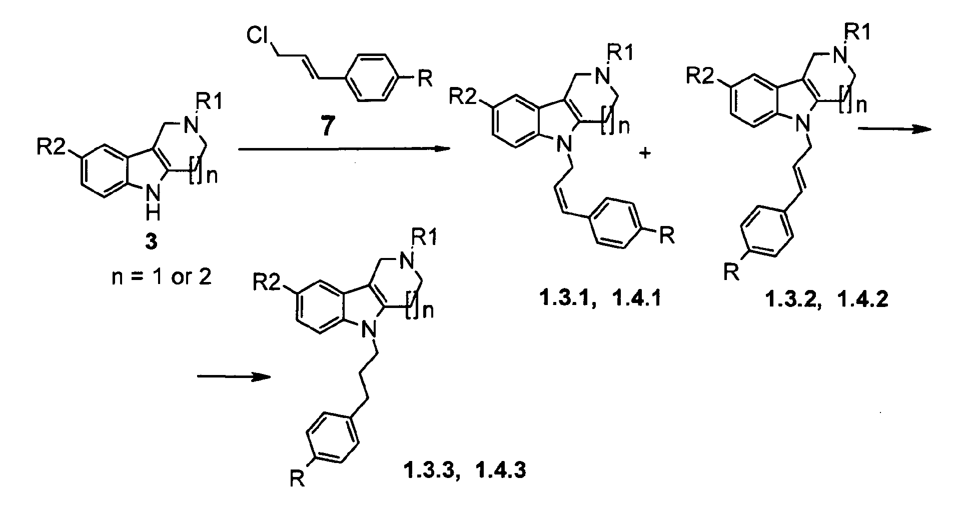

The subject of the present invention is also method for preparation of substituted hydrogenated 6-sulfonyl-azepino[4,3-b]indoles of general formula **1.8** by the reaction of compounds of formula **3** with corresponding sulfonylchlorides **8.**

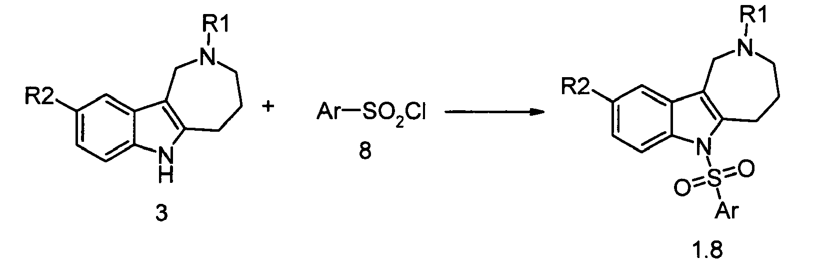

The subject of the present invention is also method for preparation of 5-benzyl-2-methyl-1,2,3,4-tetrahydro-1H-pyrido[4,3-b]indole methanesulfonate **1.9(1)·CH₃SO₃H** by reaction of 5-benzyl-2-methyl-1,2,3,4-tetrahydro-1H-pyrido[4,3-b]indole **1.9(1)·with** methanesulfonic acid **5.**

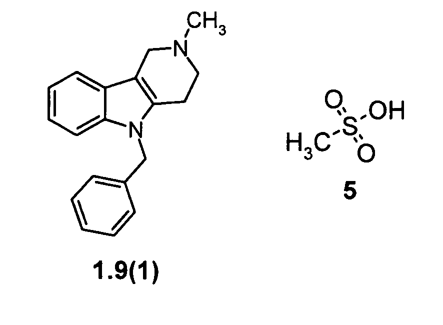

**Table 3. Ligands of general formula 1, spectrum of biological activity of which includes simultaneously alpha-adrenergic, dopamine, histamine, imidazoline and serotonin receptors.**

| **N°** | Formula | Mol. weight | LCMS, m/z(M+1) |
|---|---|---|---|
| **1.1(1)·HCl** | 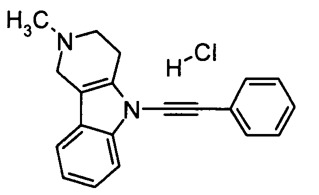 | 322.84 | 287 |
| **1.1(2)** | 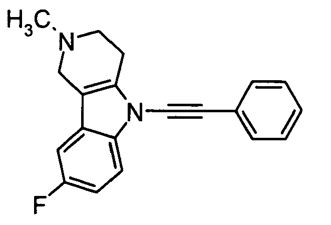 | 304.37 | 305 |
| **1.1(3)·HCl** | 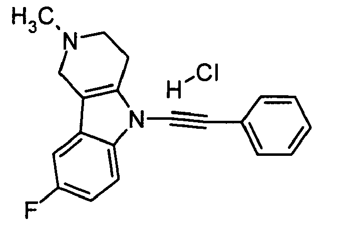 | 340.83 | 305 |
| **1.1(4)** | 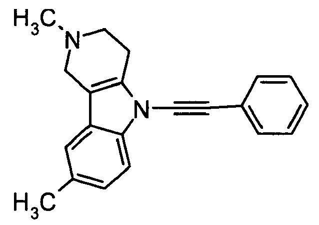 | 300.41 | 301 |
| **1.1(5)** | 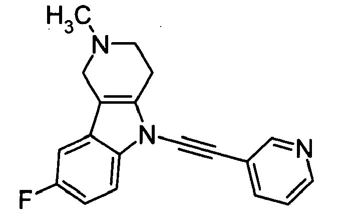 | 305.36 | 306 |
| **1.1(6)** | 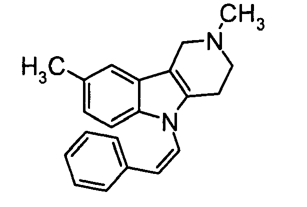 | 302.42 | 303 |
| **1.1.1(1)·HCl** | 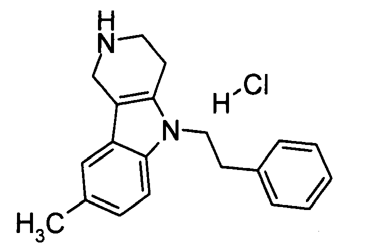 | 326.87 | 291 |
| **1.1.1(2)** | 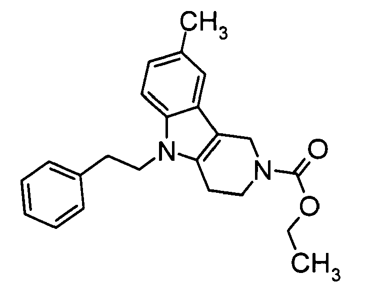 | 362.48 | 263 |
| **1.1.1(3)** | 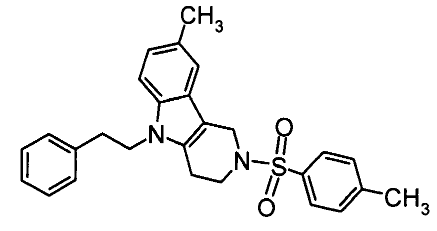 | 444.60 | 445 |
| **1.1.1(4)·2HCl** | 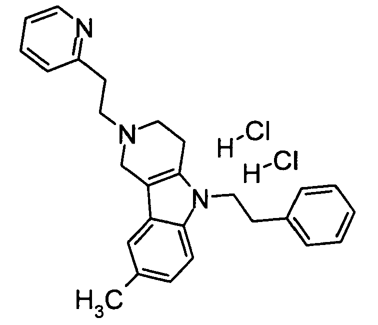 | 468.47 | 396 |
| **1.1.1(5)·2HCl** | 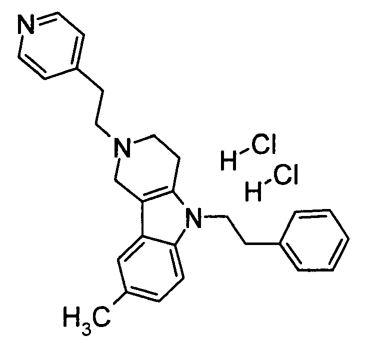 | 468.47 | 396 |
| **1.1.1(6)·2HCl** | 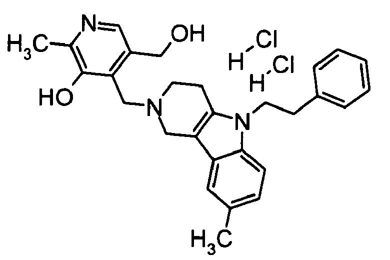 | 514.50 | 442 |
| **1.1.1(7)·2HCl** | 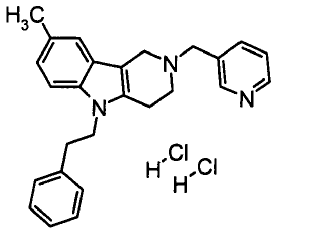 | 454.45 | 382 |
| **1.1.1(8)·HCl** | 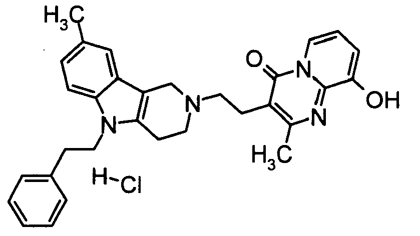 | 529.09 | 493 |
| **1.1.1(9)·HCl** | 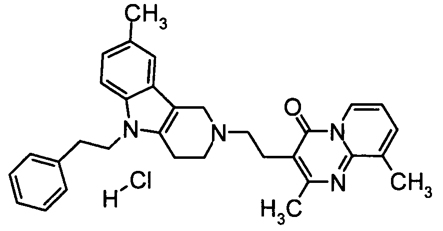 | 527.12 | 491 |
| **1.1.1(10)·HCl** | 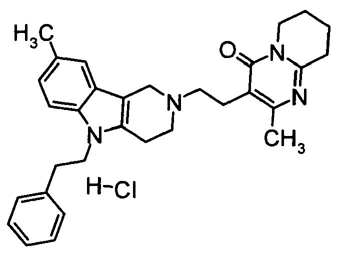 | 517.12 | 481 |
| **1.1.1(11)·HCl** | 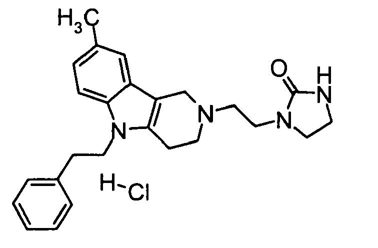 | 439.01 | 403 |
| **1.1.1(12)·2HCl** | 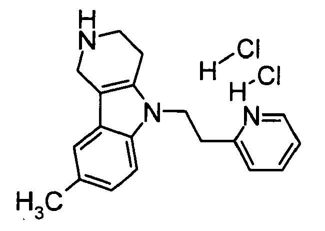 | 364.32 | 292 |
| **1.1.1(13)·2HCl** | 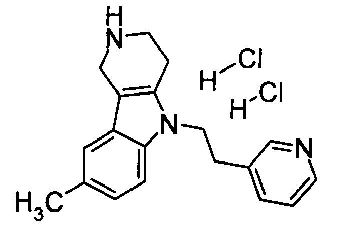 | 364.32 | 292 |
| **1.1.1(14)·2HCl** | 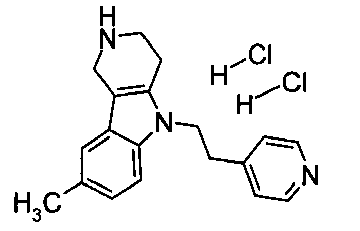 | 364.32 | 292 |
| **1.1.1(15)·3HCl** | 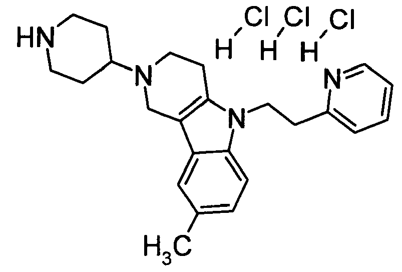 | 483.92 | 375 |
| **1.1.1(16)·3HCl** | 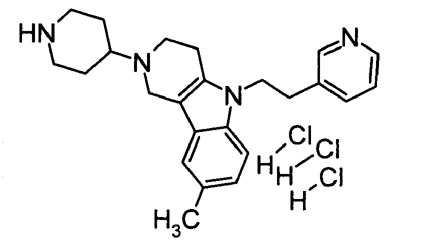 | 483.92 | 375 |
| **1.1.1(17)·3HCl** | 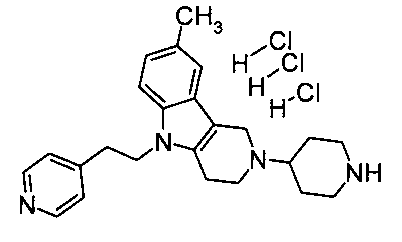 | 483.92 | 375 |
| **1.1.1(18)·3HCl** | 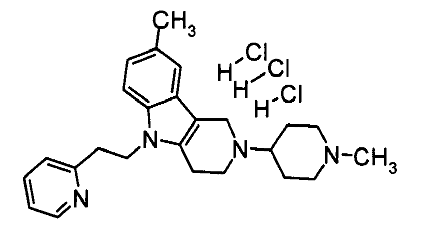 | 497.94 | 389 |
| **1.1.1(19)·3HCl** | 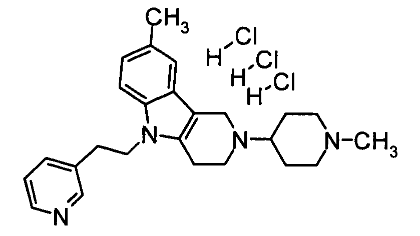 | 497.94 | 389 |
| **1.1.1(20)·3HCl** | 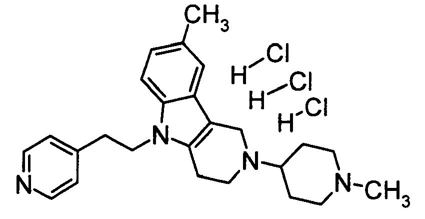 | 497.94 | 389 |
| **1.1.1(21)** | 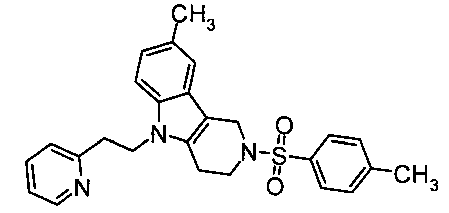 | 445.59 | 446 |
| **1.1.1(22)** | 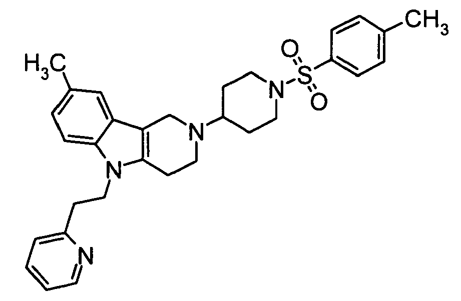 | 528.72 | 529 |
| **1.1.1(23)** | 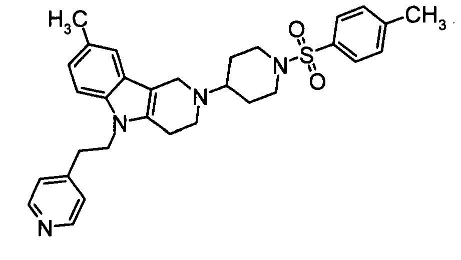 | 528.72 | 529 |
| **1.1.2(1-1)** | 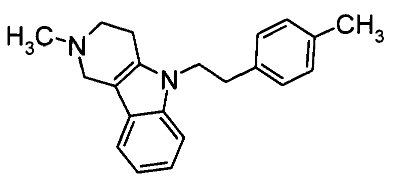 | 304.44 | 305 |
| **1.1.2(1-2)·** | 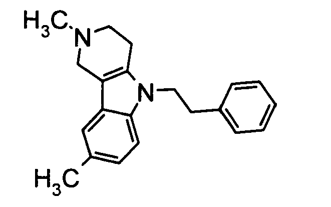 | | |
| **1.1.2(1-2)·HCl** | 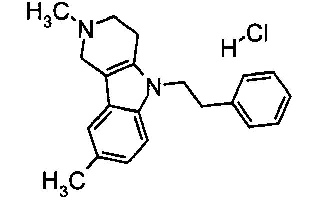 | 340.90 | 305 |
| **1.1.2(1-3)** | 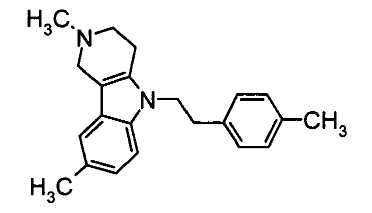 | 318.47 | 319 |
| **1.1.2(1-3)·HCl** | 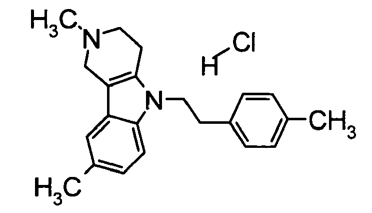 | | |
| **1.1.2(1-4)** | 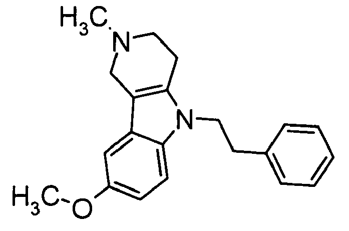 | 320.44 | 321 |
| **1.1.2(1-4)·HCl** | 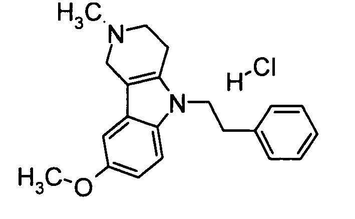 | 356.90 | 321 |
| **1.1.2(1-6)·HCl** | 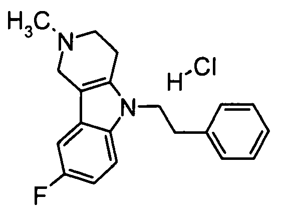 | 344.86 | 309 |
| **1.1.2(1-7)·HCl** | 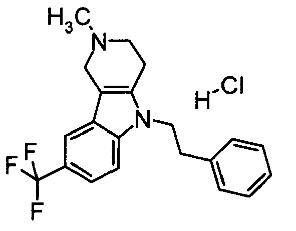 | 394.87 | 359 |
| **1.1.2(1-8)·HCl** | 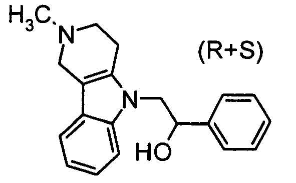 | 306.41 | 307 |
| **1.1.2(1-9)** | 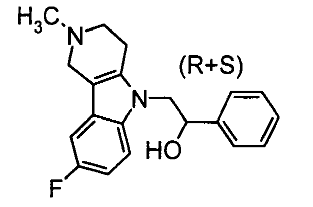 | 324.40 | 325 |
| **1.1.2(1-10)** | 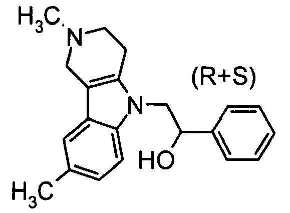 | 320.44 | 321 |
| **1.1.2(1-11)** | 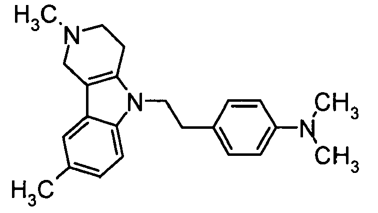 | 347.51 | 348 |
| **1.1.2(1-12)·2HCl** | 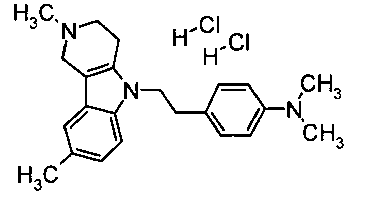 | 420.43 | 348 |
| **1.1.2(1-13)** | 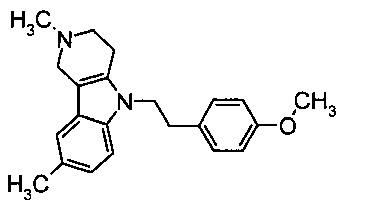 | 334.47 | 335 |
| **1.1.2(1-14)·HCl** | 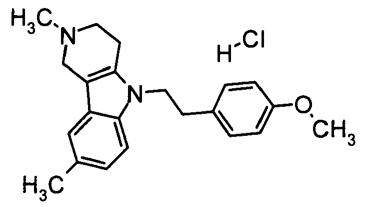 | 370.93 | 335 |
| **1.1.2(1-15)** | 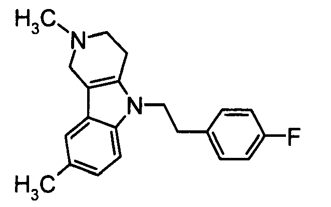 | 322.43 | 323 |
| **1.1.2(1-16)·HCl** | 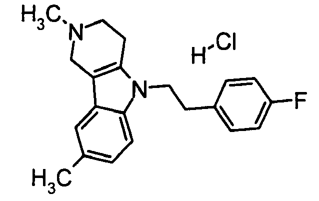 | 358.89 | 323 |
| **1.1.2(1-17)** | 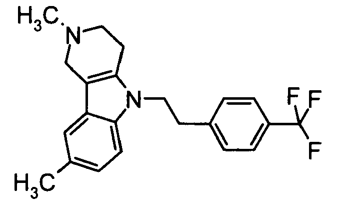 | 372.44 | 373 |
| **1.1.2(1-18)·HCl** | 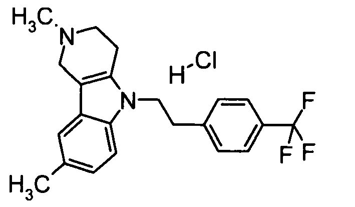 | 408.90 | 373 |
| **1.1.2(1-19)** | 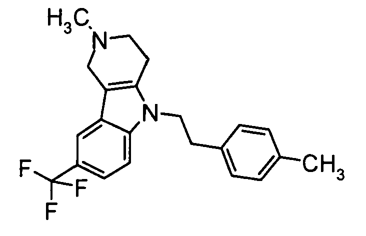 | 408.90 | 373 |
| **1.1.2(1-20)** | 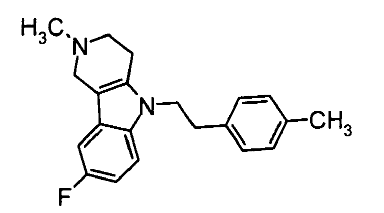 | 322.43 | 323 |
| **1.1.2(1-21)·HCl** | 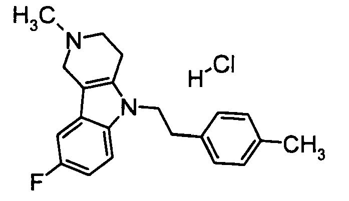 | 358.89 | 323 |
| **1.1.2(1-22)·HCl** | 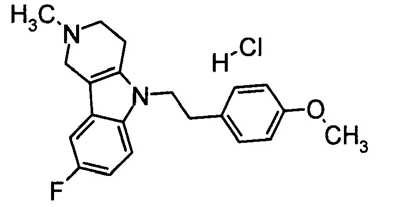 | 374.89 | 339 |
| **1.1.2(1-23)·HCl** | 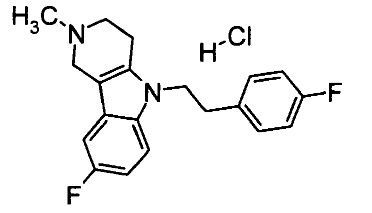 | 362.85 | 327 |
| **1.1.2(1-24)·HCl** | 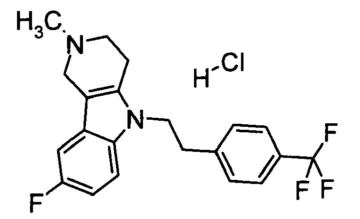 | 412.86 | 377 |
| **1.1.2(1-25)** | 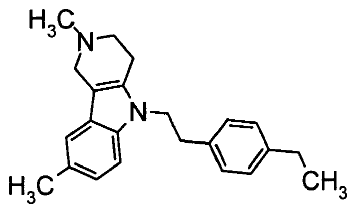 | | |
| **1.1.2(1-25)·HCl** | 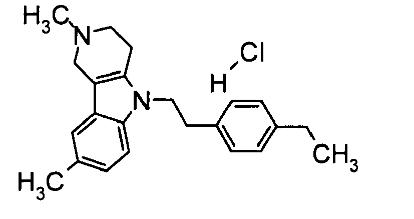 | | |
| **1.1.2(1-26)** | 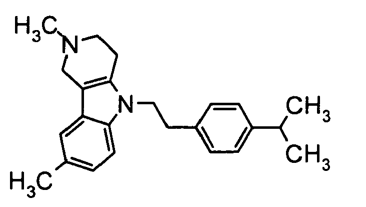 | | |
| **1.1.2(1-26)·HCl** | 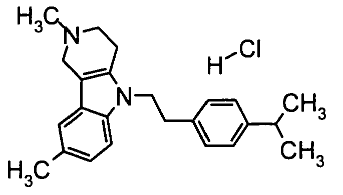 | | |
| **1.1.2(1-27)** | 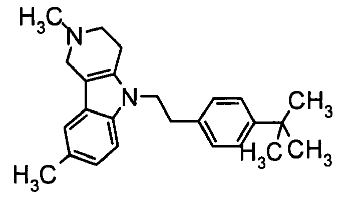 | | |
| **1.1.2(1-27)·HCl** | 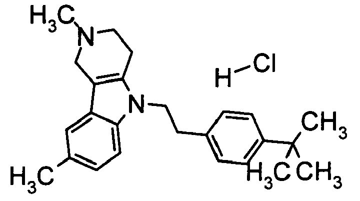 | | |
| **1.1.2(2-1)·2HCl** | 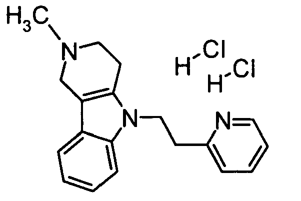 | 364.32 | 292 |
| **1.1.2(2-2)** | 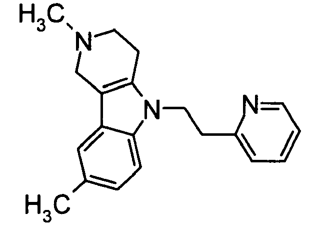 | 305.43 | 306 |
| **1.1.2(2-3)·2HCl** | 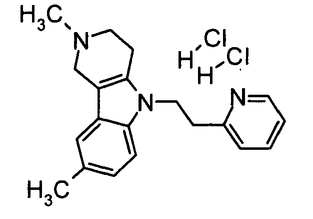 | 378.35 | 306 |
| **1.1.2(2-4)·2HCl** | 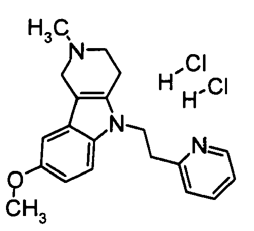 | 394.35 | 322 |
| **1.1.2(2-5)·2HCl** | 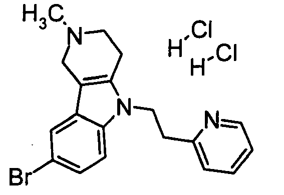 | 443.22 | 371 |
| **1.1.2(2-6)·2HCl** | 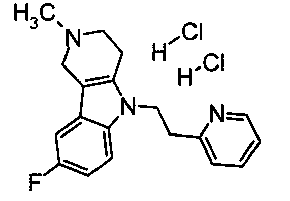 | 382.31 | 310 |
| **1.1.2(2-7)·2HCl** | 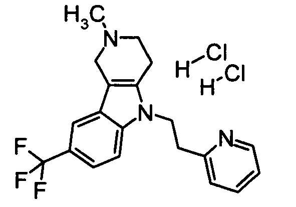 | 431.32 | 360 |
| **1.1.2(2-8)·2HCl** | 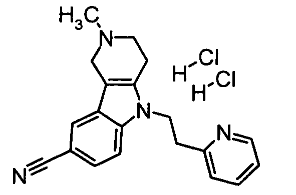 | 389.33 | 317 |
| **1.1.2(2-9)·3HCl** | 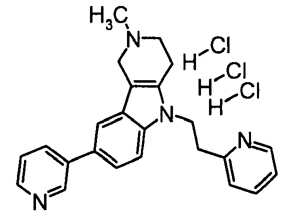 | 477.87 | 369 |
| **1.1.2(2-10)·2HCl** | 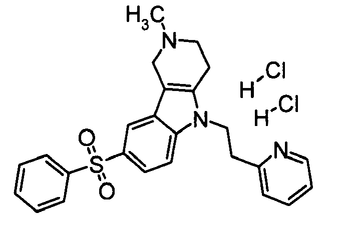 | 504.48 | 432 |
| **1.1.2(3-1)·2HCl** | 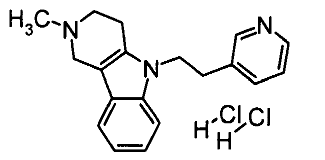 | 364.32 | 292 |
| **1.1.2(3-2)** | 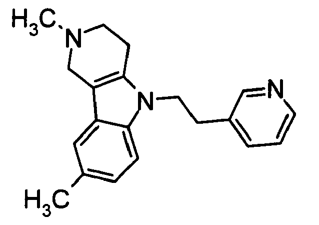 | 305.43 | 306 |
| **1.1.2(3-3)·2HCl** | 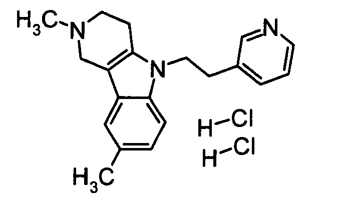 | 378.35 | 306 |
| **1.1.2(3-4)·2HCl** | 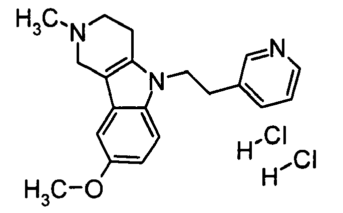 | 394.35 | 322 |
| **1.1.2(3-5)·2HCl** | 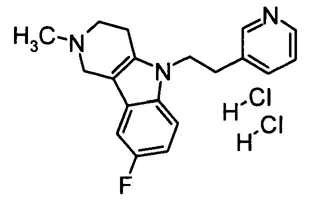 | 382.31 | 310 |
| **1.1.2(3-6)·2HCl** | 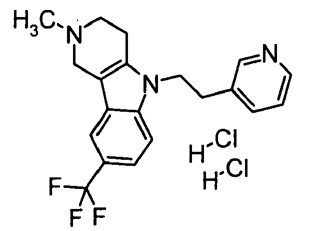 | 431.32 | 360 |
| **1.1.2(4-1)** | 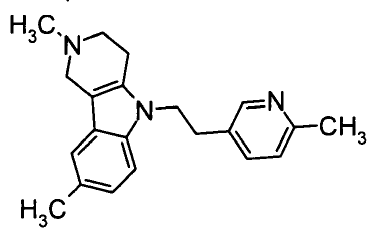 | 319.45 | 320 |
| **1.1.2(4-2)·2HCl** | 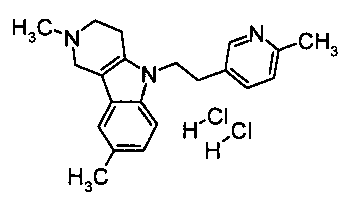 | 392.37 | 320 |
| **1.1.2(4-3)·2HCl** | 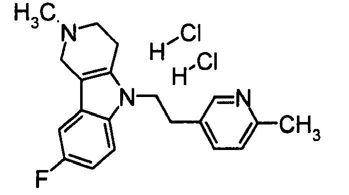 | 396.34 | 324 |
| **1.1.2(4-4) ·2CH₃SO₃H** | 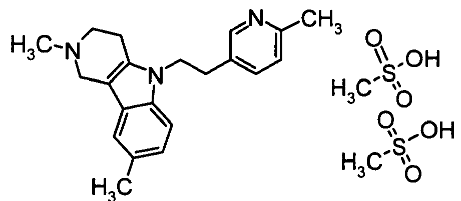 | 511.67 | 320 |
| **1.1.2(4-5) ·1/2NDSA** | 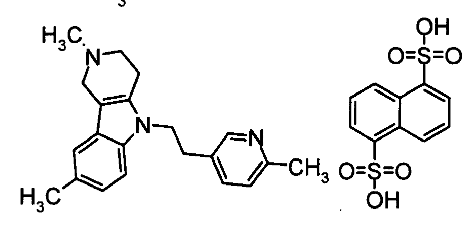 | 607.75 | 320 |
| **1.1.2(5-1)** | 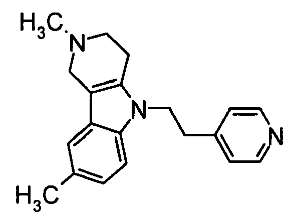 | 305.43 | 306 |
| **1.1.2(5-2)·2HCl** | 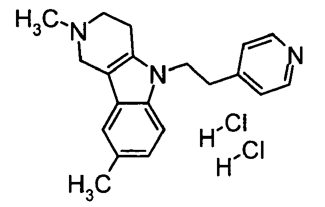 | 378.35 | 306 |
| **1.1.2(5-3)·2HCl** | 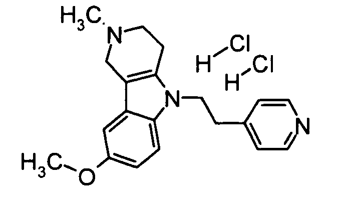 | 394.35 | 322 |
| **1.1.2(5-4)·2HCl** | 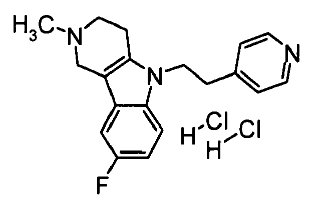 | 382.31 | 310 |
| **1.1.2(5-5)·2HCl** | 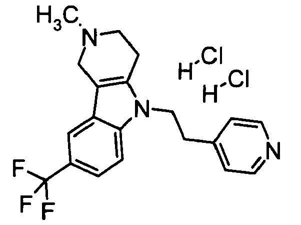 | 431.32 | 360 |
| **1.1.2(5-6)·2HCl** | 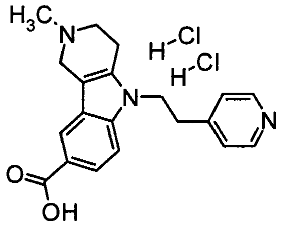 | 408.33 | 336 |
| **1.1.2(5-7)·2HCl** | 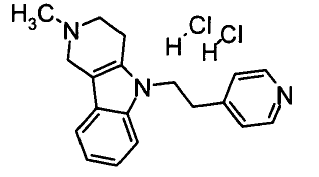 | 364.32 | 292 |
| **1.1.2(6-1)** | 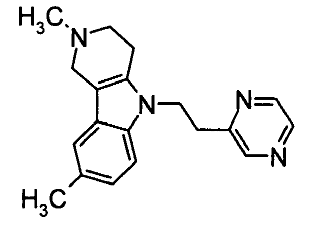 | 306.41 | 307 |
| **1.1.2(6-2)·2HCl** | 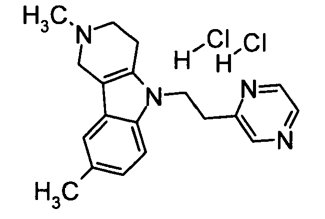 | 379.34 | 307 |
| **1.2.2(1-1)** | 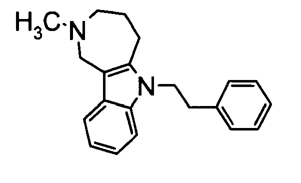 | 304.44 | 305 |
| **1.2.2(1-2)** | 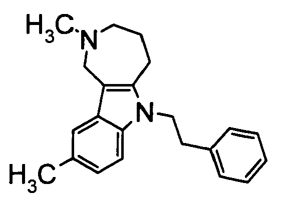 | 318.47 | 319 |
| **1.2.2(1-3)·HCl** | 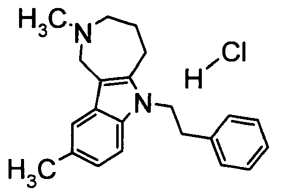 | 354.93 | 319 |
| **1.2.2(1-4)** | 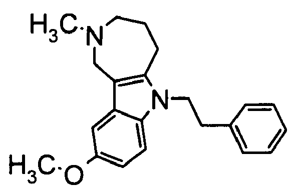 | 334.47 | 335 |
| **1.2.2(1-5)** | 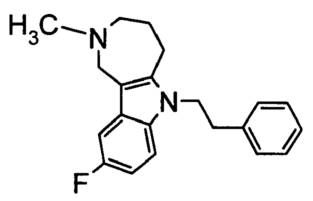 | 322.43 | 323 |
| **1.2.2(1-6)·HCl** | 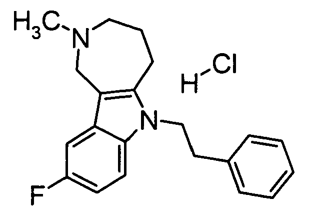 | 358.89 | 323 |
| **1.2.2(1-7)** | 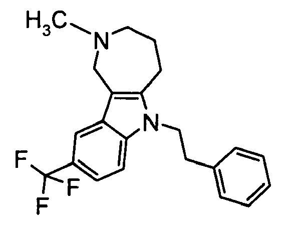 | 372.44 | 373 |
| **1.2.2(1-8)** | 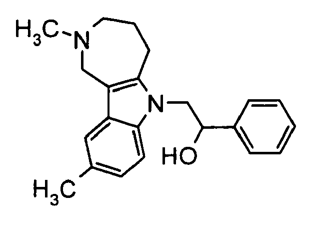 | 334.47 | 335 |
| **1.2.2(1-9)** | 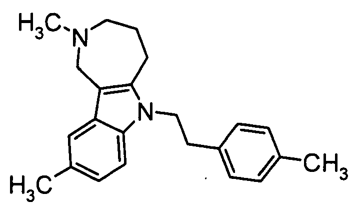 | 332.49 | 333 |
| **1.2.2(1-10)** | 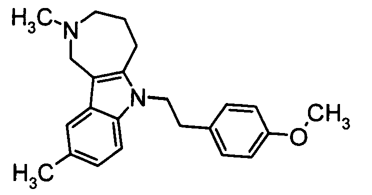 | 348.49 | 349 |
| **1.2.2(1-11)·HCl** | 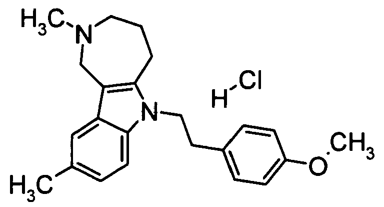 | 384.95 | 349 |
| **1.2.2(1-12)** | 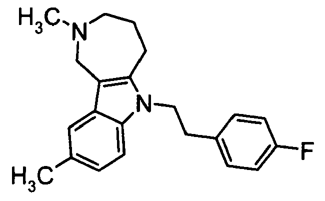 | 372.92 | 373 |
| **1.2.2(1-13)** | 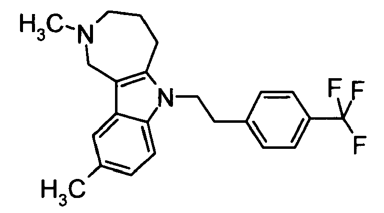 | 386.46 | 387 |
| **1.2.2(1-13)·HCl** | 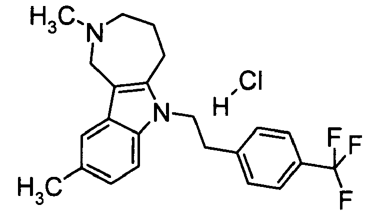 | 422.93 | 387 |
| **1.2.2(1-15)** | 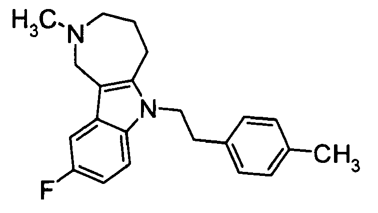 | 336.46 | 337 |
| **1.2.2(1-16)·HCl** | 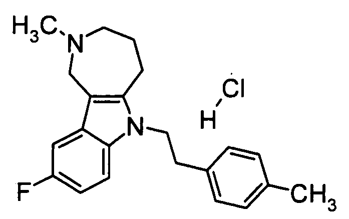 | 372.92 | 337 |
| **1.2.2(1-17)·HCl** | 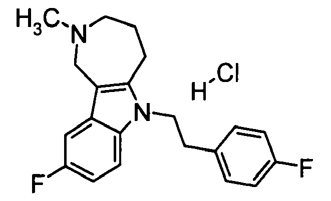 | 376.88 | 341 |
| **1.2.2(1-18)·HCl** | 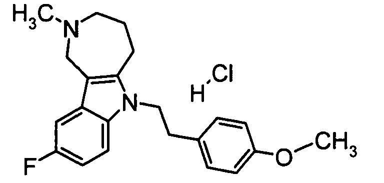 | 388.92 | 353 |
| **1.2.2(1-19)·HCl** | 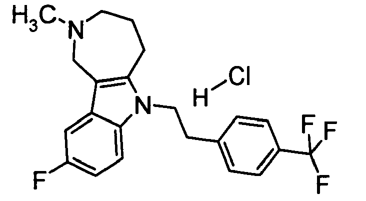 | 426.89 | 391 |
| **1.2.2(2-1)·2HCl** | 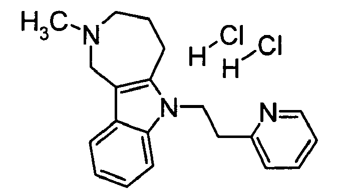 | 378.34 | 306 |
| **1.2.2(2-2)** | 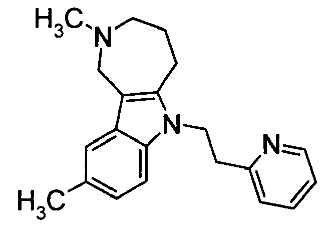 | 319.45 | 320 |
| **1.2.2(2-3)·2HCl** | 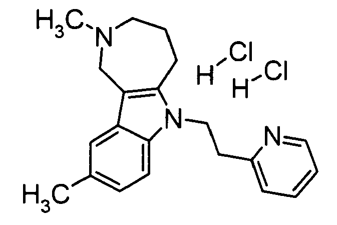 | 392.38 | 320 |
| **1.2.2(2-4)·2HCl** | 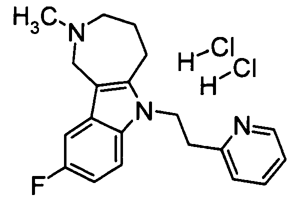 | 396.34 | 324 |
| **1.2.2(3-1)** | 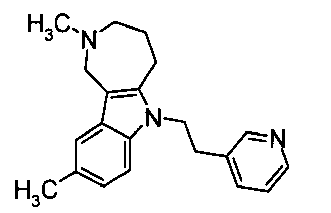 | 319.45 | 320 |
| **1.2.2(3-2)·2HCl** | 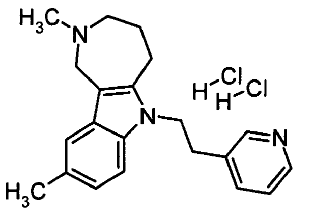 | 392.38 | 320 |
| **1.2.2(3-3)·2HCl** | 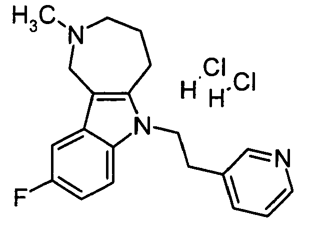 | 396.34 | 324 |
| **1.2.2(4-1)** | 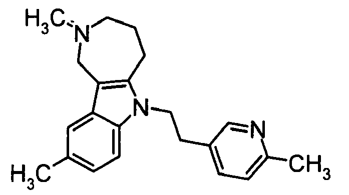 | 333.48 | 334 |
| **1.2.2(4-2)·2HCl** | 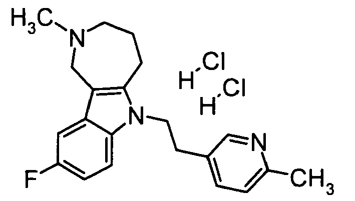 | 410.37 | 338 |
| **1.2.2(5-1)·2HCl** | 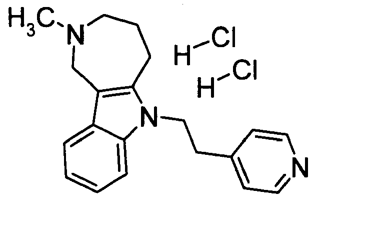 | 378.34 | 306 |
| **1.2.2(5-2)** | 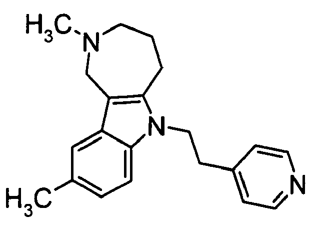 | 319.45 | 320 |
| **1.2.2(5-3)·2HCl** | 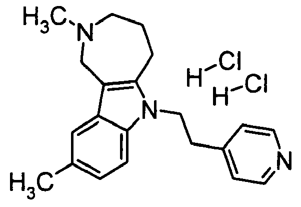 | 392.38 | 320 |
| **1.2.2(5-4)·2HCl** | 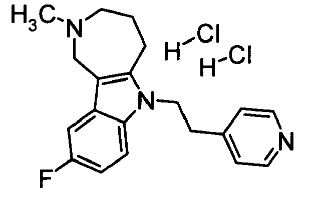 | 396.34 | 324 |
| **1.2.2(6-1)** | 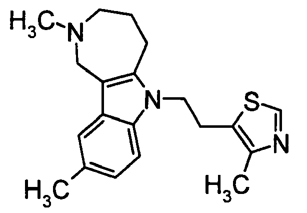 | 339.51 | 340 |
| **1.3.1(1)** | 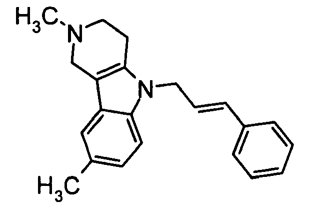 | 316.45 | 317 |
| **1.3.1(2)** | 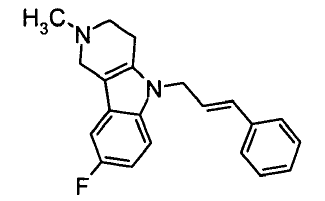 | 320.41 | 321 |
| **1.3.2(1)** | 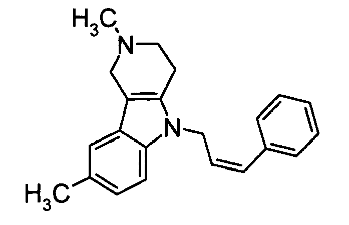 | 316.45 | 317 |
| **1.3.2(2)** | 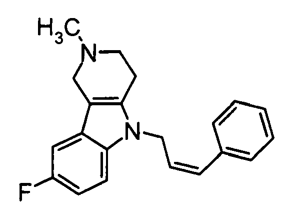 | 320.41 | 321 |
| **1.3.3(1)** | 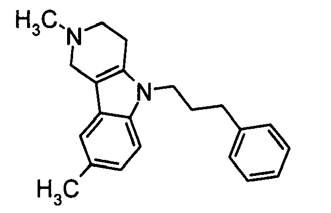 | 318.47 | 319 |
| **1.3.3(2)·HCl** | 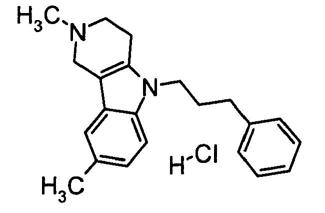 | 354.93 | 319 |
| **1.3.3(3)** | 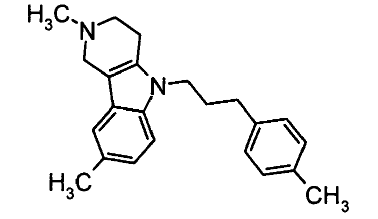 | 332.49 | 333 |
| **1.3.3(4)·HCl** | 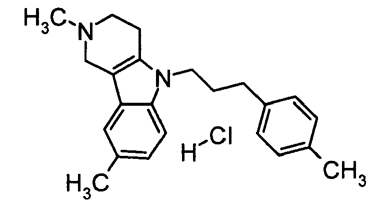 | 368.95 | 333 |
| **1.3.3(5)** | 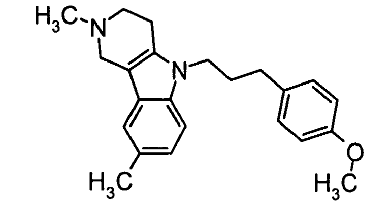 | 348.49 | 349 |
| **1.3.3(6)** | 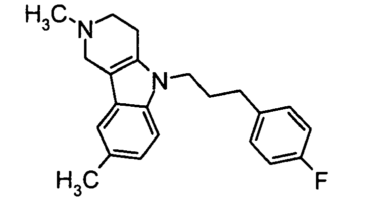 | 336.46 | 337 |
| **1.3.3(7)** | 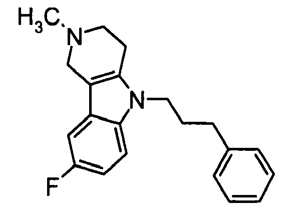 | 322.43 | 323 |
| **1.3.3(8)·HCl** | 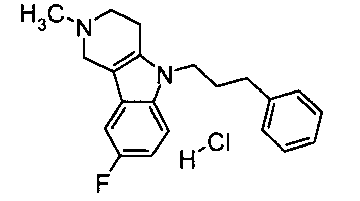 | 358.89 | 323 |
| **1.3.3(9)** | 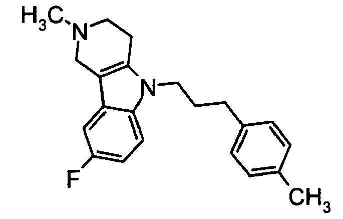 | 336.46 | 337 |
| **1.3.3(10)** | 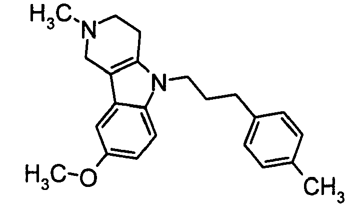 | 348.49 | 349 |
| **1.4.3(1)** | 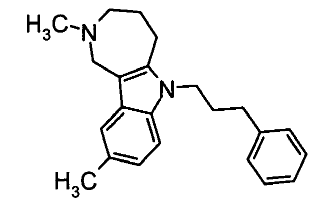 | 332.49 | 333 |
| **1.4.3(2)** | 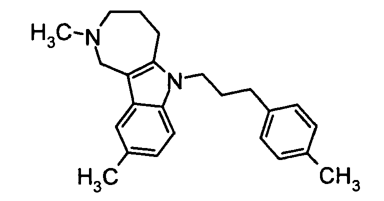 | 346.52 | 347 |
| **1.5(1)** | 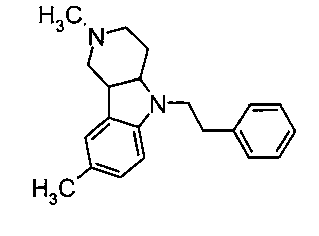 | 306.45 | 307 |
| **1.5(2)** | 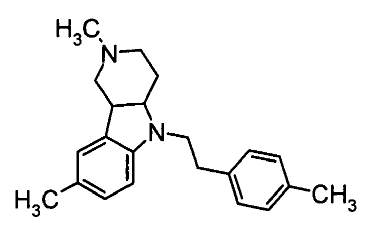 | 320.48 | 321 |
| **1.5(3)** | 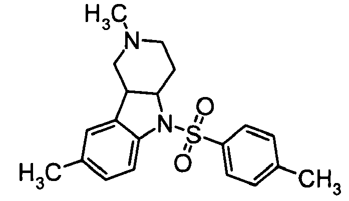 | 356.49 | 357 |
| **1.5(4)** | 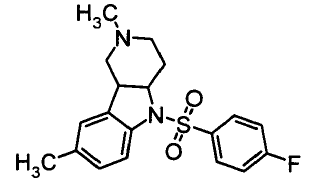 | 360.45 | 361 |
| **1.5(5)** | 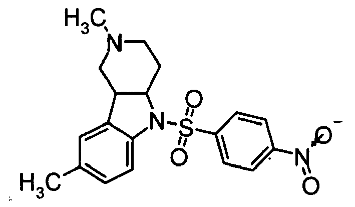 | 387.46 | 388 |
| **1.5(6)** | 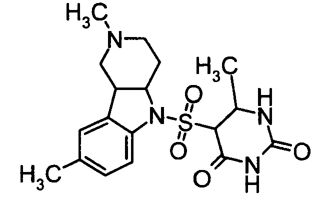 | 392.48 | 393 |
| **1.5(7)** | 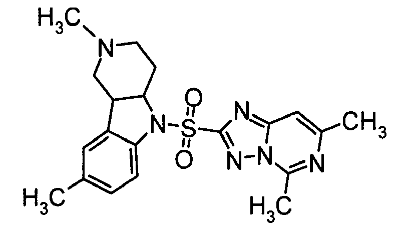 | 412.52 | 413 |
| **1.6.1(1)·3HCl** | 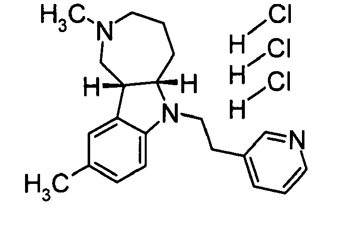 | 430.85 | 322 |
| **1.6.1(2)·3HCl** | 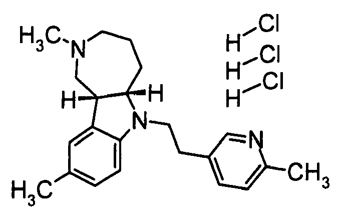 | 444.88 | 336 |
| **1.6.1(3)·3HCl** | 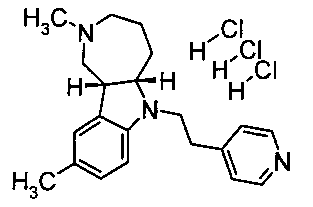 | 430.85 | 322 |
| **1.6.2(1)** | 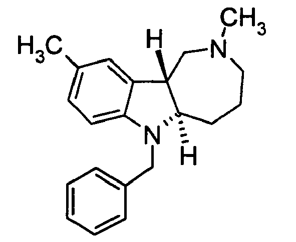 | 303.45 | 304 |
| **1.6.2(2)** | 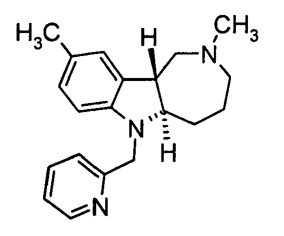 | 307.44 | 308 |
| **1.6.2(3)** | 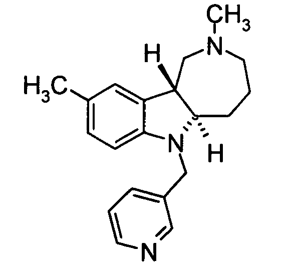 | 307.44 | 308 |
| **1.6.2(4)** | 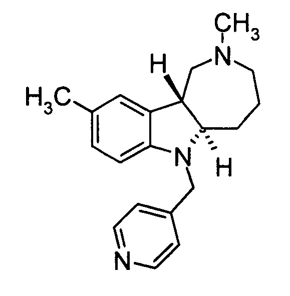 | 307.44 | 308 |
| **1.6.2(5)** | 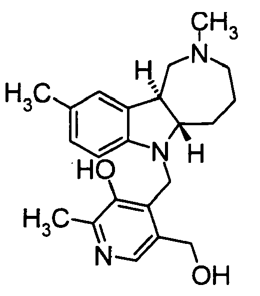 | 367.49 | 368 |
| **1.6.2(6)** | 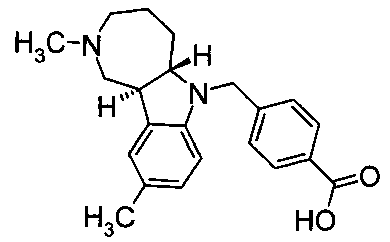 | 350.46 | 351 |
| **1.6.2(7)** | 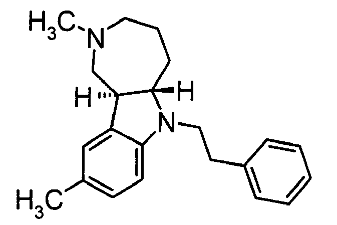 | 320.48 | 321 |
| **1.6.2(8)** | 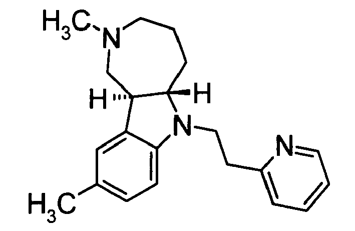 | 321.47 | 322 |
| **1.6.2(9)** | 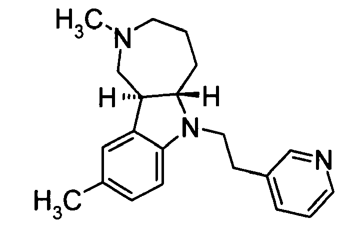 | 321.47 | 322 |
| **1.6.2(10)·3HCl** | 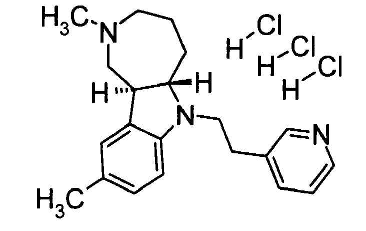 | 430.85 | 431 |
| **1.6.2(11)** | 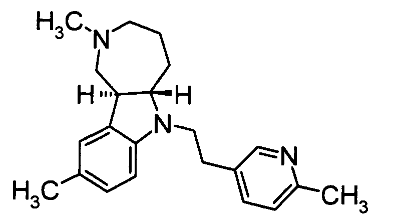 | 335.50 | 336 |
| **1.6.2(12)·3HCl** | 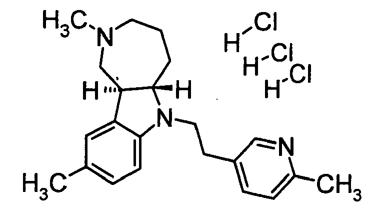 | 444.88 | 336 |
| **1.6.2(13)** | 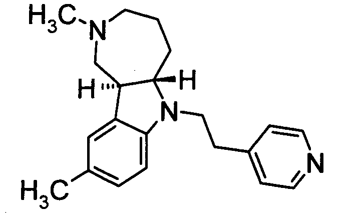 | 321.47 | 322 |
| **1.6.2(14) ·3CF₃CO₂H** | 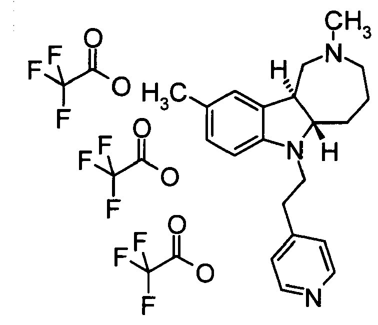 | 663.54 | 322 |
| **1.6.2(15)** | 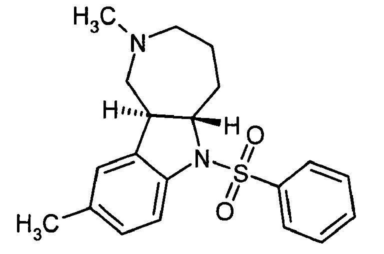 | 356.49 | 357 |
| **1.6.2(16)** | 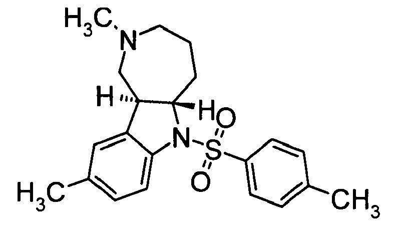 | 370.52 | 371 |
| **1.7.1(1)** | 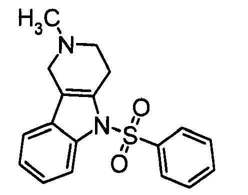 | 326.42 | 327 |
| **1.7.1(2)** | 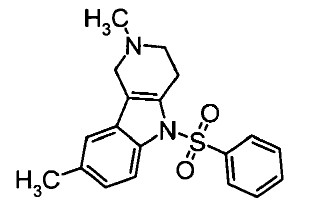 | 340.45 | 341 |
| **1.7.1(3)** | 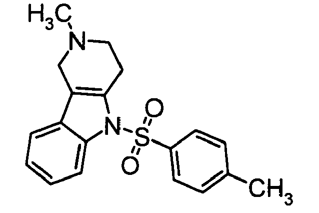 | 340.45 | 341 |
| **1.7.1(4)** | 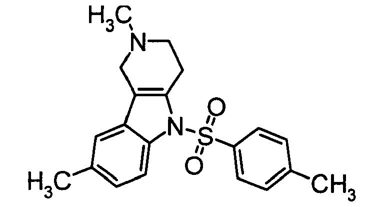 | 354.47 | 355 |
| **1.8.1(1)·HCl** | 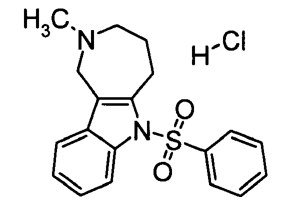 | 379.91 | 341 |
| **1.8.1(2)·HCl** | 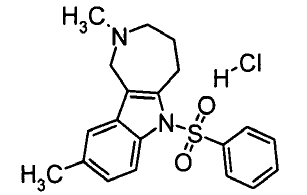 | 390.93 | 355 |
| **1.8.1(3)·HCl** | 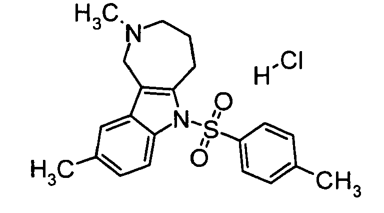 | 404.96 | 369 |
| **1.8.1(4)·HCl** | 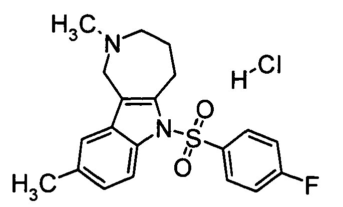 | 408.92 | 373 |
| **1.8.1(5)·HCl** | 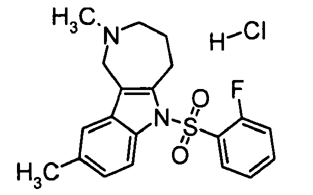 | 408.92 | 373 |
| **1.8.1(6)·HCl** | 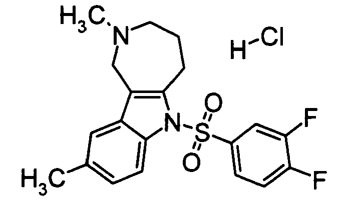 | 426.92 | 391 |
| **1.8.1(7)·HCl** | 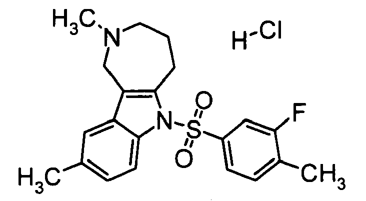 | 422.95 | 387 |
| **1.8.1(8)·HCl** | 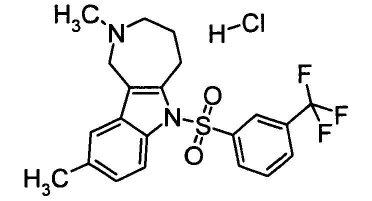 | 458.93 | 423 |
| **1.8·1(9)·HCl** | 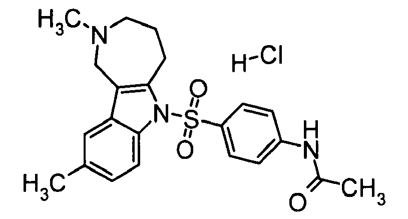 | 447.99 | 412 |
| **1.8.1(10)·HCl** | 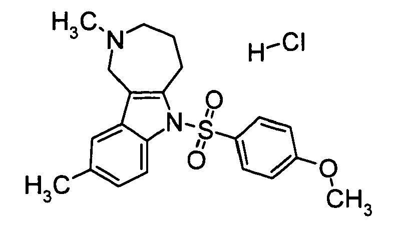 | 420.96 | 385 |
| **1.8.1(11)·HCl** | 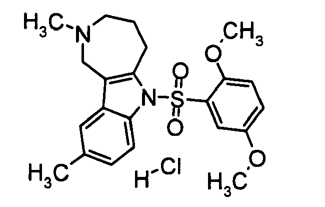 | 450.99 | 415 |
| **1.8.1(12)·HCl** | 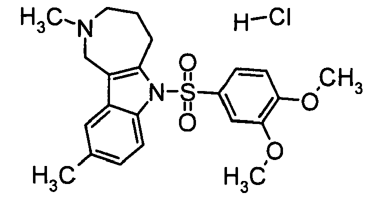 | 450.99 | 415 |
| **1.8.1(13)·HCl** | 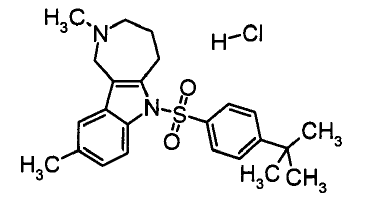 | 447.04 | 411 |
| **1.8.1(14)·HCl** | 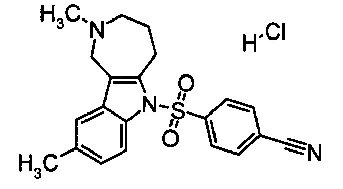 | 415.94 | 380 |
| **1.8.1(15)·HCl** | 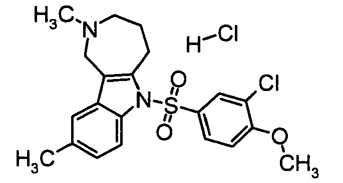 | 455.41 | 419 |
| **1.8.1(16)·HCl** | 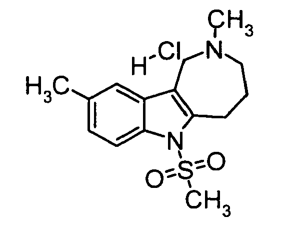 | 328.86 | 293 |
| **1.8.1(17)·HCl** | 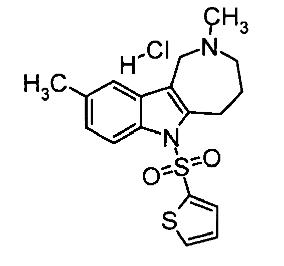 | 396.96 | 361 |
| **1.8.1(18)·HCl** | 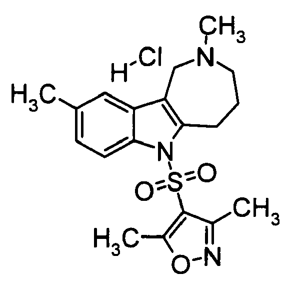 | 414.82 | 374 |
| **1.8.1(19)·HCl** | 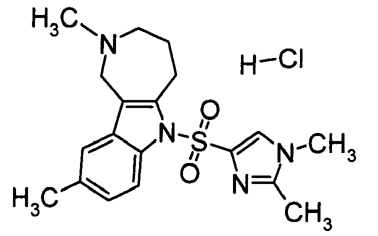 | 408.95 | 373 |
| **1.8.1(20)·HCl** | 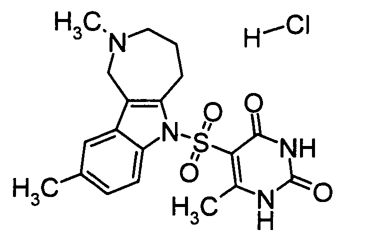 | 438.94 | 403 |
| **1.8.1(21)·HCl** | 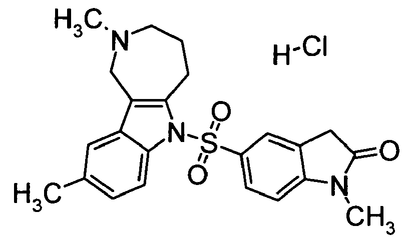 | 460.00 | 424 |
| **1.8.1(22)·HCl** | 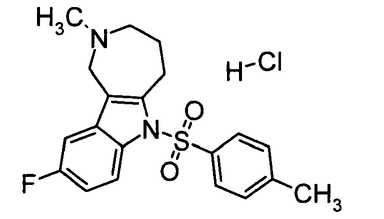 | 408.92 | 373 |
| **1.8.1(23)·HCl** | 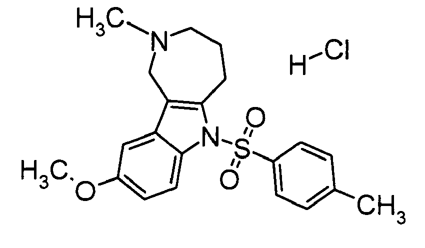 | 420.96 | 385 |
| **1.9(1)·HCl** | 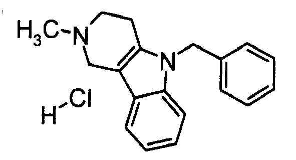 | 312.84 | 277 |
| **1.9(1)·CH₃SO₃H** | 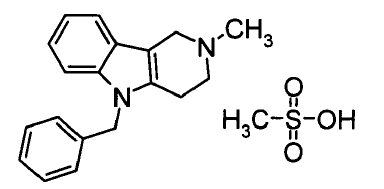 | 372.49 | 277 |
| **1.9(1)·1/2NDSA** | 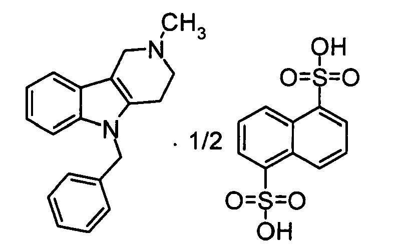 | 841.06 | 277 |
| **1.9(2)·HCl** | 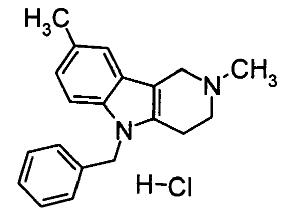 | 327.87 | 291 |
| **1.9(3)·HCl** | 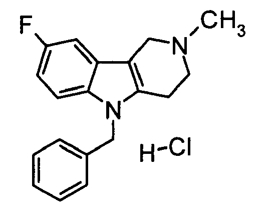 | 330.84 | 295 |
| **1.9(4)** | 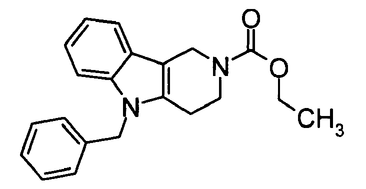 | 334.42 | 335 |
| **1.9(5)** | 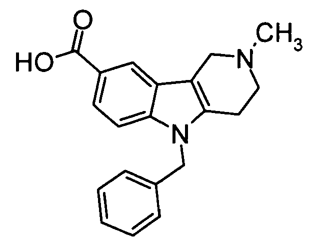 | 320.39 | 321 |
| **1.9(6)** | 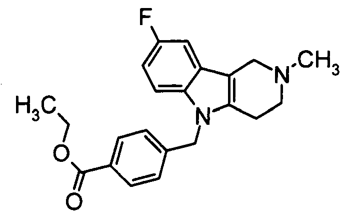 | 366.44 | 367 |
| **1.9(7)·HCl** | 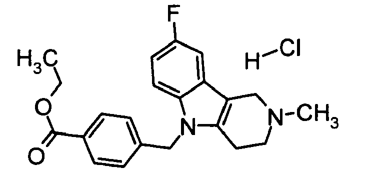 | 402.90 | 367 |
| **1.9(8)·HCl** | 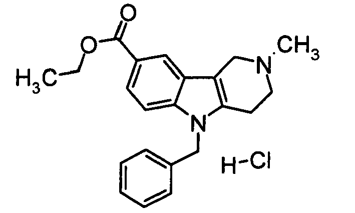 | 384.91 | 349 |
| **1.9(9)**·**HCl** | 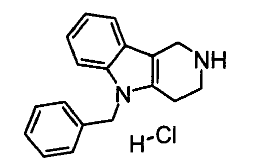 | 298.82 | 263 |
| **1.9(10)·2HCl** | 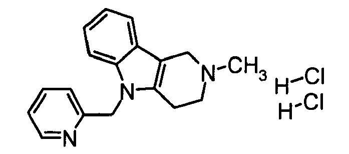 | 350.29 | 278 |
| **1.9(11)·2HCl** | 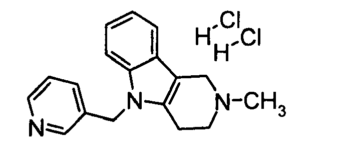 | 350.29 | 278 |
| **1.9(12)·2HCl** | 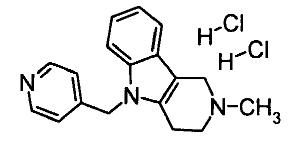 | 350.29 | 278 |
| **1.9(13)·2HCl** | 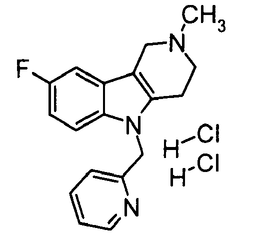 | 368.28 | 296 |
| **1.9(14)·2HCl** | 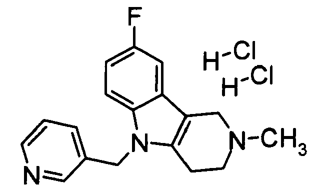 | 368.28 | 296 |
| **1.9(15)·2HCl** | 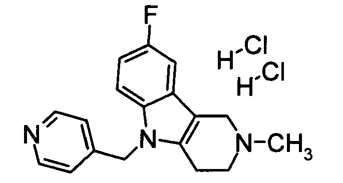 | 368.28 | 296 |
| **1.9(16)·HCl** | 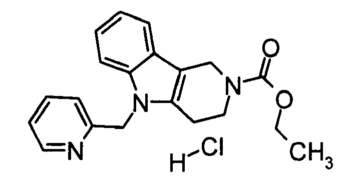 | 371.87 | 336 |
| **1.9(17)·HCl** | 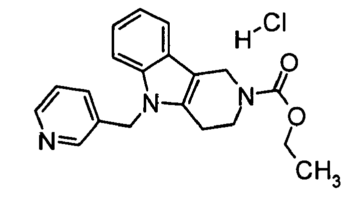 | 371.87 | 336 |
| **1.9(18)·HCl** | 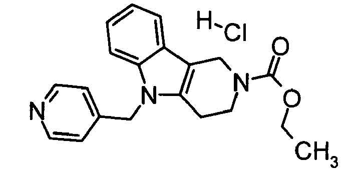 | 371.87 | 336 |
| **1.9(19)·2HCl** | 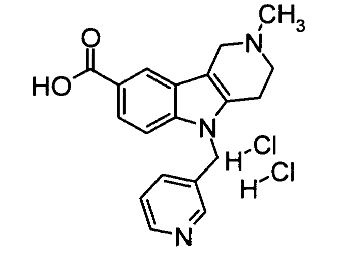 | 394.30 | 322 |
| **1.9(20)·2HCl** | 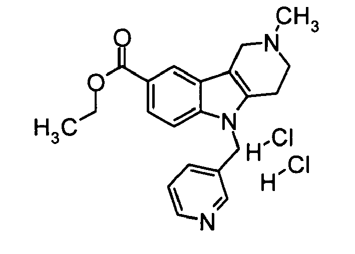 | 422.36 | 350 |
| **1.9(21)·2HCl** | 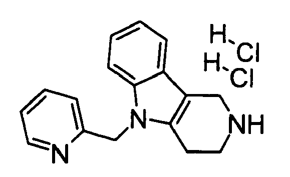 | 336.27 | 264 |
| **1.9(22)·2HCl** | 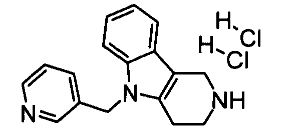 | 336.27 | 264 |
| **1.9(23)·2HCl** | 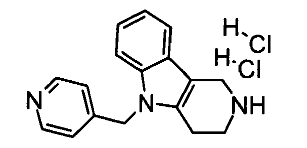 | 336.27 | 264 |
| **1.10(1)** | 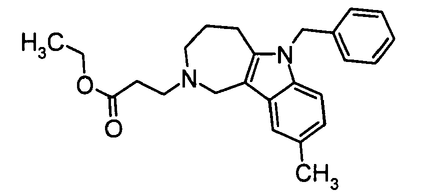 | 390.53 | 391 |
| **1.10(2)·HCl** | 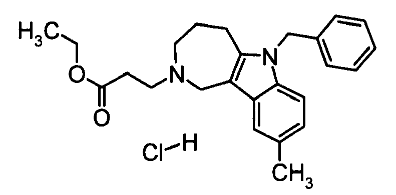 | 426.99 | 391 |
| **1.10(3)** | 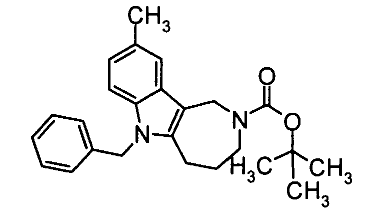 | 390.53 | 391 |
| **1.10(4)·HCl** | 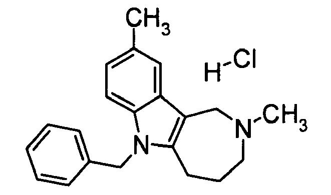 | 340.90 | 305 |
| **1.10(5)·HCl** | 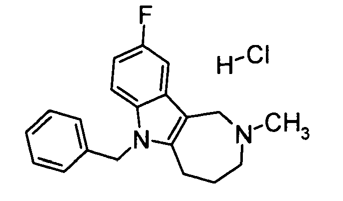 | 344.86 | 309 |
| **1.10(6)·HCl** | 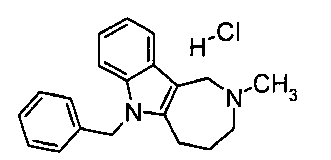 | 326.87 | 291 |
| **1.10(7)** | 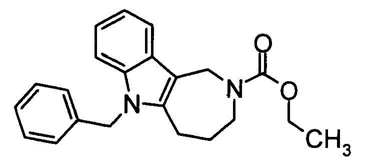 | 348.45 | 349 |
| **1.10(8)·HCl** | 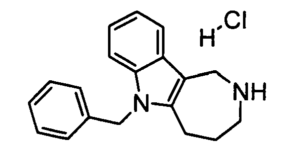 | 312.85 | 277 |
| **1.10(9)** | 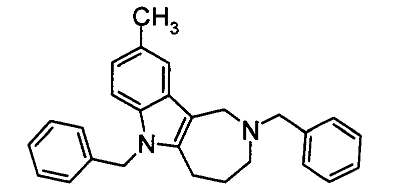 | 380.54 | 381 |
| **1.10(10)** | 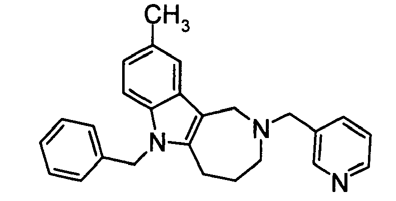 | 381.53 | 382 |
| **1.10(11)** | 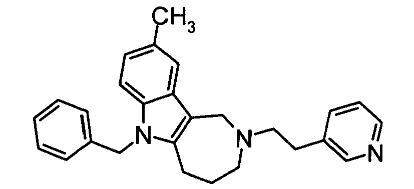 | 395.55 | 396 |
| **1.10(12)** | 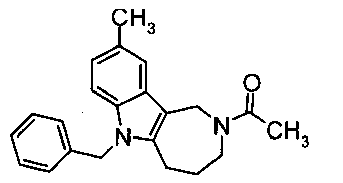 | 332.45 | 333 |
| **1.10(13)** | 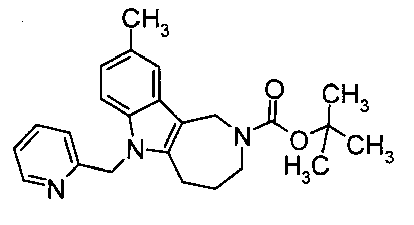 | 391.52 | 392 |
| **1.10(14)·2HCl** | 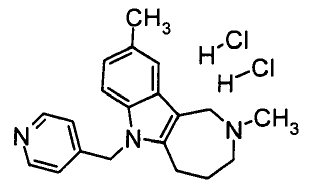 | 378.35 | 379 |
| **1.10(15)·2HCl** | 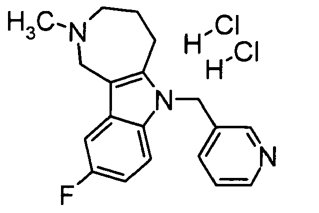 | 382.31 | 310 |
| **1.10(16)·2HCl** | 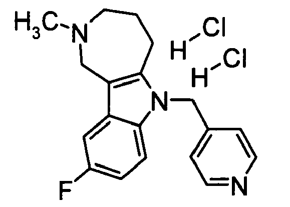 | 382.31 | 310 |
| **1.10(17)·2HCl** | 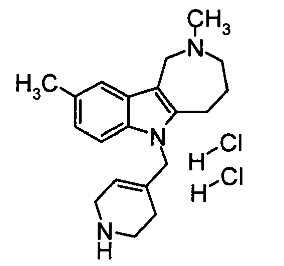 | 382.38 | 310 |
| **1.10(18)·2HCl** | 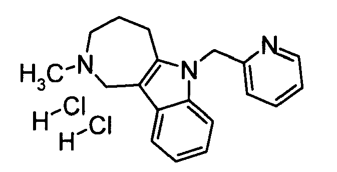 | 364.32 | 292 |
| **1.10(19)·2HCl** | 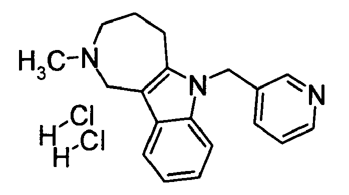 | 364.32 | 292 |
| **1.10(20)·2HCl** | 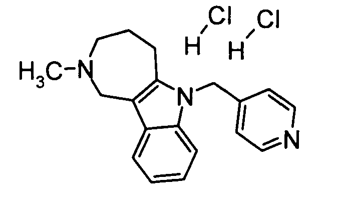 | 364.32 | 292 |
| **1.10(21)·HCl** | 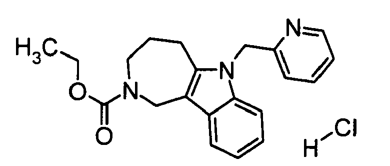 | 385.90 | 350 |
| **1.10(22)·HCl** | 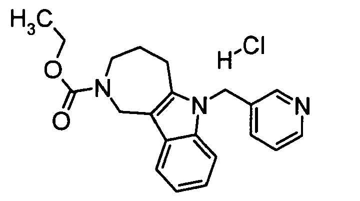 | 385.90 | 350 |
| **1.10(23)·HCl** | 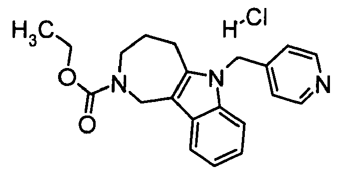 | 385.90 | 350 |
| **1.10(24)·2HCl** | 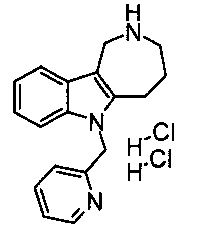 | 350.29 | 278 |
| **1.10(25)·2HCl** | 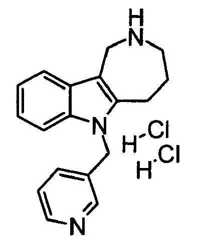 | 350.29 | 278 |
| **1.10(26)·2HCl** | 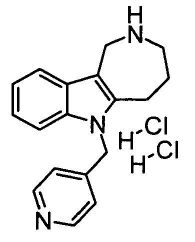 | 350.29 | 278 |

### Best Embodiment of the invention

The invention is illustrated by the following figures.

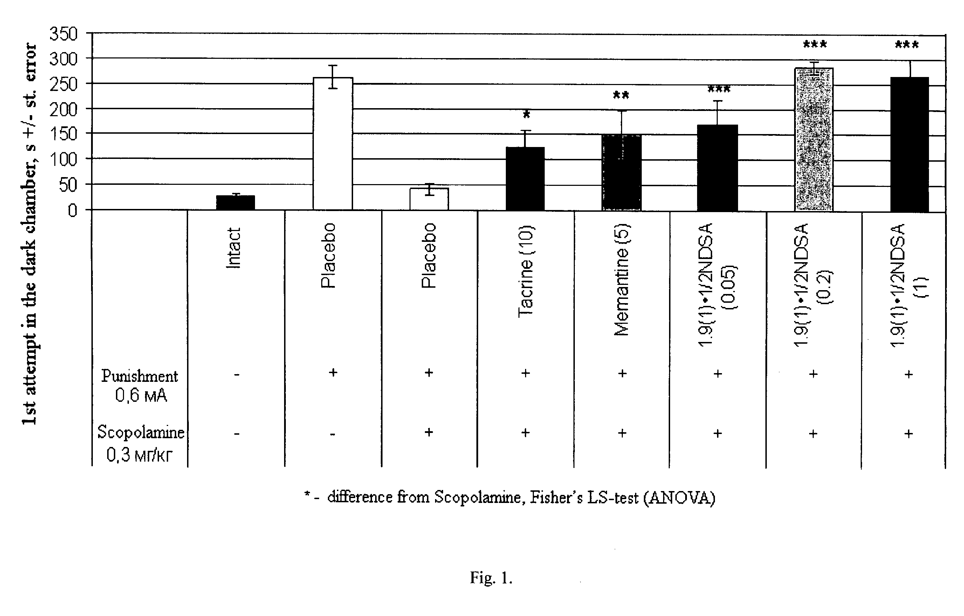

**Fig. 1****.** Enhancement of memory disturbed by Scopolamine in male mice of BALB/c line under the influence of ingredient **1.9(1)-1/2NDSA** and reference substances (Tacrine and Memantine) in test «Passive avoidance of mice in the shuttle chamber». The time, after which animals do the first entry into the dark chamber. The dosages of ligands in mg/kg are mentioned in brackets.

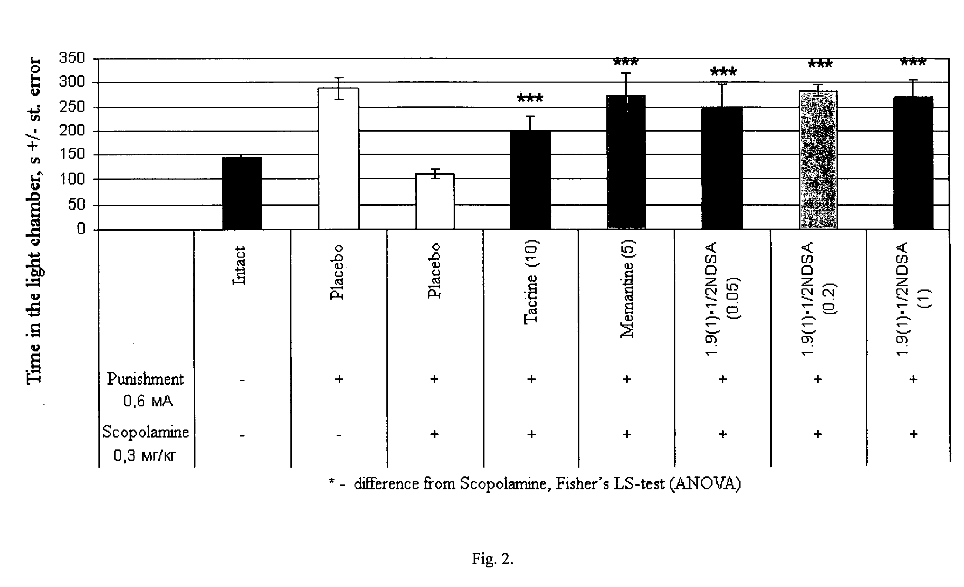

**Fig. 2****.** Enhancement of memory disturbed by Scopolamine in male mice of BALB/c line under the influence of ingredient **1.9(1)**·**1/2NDSA** and reference substances (Tacrine and Memantine) in test «Passive avoidance of mice in the shuttle chamber». The time, animals spent in the light chamber. The dosages of ligands in mg/kg are mentioned in brackets.

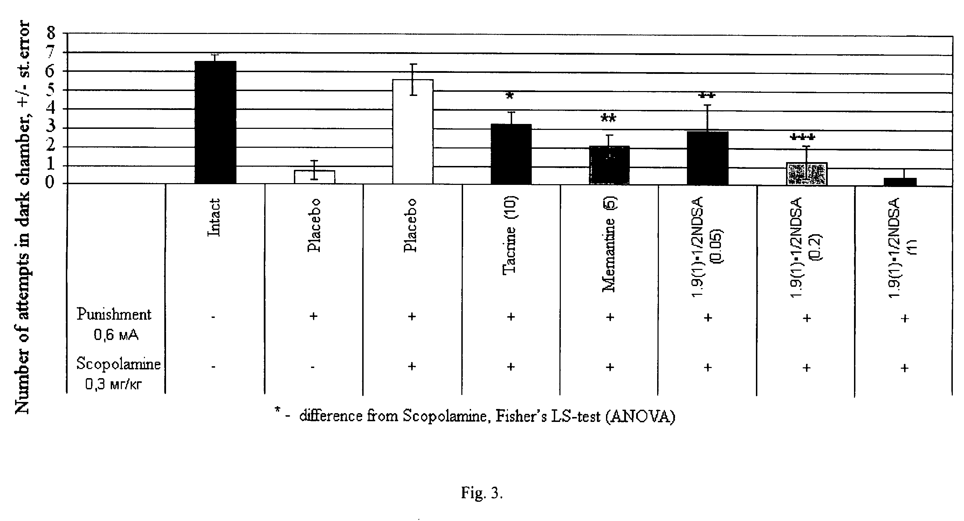

**Fig. 3****.** Enhancement of memory disturbed by Scopolamine in male mice of BALB/c line under the influence of ingredient **1.9(1)·1/2NDSA** and reference substances (Tacrine and Memantine) in test «Passive avoidance of mice in the shuttle chamber». The number of entries into the dark chamber. The dosages of ligands in mg/kg are mentioned in brackets.

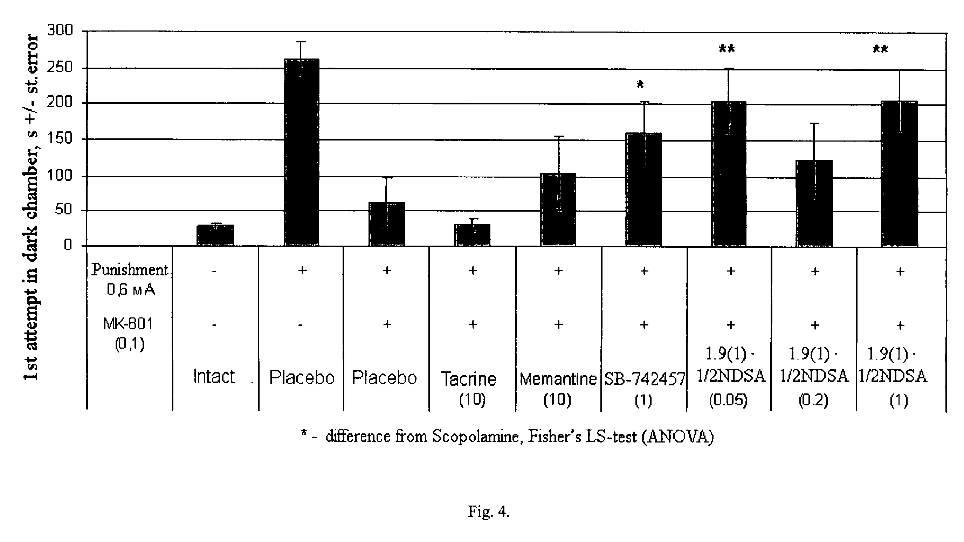

**Fig. 4****.** Enhancement of memory disturbed by MK-801 in male mice of BALB/c line under the influence of ingredient **1.9(1)·1/2NDSA** and reference substances (Tacrine and Memantine) in test «Passive avoidance of mice in the shuttle chamber». The time, after which animals do the first entry into the dark chamber. The dosages of ligands in mg/kg are mentioned in brackets.

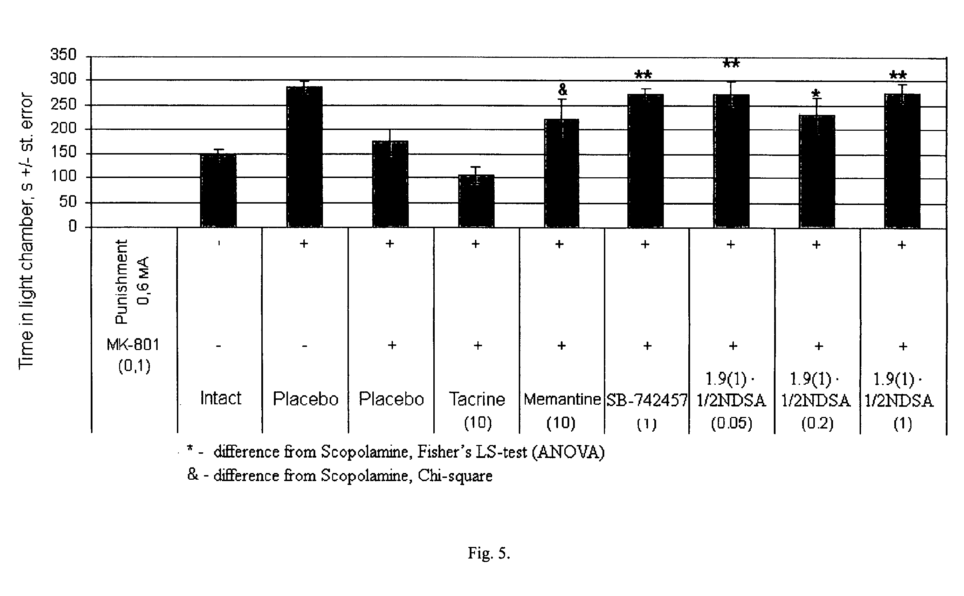

**Fig. 5****.** Enhancement of memory disturbed by MK-801 in male mice of BALB/c line under influence of ingredient **1.9(1)**·**1/2NDSA** and reference substances (Tacrine and Memantine) in test «Passive avoidance of mice in the shuttle chamber». The time, the animals spent in the light chamber. The dosages of ligands in mg/kg are mentioned in brackets.

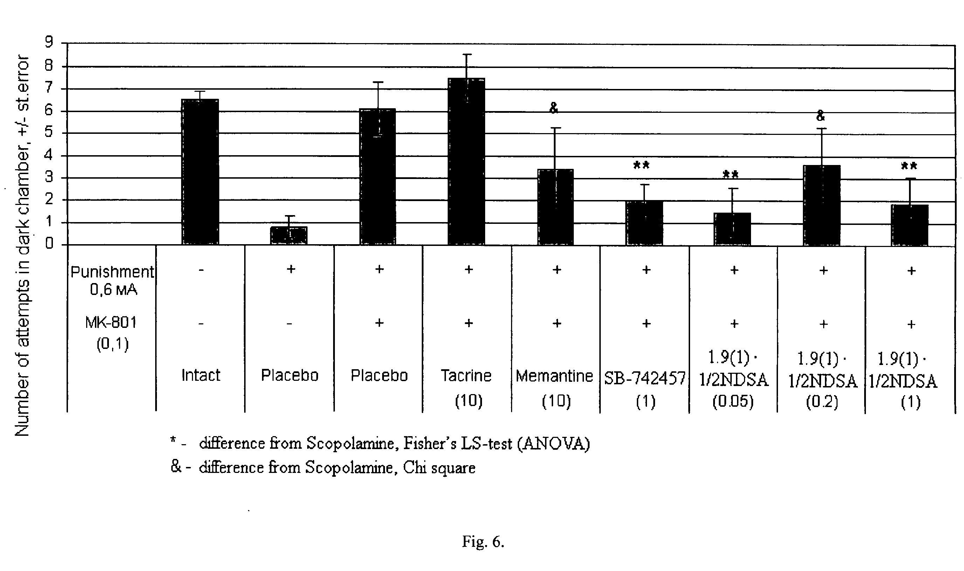

**Fig. 6**. Enhancement of memory disturbed by MK-801 in male mice of BALB/c line under influence of ingredient **1.9(1)·1/2NDSA** and reference substances (Tacrine and Memantine) in test «Passive avoidance of mice in the shuttle chamber». The number of entries into the dark chamber. The dosages of ligands in mg/kg are mentioned in brackets.

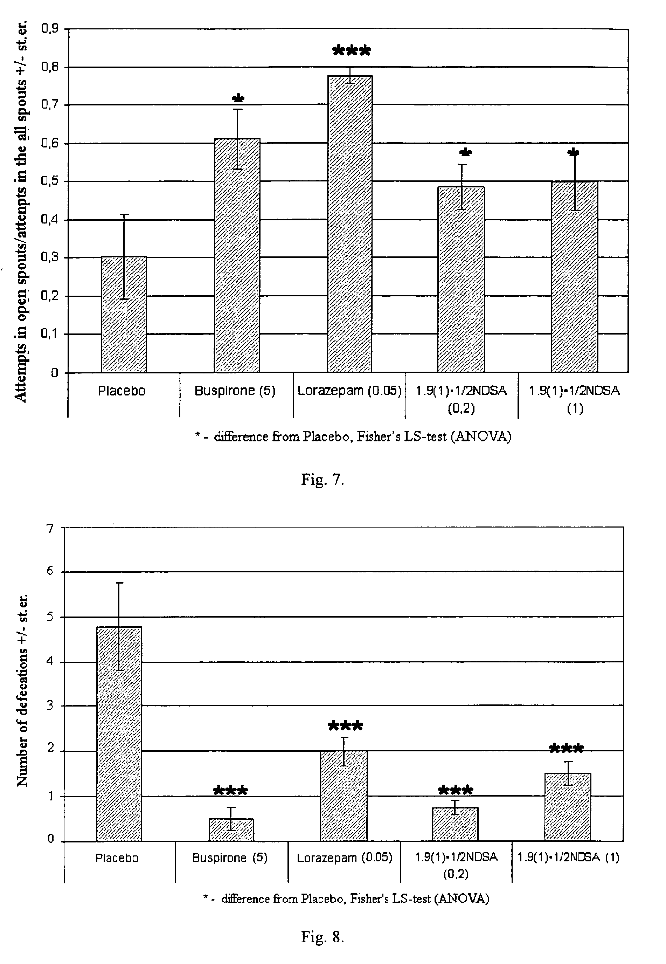

**Fig. 7****.** Male mice behaviour of BALB/c line under influence of ingredient **1.9 (1) ·1/2NDSA** and reference substances (Buspirone and Lorazepam) in test «Mice behaviour in the elevated plus maze». The ratio of the number of entries into the open arms to the number of entries into all arms. The dosages of ligands in mg/kg are mentioned in brackets.
**Fig. 8**. Male mice behaviour of of BALB/c line under influence of ingredient **1.9 (1) ·1/2NDSA** and reference substances (Buspirone and Lorazepam) in test «Mice behaviour in the elevated plus maze». The number of defecations. The difference from the group of animals receiving Scopolamine at p<0.05, *** - at p<0.001.e dosages of ligands in mg/kg are mentioned in brackets.

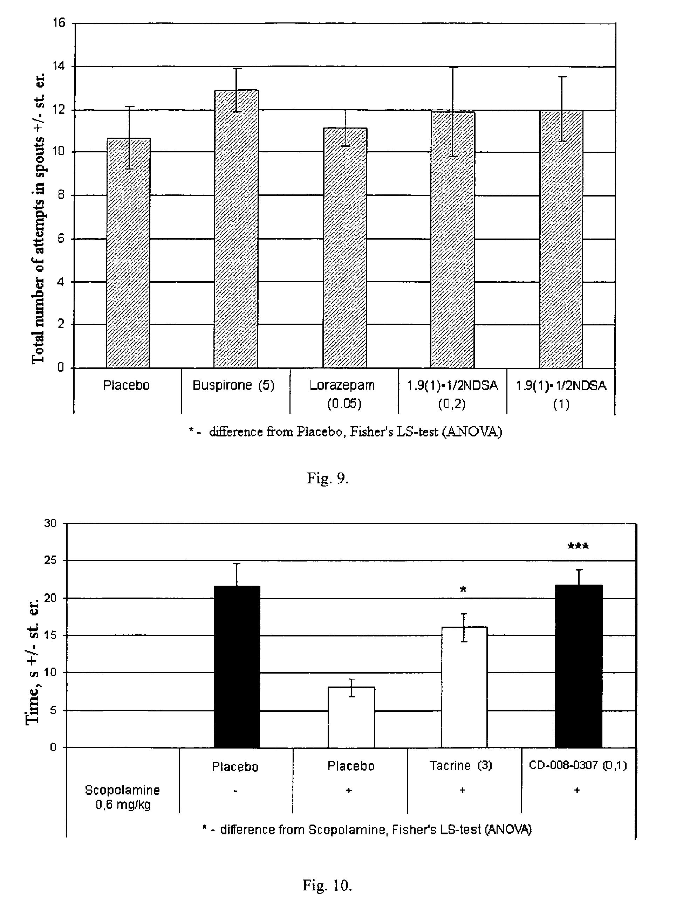

**Fig. 9****.** Male mice behaviour of of BALB/c line under influence of ingredient **1.9 (1) ·1/2NDSA** and reference substances (Buspirone and Lorazepam) in test «Mice behaviour in the elevated plus maze». The whole number of entries into the arms. The dosages of ligands in mg/kg are mentioned in brackets.
**Fig. 10****.** Time spent by mice in area of the platform after two days training in Morris water maze. The dosages of ligands in mg/kg are mentioned in brackets. The difference from the group of animals received Scopolamine: * - p<0,05; *** p<0,001, ANOVA LS-Fisher's test. CD-008-0307 corresponds to ingredient **1.1.2(4-2).**

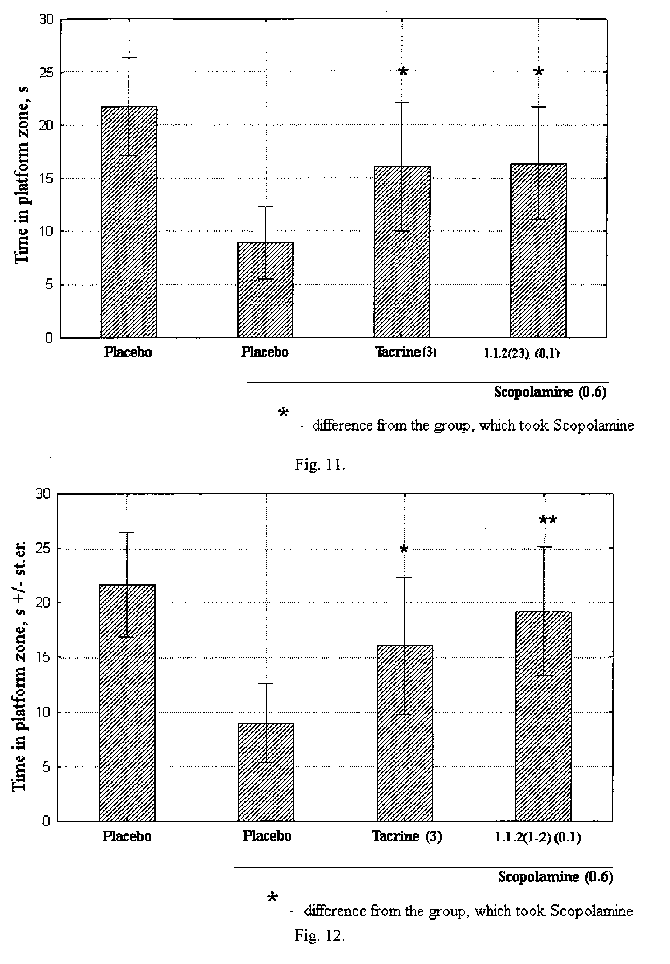

**Fig. 11****.** Test data for **1.1.2(2-3)** ligand in "Mice training in Morris water maze" test (single administration of ligand in dose of 0,1 mg/kg);
**Fig. 12****.** Test data for **1.1.2(1-2)** ligand in "Mice training in Morris water maze" test (single administration of ligand in dose of 0,1 mg/kg);

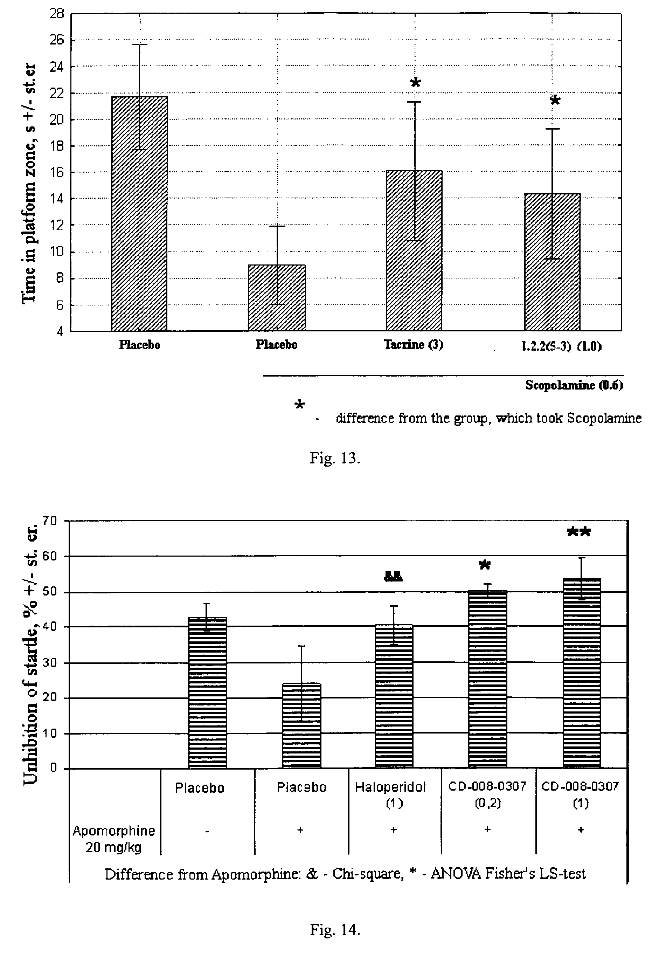

**Fig. 13****.** Test data for **1.2.2(5-3)** ligand in "Mice training in Morris water maze" test (single administration of ligand in dose of 0,1 mg/kg);
**Fig. 14****.** Influence of ligands on prepulse inhibition of the startle response (Prepulse inhibition of the Startle Response) in reply to acoustic stimulus. The dosages of ligands in mg/kg are mentioned in brackets. The difference from the group of animals received placebo: * - by LS-Fisher's test (Fisher's test); & - by Chi-squared test. CD-008-0307 corresponds to ingredient **1.1.2(4-2).**

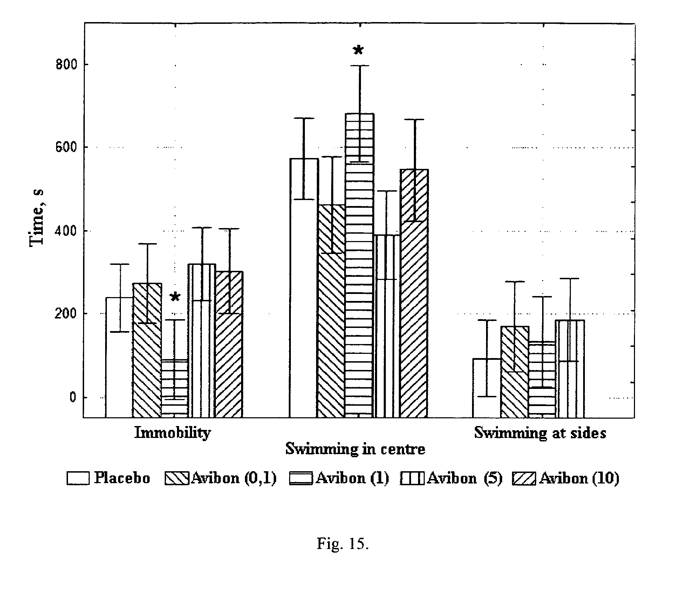

Fig. 15. Duration of depressively-like behaviour and swimming of mice in the centre and on pool periphery in test Porsolta (average value ± standard error) after administration of 1 mg/kg dose of **1.2.2 (5-1)** ligand (Avibon) during 4 days. Number in brackets is dose of ligand in mg/kg. Difference from the group receiving placebo: * - p <0,05.

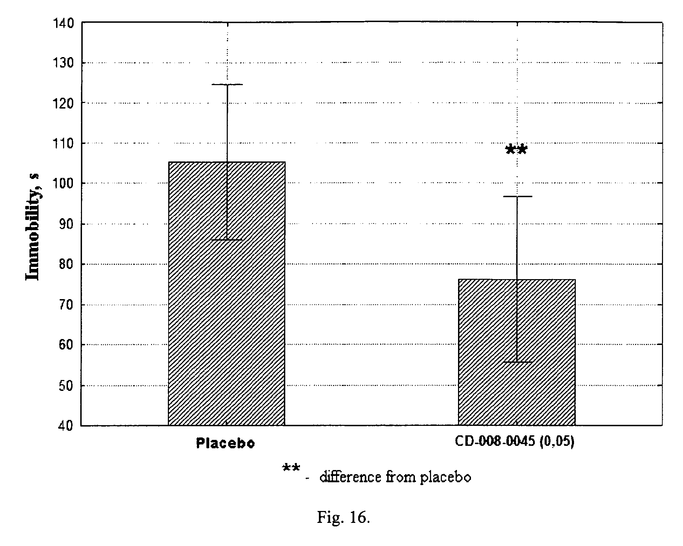

**Fig. 16****.** Test results for **1.1.2(1-2)** ligand (CD-008-0045) in tail suspention test.

Below the invention is described by means of specific examples, which illustrate but not limit scope of the invention.

**Example 1. A.** General method for preparation of substituted 1,2,3,4-tetrahydro-1H-pyrido[4,3-b]indoles of general formula **1.1.1 (R3 = H)** and 1,2,3,4,5,6-hexahydroazepino[4,3-b]indoles of general formula **1.2.1 (R3 = H)**. 20 g Of the proper vinyl derivate of general formula 2 is dissolved in 980 ml of ethanol in 21 flask. The flask is filled with argon and during 30 min argon is passed through the solution. Then 980 mg of PtO₂ is introduced into the flask in the current of argon, and hydrogen is passed through the solution for 24 hs at room temperature. The completeness of the reaction is controlled by LCMS. After that PtO₂ is filtered off through celit, the filtrate is evaporated. It gives compounds of the general formula **1**, yield 98-99.5%, among them: 2-methyl-5-phenethyl-1,2,3,4-tetrahydro-1H-pyrido[4,3-b]indole **1.1.2(1-1),** LCMS: m/z 291 [M+H], C₂₀H₂₂N₂, mol. weight 290,41; 2-methyl-5-[2-(4-methylphenyl)ethyl]-1,2,3,4-tetrahydro-1H-pyrido[4,3-b]indole **1.1.2(1-3),** LCMS: m/z 319 [M+H], C₂₂H₂₆N₂, mol. weight 318,47; 2,8-dimethyl-5-phenethyl-1,2,3,4-tetrahydro-1H-pyrido[4,3-b]indole **1.1.2(1-2),** LCMS: m/z 305 [M+H], C₂₁H₂₄N₂, mol. weight 304, 44; ¹H NMR (DMSO-d₆, 400 MHz) δ 7,30 (d, *J=8,0 Hz*, 1H), 7,26-7,19 (m, 3H), 7,11 (s, 1H), 7,089-7,068 (m, 2H), 6,88 (dd, *J₁=9,2 Hz*, *J₂=0,8 Hz,* 1H), 4,20 (t, *J=7,2 Hz,* 2H), 3,45 (s, 2H), 2,91 (t, *J=7,2 Hz,* 2H), 2,57 (t, *J=5,6 Hz*, 2H), 2,45 (t, *J=6,4 Hz*, 2H), 2,36 (2s, 6H); 2-methyl-8-methoxy-5-phenethyl-1,2,3,4-tetrahydro-1H-pyrido[4,3-b]indole **1.1.2(1-4),** LCMS: m/z 305 [M+H], C₂₁H₂₄N₂O, mol. weight 320, 44; 2-methyl-8-trifluoromethyl-5-[2-(4-methylphenyl)ethyl]-1,2,3,4-tetrahydro-1H-pyrido[4,3-b]indole **1.1.2(1-19),** LCMS: m/z 373 [M+H], C₂₂H₂₃F₃N₂, mol. weight 372, 44; 2,8-dimethyl-5-[2-(4-N,N-dimethylaminophenyl)ethyl]-1,2,3,4-tetrahydro-1H-pyrido[4,3-b]indole **1.1.2(1-11),** LCMS: m/z 348 [M+H], C₂₃H₂₉N₃ mol. weight 347,51; 2,8-dimethyl-5-[2-(4-methoxyphenyl)ethyl]-1,2,3,4-tetrahydro-1H-pyrido[4,3-b]indole **1.1.2(1-13),** LCMS: m/z 335 [M+H], C₂₂H₂₆N₂O, mol. weight 334,47; 2,8-dimethyl-5-[2-(4-fluorophenyl)ethyl]-1,2,3,4-tetrahydro-1H-pyrido[4,3-b]indole **1.1.2(1-15),** LCMS: m/z 323 [M+H], C₂₁H₂₃FN₂, mol. weight 322, 43; ¹H NMR (DMSO-d₆, 400 MHz) δ 7.34-7.32 (m,1H), 7.18 (s 1H), 7,12-6.93 (m, 5H), 4.26-4.19 (m, 2.5), 3.33 (m, 2H), 2.92-2.88 (m, 2H), 2.85 (s, 3H), 2.47 (m, 2H), 2.35(m, 3H); 2,8-dimethyl-5-[2-(4-trifluoromethylphenyl)ethyl]-1,2,3,4-tetrahydro-1H-pyrido[4,3-b]indole 1.1.2(1-17) LCMS: m/z 373 [M+H], C₂₂H₂₃F₃N₂, mol. weight 372, 44; ¹H NMR (DMSO-d₆, 400 MHz) δ 7.60-7.78 (m, 2H), 7.37-7.34 (m, 3H), 7.20 (m, 1H), 6.96-6.94 (m, H), 4.31 (m, 2H), 3.36 (m, 2H), 3.02 (m, 2H), 2.84 (s, 3H), 2.36 (s, 3H); 2-methyl-5-[2-(4-methylphenyl)ethyl]-8-fluoro-1,2,3,4-tetrahydro-1H-pyrido[4,3-b]indole **1.1.2(1-20)** LCMS: m/z 323 [M+H], C₂₁H₂₃FN₂, mol. weight 322, 43; 2-methyl-8-trifluoromethyl-5-phenethyl-1,2,3,4-tetrahydro-1H-pyrido[4,3-b]indole **1.1.2(1-7),** LCMS: m/z 359 [M+H], C₂₁H₂₁F₃N₂, mol. weight 394,87; ¹H NMR (DMSO-d₆, 400 MHz) δ 7.72-7.59 (m, 2H), 7.34-7.05 (m, 6H), 4.33-4.29 (m, 2H), 3.52 (s, 2H), 2.96-2.92 (m, 2H), 2.60-2.57 (m, 2H), 2.36(s, 3H); 2-methyl-6-phenethyl-1,2,3,4,5,6-hexahydroazepino[4,3-b]indole **1.2.2(1-1),** LCMS: m/z 305 [M+H], C₂₁H₂₄N₂, mol. weight 304,44; 2,9-dimethyl-6-phenethyl-1,2,3,4,5,6-hexahydroazepino[4,3-b]indole **1.2.2(1-2),** LCMS: m/z 319 [M+H], C₂₂H₂₆N₂, mol. weight 318,47; 2-methyl-9-methoxy-6-phenethyl-1,2,3,4,5,6-hexahydroazepino[4,3-b]indole **1.2.2(1-4),** LCMS: m/z 335 [M+H], C₂₂H₂₆N₂O, mol. weight 334,47; 2-methyl-6-phenethyl-9-fluoro-1,2,3,4,5,6-hexahydroazepino[4,3-b]indole **1.2.2(1-5),** LCMS: m/z 323 [M+H], C₂₁H₂₃FN₂, mol. weight 322,43; 2-methyl-9-trifluoromethyl-6-phenethyl-1,2,3,4,5,6-hexahydroazepino[4,3-b]indole **1.2.2(1-7),** LCMS: m/z 373 [M+H], C₂₂H₂₃F₃N₂, mol. weight 372,44; 2,9-dimethyl-6-[2-(4-methylphenyl)ethyl]-1,2,3,4,5,6-hexahydroazepino[4,3-b]indole **1.2.2(1-9),** LCMS: m/z 333 [M+H], C₂₃H₂₈N₂, mol. weight 332,49; 2,9-dimethyl-6-[2-(4-methoxyphenyl)ethyl]-1,2,3,4,5,6-hexahydroazepino[4,3-b]indole **1.2.2(1-10),** LCMS: m/z 349 [M+H], C₂₃H₂₈N₂O, mol. weight 348,49; 2,9-dimethyl-6-[2-(4-fluorophenyl)ethyl]-1,2,3,4,5,6-hexahydroazepino[4,3-b]indole **1.2.2(1-12),** LCMS: m/z 337 [M+H], C₂₂H₂₅FN₂, mol. weight 336,46; 2,9-dimethyl-6-[2-(4-trifluoromethylphenyl)ethyl]-1,2,3,4,5,6-hexahydroazepino[4,3-b]indole **1.2.2(1-13)** LCMS: m/z 387 [M+H], C₂₃H₂₅F₃N₂, mol. weight 386,46; 2-methyl-6-[2-(4-methylphenyl)ethyl]-9-fluoro-1,2,3,4,5,6-hexahydroazepino[4,3-b]indole **1.2.2(1-15)** LCMS: m/z 337 [M+H], C₂₂H₂₅FN₂, mol. weight 336,46 and other analogous compounds represented in Table 3.

**B.** Method for preparation of 2,8-dimethyl-5-[2-(4-methylpyridin-3-yl)ethyl]-1,2,3,4-tetrahydro-1H-pyrido[4,3-b]indole bis-methylsulfonate **1.1.2(4-4)·2CH₃SO₃H.** 450 Mg (1,41 mmol) of 2,8-dimethyl-5-[2-(4-methylpyridin-3-yl)ethyl]-1,2,3,4-tetrahydro-1H-pyrido[4,3-b]indole **1.1.2(4-1)** is dissolved in 60 ml of acetone. 200 Mkl (271 mg, 2.82 mmol) of methanesulfonic acid is added to the prepared solution. The precipitated in several minutes solid is filtered off, washed with acetone and dried *in vacuo.* It gives ligand **1.1.2(4-4)·2CH₃SO₃H** as white crystalline compound. ¹H NMR (DMSO-D₆, 400 MHz) δ 10,03 (br.s, 1H), 8,58 (d, *J* = 1,6 Hz, 1H), 8,20 (dd, *J₁* = 8,0 Hz, *J₂* = 1,6 Hz, 1H), 7,79 (d, *J* = 8,0 Hz, 1H), 7,35 (d, *J* = 8,4 Hz, 1H), 7,21 (s, 1H), 6,95 (d, *J* = 8,4 Hz, 1H), 4,58 (d, *J* = 14,0 Hz, 1H), 4,38 (t, *J* = 6,8 Hz, 2H), 4,26 (d, *J* = 14,0 Hz, 1H), 3,76 (br.s, 1H), 3,14 (t, *J* = 6,8 Hz, 2H), 3,09 (m, 2H), 3,00 (s, 3H), 2,65 (s, 3H), 2,36 (s, 3H), 2,35 (s, 6H).

**C.** Method for preparation of 2,8-dimethyl-5-[2-(4-methylpyridin-3-yl)ethyl]-1,2,3,4-tetrahydro-1H-pyrido[4,3-b]indole naphthalene-1,5-disulfonate **1.1.2(4-5)·1/2NDSA.** Solution of 464 mg (1,39 mmol) of sodium 1,5-naphthalenedisulfonate in 15 ml of distilled water is added to solution of 545 mg of 2,8-dimethyl-5-[2-(4-methylpyridin-3-yl)ethyl]-1,2,3,4-tetrahydro-1H-pyrido[4,3-b]indole dihydrochloride in 15 ml of water. The solid precipitated in several minutes is filtered off, washed with water and dried *in vacuo.* It gives ligand **1.1.2(4-5)**·**1/2NDSA** as white crystalline compound. ¹H NMR (DMSO-D₆, 400 MHz) δ 9,89 (br.s, 1H), 8,85 (d, *J* = 8,8 Hz, 2H), 8,54 (d, *J* = 1,6 Hz, 1H), 8,12 (dd, *J₁* = 8,4 Hz, *J₂* = 1,6 Hz, 1H), 7,91 (d, *J =* 7,2 Hz, 2H), 7,66 (d, *J* = 8,4 Hz, 1H), 7,36 (dd, *J₁* = 8,8 Hz, *J₂* = 7,2 Hz, 2H), 7,32 (d, *J* = 8,4 Hz, 1H), 7,20 (s, 1H), 6,94 (d, *J* = 8,4 Hz, 1H), 4,57 (d, *J* = 14,0 Hz, 1H), 4,32 (q, *J* = 7,2 Hz, 2H), 4,23 (d, *J* = 14,0 Hz, 1H), 3,72 (br.s, 1H), 3,03 (m, 4H), 2,98 (s, 3H), 2,60 (s, 3H), 2,36 (s, 3H).

**Example 2.** General method for preparation of hydroxy derivatives of 1,2,3,4-tetrahydro-1H-pyrido[4,3-b]indoles of general formula **1.1.1 (R3 = OH)** and 1,2,3,4,5,6-hexahydroazepino[4,3-b]indoles of general formula **1.2.1 (R3 = OH).** 6 Mmol of dry powdered K₃PO₄ and 4 mmol of racemic styrene oxide **4** are added to solution of 3 mmol of compound 3 in 15 ml of dry DMF and reaction mixture is stirred vigorously in argon atmosphere for 12 hs at 70°C. The reaction is monitored by LCMS. After the completeness of the reaction the reaction mixture is poured into 150 ml of water and extracted three times with ethyl acetate. The extract is washed with diluted K₂CO₃ solution, dried over Na₂SO₄ and evaporated *in vacuo.* The prepared product is recrystallized from proper solvent, for example, ethyl acetate. It gives compounds **1.1.1 (R3** = **OH)** and **1.2.1 (R3** = **OH),** among them: 2-(2-methyl-1,2,3,4-tetrahydropyrido[4,3-b]indol-5-yl)-1-phenyl-ethanol **1.1.2(1-8),** LCMS: m/z 307 [M+H], C₂₀H₂₂N₂O, mol. weight 306,41; ¹H NMR (400 MHz, DMSO-d₆) δ d 7.42-7.40 (m, 1H), 7.33-7.28 (m, 6H), 7.07-6.95 (m, 2H), 5.59 (s, 1H), 4.87 (m, 1H), 4.18-4.06 (m, 2H), 3.49 (s, 2H), 2.76-2.72 (m, 1H), 2.63-2.58 (m, 2H), 2.54-2.38 (m, 3H); ¹³C NMR (100 MHz, DMSO-d₆) δ 22.39, 45.50, 50.77, 51.41, 52.17, 71.63, 106.76, 109.68, 117.02, 118.40, 120.08, 125.17, 125.98, 127.26, 128.06, 134.41, 136.28, 143.28; 2,8-dimethyl-5-[(2-phenyl-2-hydroxy)ethyl]-1,2,3,4-tetrahydro-1H-pyrido[4,3-b]indole **1.1.2(1-10),** LCMS: m/z 321 [M+H], C₂₁H₂₄N₂O, mol. weight 320,44; ¹³C NMR (100 MHz, DMSO-d₆) δ d 21.37, 22.32, 45.36, 51.01, 51.50, 52.22, 71.80, 105.94, 109.62, 117.05, 121.82, 125.51, 126.16, 127.07, 128.24, 134.36, 134.94, 143.40; 2-methyl-5-[(2-hydroxy-2-phenyl)ethyl]-8-fluoro-1,2,3,4-tetrahydro-1H-pyrido[4,3-b]indole, **1.1.2(1-6),** LCMS: m/z 325 [M+H], C₂₀H₂₁FN₂O, mol. weight 324,40; ¹³C NMR (100 MHz, DMSO-d₆) δ d 22.50, 45.43, 50.85, 51.22, 52.03, 71.67, 101.88, 102.11, 107.01, 107.50, 110.58, 125.98, 128.04, 133.03, 136.54, 143.14, 155.64, 157.93; 6-[(2-hydroxy-2-phenyl)ethyl]-2,9-dimethyl-1,2,3,4,5,6-hexahydroazepino[4,3-b]indole **1.2.2(1-8),** LCMS: m/z 335 [M+H], C₂₂H₂₆N₂O, mol. weight 334,47 and other analogous compounds represented in Table 3.

**Example 3.** General method for preparation of substituted 1,2,3,4-tetrahydro-1H-pyrido[4,3-b]indoles of general formula **1.3** and 1,2,3,4,5,6-hexahydroazepino[4,3-b]indoles of general formula **1.4.**

**A.** 4.5 Mmol of NaH is added at stirring to solution of 3 mmol of compound **3** in 6 ml of dry DMF, previously cooled to -50-60°C in argon atmosphere, the temperature of the reaction mixture is raised to 20°C, if required, bleeding excessive hydrogen with a needle. After effervescence of hydrogen is completed, the russet mixture is stirred for additional 15-20 min at 20°C. After cooling to -50-60°C, 3.2 mmol of cinnamyl chloride **5** is added at once. The mixture is warmed to room temperature and stirred for 12 hs at 20°C. The completeness of reaction is controlled by LCMS. Then the reaction mixture is evaporated *in vacuo,* and the residue comprising the mixture of two isomers is subjected to chromatogrphy on silica gel. It gives compounds **1.3.1, 1.3.2, 1.4.1, 1.4.2**, among them:

E-2,8-dimethyl-5-(3-phenylallyl)-2,3,4,5-tetrahydro-1H-pyrido[4,3-b]indole **1.3.1(1),** LCMS: m/z 317 [M+H], C₂₂H₂₄N₂, mol.weight 316,45; ¹H NMR(400 MHz, DMSO-d₆) δ 7.45-7.20 (m, 8H), 6.88-6.86 (m, 1H), 6.32 (m, 2H), 4.80 (m, 2H), 3.49 (m, 2H), 2.79-2.70 (m, 4H), 2.40 (s, 3H), 2.35 (s, 3H); Z-2,8-dimethyl-5-(3-phenylallyl)-2,3,4,5-tetrahydro-1H-pyrido[4,3-b]indole 1.3.2(1), LCMS: m/z 317 [M+H], C₂₂H₂₄N₂, mol.weight 316,45; ¹H NMR (400 MHz, DMSO-d₆) δ 7.34-7.13 (m, 8H), 6.87-6.85 (m, 1H), 6.44-6.33 (m, 2H), 4.64 (m, 2H), 3.65-3.55 (m, 1H), 3.32-3.47 (m, 2H), 2.95-2.75 (m, 4H), 2.43 (s, 3H), 2.35 (s, 3H); E-2-methyl-5-(3-phenylallyl)-8-fluoro-2,3,4,5-tetrahydro-1H-pyrido[4,3-b]indole **1.3.1(2),** LCMS: m/z 321 [M+H], C₂₁H₂₁FN₂, mol.weight 320,41; Z-2-methyl-5-(3-phenylallyl)-8-fluoro-2,3,4,5-tetrahydro-1H-pyrido[4,3-b]indole **1.3.2(2),** LCMS: m/z 321 [M+H], C₂₁H₂₁FN₂, mol.weight 320,41 and other analogous compounds represented in Table 3.

**B.** Solution of 3 mmol of mixture of compounds 1.3.1 and **1.3.2** or compounds **1.4.1** and **1.4.2** (or individual compounds **1.3.1, 1.3.2, 1.4.1, 1.4.2)** in 30 ml of ethanol is hydrogenated over 100 mg of PtO2 (1 atm, 18 hs, 30-40°C, LCMS and TLC monitoring). After the reaction is over the product is purified by column chromatogrphy on silica gel impregnated with triethylamine, eluent - hexane-chlorofom 1:1 mixture. It gives compounds **1.3.3, 1.4.3,** among them: 2,8-dimethyl-5-(3-phenylpropyl)-2,3,4,5-tetrahydro-1H-pyrido[4,3-b]indole **1.3.3(1),** LCMS: m/z 319 [M+H], C₂₂H₂₆N₂, mol.weight 318,47; 2-methyl-5-(3-phenylpropyl)-8-fluoro-2,3,4,5-tetrahydro-1H-pyrido[4,3-b]indole **1.3.3(7),** LCMS: m/z 323 [M+H], C₂₁H₂₃FN₂, mol.weight 322,43; 2,9-dimethyl-6-(3-phenylpropyl)-1,2,3,4,5,6-hexahydroazepino[4,3-b]indole **1.4.3(1),** LCMS: m/z 333 [M+H], C₂₃H₂₈N₂, mol.weight 332,49 and other analogous compounds represented in Table 3.

**Example 4.** General method for preparation of substituted 6-sulfonyl-1,2,3,4,5,6-hexahydroazepino[4,3-b]indoles hydrochlorides of general formula **1.8.** 1,5 Mmol of 1,2,3,4,5,6-hexahydroazepino[4,3-b]indole of formula **3** is added carefully to suspension of 4,5 mmol of 60% NaH in 5 ml of dry DMF at cooling in argon atmosphere. The solution of 2,3 mmol of sulfochloride **6** in 5 ml of DMF is added to the reaction mixture after stirring it at cooling for 1 h, the resultant mixture is stirred for ∼ 1,5 hs at room temperature (TLC-control, eluent - ethyl acetate-hexane-triethylamine 7:3:1mixture). Upon completeness of the reaction acetic acid is added drop by drop in order to neutralize excess of NaH. Product is extracted with ethyl acetate (3 x 50 ml), organic layer is washed with water carefully (3 x 50 ml), dried over Na₂SO₄, evaporated to dryness *in vacuo.* The product is separated by means of column chromatogrphy (eluent - ethyl acetate:hexane 7:3 mixture). The obtained oily compound is dissolved in minimal amount of acetone or ethyl acetate and transformed into hydrochloride by adding consecutivly enough amount of ether and solution of HCl in dioxane (100 mg/ml). It gives 6-sulfonyl-1,2,3,4,5,6-hexahydroazepino[4,3-b]indoles of general formula **1.8** as hydrochlorides, among them: 2,9-dimethyl-6-[3-(trifluoromethyl)phenylsulfonyl]-1,2,3,4,5,6-hexahydroazepino[4,3-b]indole hydrochloride **1.8.1(8),** ¹H NMR (DMSO-D₆, 400 MHz) δ 10,85 (s, 1H), 8,12 (m, 3H), 7,94 (d, *J* = 8,5 *Hz*, 1H), 7,82 (m, 1H), 7,51 (s, 1H), 7,19 (d, J = *8,8 Hz,* 1H), 4,59 (d, J = *14,4 Hz*, 1H), 4,44 (m, 1H), 3,62-3,48 (br.s, 2H), 3,44-3,33 (br.s, 2H), 2,79 (s, 3H), 2,37 (s, 3H), 2,20-2,07 (br.s, 1H), 2,07-1,96 (br.s, 1H); 6-[(3,4-difluorophenyl)sulfonyl]-2,9-dimethyl-1,2,3,4,5,6-hexahydroazepino[4,3-b]indole hydrochloride **1.8.1(6),** ¹H NMR (DMSO-D₆, 400 MHz) δ 11,16-10,62 (br.s, 1H), 8,08 (m, 1H), 7,92 (m, 1H), 7,75 (m, 1H), 7,65 (m, 1H), 7,5 (s, 1H), 7,18 (d, *J* = *8,8 Hz*, 1H), 4,58 (d, *J* = *16,0 Hz*, 1H), 4,44 (m, 1H), 3,61-3,56 (m, 1H), 3,52-3,29 (br.s, 3H), 2,8 (s, 3H), 2,38 (s, 3H), 2,23-2,10 (br.s, 1H), 2,07-1,96 (br.s, 1H); 6-[(3-fluoro-4-methylphenyl)sulfonyl]-2,9-dimethyl-1,2,3,4,5,6-hexahydroazepino[4,3-b]indole hydrochloride **1.8.1(7),** ¹H NMR (DMSO-D₆, 400 MHz) δ 11,01-10,74 (br.s, 1H), 7,93 (m, 1H), 7,66 (dd, *J₁* = *9,2 Hz*, *J₂* = *1,2*, 1H), 7,61 (dd, *J₁ = 8, 0 Hz, J₂ =2, 0 Hz,* 1H), 7,50 (m, 2H), 7,16 (d, *J* = *8,4 Hz,* 1H), 4,58 (d, *J* = *14,8 Hz*, 1H), 4,43 (m, 1H), 3,62 (m, 1H), 3,53-3,36 (br.s, 3H), 2,9 (d, *J* = *4,4 Hz,* 3H), 2,37 (s, 3H), 2,24 (s, 3H) 2,20-2,09 (br.s, 1H), 2,07-1,96 (br.s, 1H); 6-[(4-*tert*-butylphenyl)sulfonyl]-2,9-dimethyl-1,2,3,4,5,6-hexahydroazepino[4,3-b]indole hydrochloride **1.8.1(13),** ¹H NMR (DMSO-D₆, 400 MHz) δ 10,85-10,60 (br.s, 1H), 7,94 (d, *J* = *8,8 Hz,* 1H), 7,79 (d, *J* =*8,4 Hz,* 2H), 7,59 (d, *J* = *8,4 Hz,* 2H), 7,50 (s, 1H), 7,16 (d, *J* = *9,2 Hz,* 1H), 4,59 (d, *J* = *14,8 Hz*, 1H), 4,43 (m, 1H), 3,64-3,54 (br.s, 1H), 3,53-3,36 (br.s, 3H), 2,78 (d, *J* = *4,0 Hz,* 3H), 2,37 (s, 3H), 2,18-2,07 (br.s, 1H), 2,06-1,95 (br.s, 1H), 1,22 (s, 9H); 6-[(3-chloro-4-methoxyphenyl)sulfonyl]-2,9-dimethyl-1,2,3,4,5,6-hexahydroazepino[4,3-b]indole hydrochloride **1.8.1(15),** ¹H NMR (DMSO-D₆, 400 MHz) δ 10,98 (s, 1H), 7,76 (d, *J* = *2 Hz,* 1H), 7,72 (dd, *J₁* =*8,8 Hz*, *J₂* = *2,0 Hz*, 1H), 7,62 (d, *J = 2, 0 Hz,* 1H), 7,58 (dd, *J₁* =*8,8 Hz*, *J₂* = *2,0 Hz*, 1H), 7,33 (d, *J* =*8,8 Hz,* 1H), 7,21 (d, *J =8, 8 Hz*, 1H), 7,10 (d, *J =8,4 Hz*, 1H), 4,67 (s, 2H), 3,94 (s, 3H), 3,90 (s, 3H), 2,93 (t, *J* = *7,2 Hz,* 2H), 2,65 (s, 3H), 2,52 (m, 2H), 2,22 (s, 3H), 1,82-1,71 (br.s, 2H) and other analogous compounds represented in Table 3.

**Example 5.** General method for preparation of compounds of general formula 1 in the form of hydrochlorides. 3 Ml (1,2 eq) of HCl dioxane solution (concentration of HCl in dioxane is 100 mg/ml) is added at stirring to solution 2 g of free base of compound 1 in 90 ml of acetone, stirring is continued for 15 minutes. The precipitated white solid is separated, washed twice with acetone and dried *in vacuo.* Product is recrystallzed from ethanol or isopropanol ( compound dries up from isopropanol very slowly ). It gives compounds **1** in the form of hydrochlorides. Salts are prepared with 75-87% yield, among them: 2,8-dimethyl-5-phenethyl-1,2,3,4-tetrahydro-1H-pyrido[4,3-b]indole hydrochloride **1.1.2(1-2),** LCMS: m/z 305 [M+H], C₂₁H₂₅ClN₂, mol. weight 340,90; ¹H NMR (DMSO-d₆, 400 MHz) δ 11.47 (br.s, 1H); 7.37 (d, J=13.2 Hz, 1H); 7.15-7.29 (m, 4H); 7.04-7.13 (m, 2H); 6.97 (d, J=13.2 Hz, 1H); 4.47 (d, 1H); 4.12-4.39 (m, 3H); 3.48-3.61 (m, 1H); 3.2-3.35 (m, 1H); 2.87-3.09 (m, 3H); 2.83 (d, 3H); 2.64 (d, 1H); 2.35 (s, 3H); ¹³C-NMR (D₂O 400 MHz,) δ 138.64, 134.67, 131.06, 128.99, 128.43, 127.9, 126.49, 124.73, 122.98, 126.4, 117.41, 109.66, 100.68, 49.93, 49.4, 44.53, 41.21, 40.41, 40.13, 35.73, 21.21, 18.85 and other analogous compounds represented in Table 3.

**Example 6.** Activity test for ligands of general formula **1** for ability to bind to α-adrenergic receptors.

**A.** Activity test for ligands of general formula **1** for ability to bind to adrenergic receptor α_{1A}. Screening of disclosed compounds for potential ability to interact with adrenergic receptor α_{1A} is carried out by method of radioligand binding. For this purpose membrane species from rat submaxillary glands (rat of Wistar breed) are prepared by homogenization of the latter in glass homogenizer with subsequent separation of plasmatic membranes from cell nuclei, mitochondrias and cell wreckages by differential centrifugation. Determination of investigated compounds' binding to α_{1A} receptor was carried out according to the method described in [Michel AD, Loury DN and Whiting R1. Identification of a single α1A-adrenoceptor corresponding to the α1A-subtype in rat submaxillary gland. Br J Pharmacol. 98:883-889, 1989]. In preferable embodiment membrane species are incubated with radioligand (0.25 nM [³H] Prazosin) in the presence of investigated compounds and without them for 60 minutes at 25°C in medium consisting of 50mM Tris-HCl, pH 7.4, 0.5mM EDTA. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane preparation with radioligand in the presence of 10 µM of Phentolamine. Prazosin is used as positive control. Tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$ wherein: *TA -* is overall radioactivity in the presence of radioligand only, *CA -* is radioactivity in the presence of radioligand and tested compound and *NA -* is radioactivity in the presence of radioligand and Phentolamine (10 µM).

Test results for some representatives of ligands of general formula **1** presented in Tables 4, 5 confirm their activity towards adrenergic α_{1A} receptors

**6-B.** Activity test for ligands of general formula **1** for binding to adrenergic receptor α_{1B}-Screening of disclosed compounds for potential ability to interact with adrenergic receptor α_{1B} is carried out by method of radioligand binding. For this purpose membrane species from rat liver (rat of Wistar breed) are prepared by means of liver homogenization in glass homogenizer with subsequent separation of plasmatic membranes from cell nuclei, mitochondrias and cell wreckages by differential centrifugation. Determination of investigated compounds' binding to α_{1B} receptor is carried out according to method described in [Garcia-Sainz JA, Romero-Avila MT, Hernandez RA, Macias-Silva M, Olivares-Reyes A and Gonzalez-Espinosa C. Species heterogeneity of hepatic α1A-, α1B-, and α1C-subtypes. Biochem Biophys Res Commun. 186:760-7676 1992]. In preferable embodiment the membrane preparations are incubated with radioligand (0.25 nM [³H] Prazosin) in the presence of investigated compounds and without them for 60 minutes at 25°C in the medium consisting of 50mM Tris-HCl, pH 7.4, 0.5mM EDTA. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane species with radioligand in the presence of 10 µM of Phentolamine. Prazosin is used as positive control. The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Phentolamine (10 µM).

Test results for some representatives of ligands of general formula **1** presented in Tables 4, 5 confirm their activity towards adrenergic α_{1B} receptors.

**6-C.** Activity test for ligands of general formula **1** for binding to adrenergic receptor α_{1D}. Screening of the disclosed compounds for potential ability to interact with adrenergic receptor α_{1D} is carried out by method of radioligand binding. For this purpose sDNA of human α_{1D} receptor is expressed in HEK-293 cells, as it was described in [TL Theroux, TA Esbenshade, RD Peavy and KP Minneman. Coupling efficiencies of human alpha 1-adrenergic receptor subtypes: titration of receptor density and responsiveness with inducible and repressible expression vectors. Mol Pharmacol.; 50: 1376-1387, 1996]. From the cells transfected in this way membrane species are prepared by homogenization of these cells in glass homogenizer with subsequent separation of plasmatic membranes from cell nuclei, mitochondrias and cell wreckages by differential centrifugation. Determination of investigated compounds' binding to α_{1D} receptor is carried out according to method described in [Kenny BA, Chalmers DH, Philpott PC and Naylor AM. Characterization of an α1D-adrenoceptor mediating the contractile response of rat aorta to noradrenaline. Br J Pharmacol. 115:981-9866 1995]. In the preferable embodiment the membrane species are incubated with radioligand (0.6 nM [³H] Prazosin) in the presence of investigated compounds and without them for 60 minutes at 25°C in the medium consisting of 50mM Tris-HCl, pH 7.4. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 20% of the overall binding is determined by incubation of membrane species with radioligand in the presence of 10 µM of Phentolamine. Prazosin is used as positive control. The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Phentolamine (10 µM).

Test results for some representatives of ligands of general formula **1** presented in Tables 4, 5 confirm their activity towards adrenergic α_{1D} receptors.

**6-D.** Activity test for ligands of general formula **1** for binding to adrenergic receptor α_{2A}. Screening of disclosed compounds for potential ability to interact with adrenergic receptor α_{2A} is carried out by method of radioligand binding. For this purpose sDNA of human α_{2A} receptor is expressed in Sf9 cells, as described in [Uhlen S, Porter AC and Neubig RR. The novel α2 adrenergic radioligand [3H]MK912 is α2C selective among human α2A, α2B and α2C adrenoceptors. J Pharmacol ExpTher. 271:1558-1565, 1994]. From the cells transfected in this way membrane species are prepared by homogenization of these cells in glass homogenizer with subsequent separation of plasmatic membranes from cell nuclei, mitochondrias and cell wreckages by differential centrifugation. Determination of investigated compounds' binding to α_{2A} receptor is carried out according to method described in [Uhlen S, Porter AC and Neubig RR. The novel α₂ adrenergic radioligand [3H]NM912 is α2C selective among human α2A, α2B and α2C adrenoceptors. J Pharmacol ExpTher. 271:1558-1565, 1994]. In the preferable embodiment the membrane species are incubated with radioligand (1 nM [³H] MK-912) without and in the presence of investigated compounds for 60 minutes at 25°C in the medium consisting of 50mM Tris-HCl, pH 7.4, 12.5mM MgCl₂., 2mM EDTA. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 5% of the overall binding is determined by incubation of membrane species with radioligand in the presence of 10 µM of WB-4101. Yohimbine is used as positive control. The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA* - is overall radioactivity in the presence of radioligand only, *CA -* is radioactivity in the presence of radioligand and tested compound and *NA -* is radioactivity in the presence of radioligand and WB-4101 (10 µM).

Test results for some representatives of ligands of general formula **1** presented in Tables 4, 5 confirm their activity towards adrenergic α_{2A} receptors.

**Table 4. Biological activity of 10 µM ligands of general formula 1 towards α-adrenergic receptors.**

| **Ligand** | α**-Adrenoceptor, % inhibition** | | | |
|---|---|---|---|---|
| | α_{1A} | α_{1B} | α_{1D} | α_{2A} |
| **1.1.2(1-2)·HCl** | 99 | 101 | 102 | 95 |
| **1.1.2(2-3)·2HCl** | 81 | 98 | 92 | 93 |

| Table 4 | | | | |
|---|---|---|---|---|
| **1.1.2(4-2) ·2HCl** | 97 | 101 | 98 | 95 |
| **1.2.2(2-2)** | 64 | 80 | 63 | 90 |
| **1.2.2(3-2)·2HCl** | 61 | 75 | 60 | 71 |
| **1.2.2(4-1)** | 80 | 85 | 68 | 79 |
| **1.2.2(5-2)** | 74 | 89 | 68 | 74 |
| **1.6.1(1)·3HCl** | 90 | 94 | 90 | 84 |
| **1.6.1(2)·3HCl** | 91 | 93 | 87 | 82 |
| **1.6.2(10) ·3HCl** | 86 | 92 | 86 | 92 |
| **1.6.2(11)** | 90 | 94 | 90 | 95 |
| **1.8.1(3) ·HCl** | 54 | 58 | 55 | 84 |
| **1.9(1)·HCl** | 70 | 70 | 77 | 100 |
| **1.9(1)·1/2NDSA** | 67(1 µM) | 62(3 µM) | 70(3 µM) | 73(0.3 µM) |

Efficiencies of some ligands' interactions of general formula 1 to α-adrenergic receptors are presented in Table 5, which demonstrate high activity of these compounds towards α-adrenergic receptors.

**Table 5. Interaction efficiencies of ligands of general formula 1 with α- adrenergic receptors.**

| **Ligand** | **Receptor** | **IC₅₀** | **Kᵢ** | **n_{H}** |
|---|---|---|---|---|
| | | **nM** | | |
| **1.1.2(1-3)** | α**_{1A}** | 46,7 | 18,9 | 1,26 |
| **1.1.2(2-3)·2HCl** | | 450 | 182 | 1.15 |
| **1.1.2(4-2)·2HCl** | | 136 | 55 | 1.12 |
| **1.9(1)·1/2NDSA** | | 510 | 206 | 1,08 |
| **1.1.2(1-3)** | α**_{1B}** | 17,0 | 9,4 | 1,13 |
| **1.1.2(2-3)·2HCl** | | 47 | 26 | 1.03 |
| **1.1.2(4-2)·2HCl** | | 87 | 48 | 1.54 |
| **1.9(1)·1/2NDSA** | | 1580 | 872 | 0,956 |
| **1.1.2(1-3)** | α**_{1D}** | 61,5 | 30,2 | 1,21 |
| **1.1.2(2-3)·2HCl** | | 282 | 139 | 1.18 |
| **1.1.2(4-2)·2HCl** | | 239 | 118 | 0.841 |
| **1.9(1)·1/2NDSA** | | 1330 | 656 | 1,05 |
| **1.1.2(1-3)** | α**_{2A}** | 4,72 | 1,77 | 1.12 |
| **1.9(1)·1/2NDSA** | | 123 | 46,1 | 1,13 |
| **1.1.2(2-3)·2HCl** | | 128 | 47.9 | 1.26 |
| **1.1.2(4-2)·2HCl** | | 286 | 107 | 0.828 |

**Example 7.** Activity test for ligands of general formula 1 for binding to dopamine receptors.

**7-A.** Activity test for ligands of general formula 1 for binding to dopamine receptors D₁. Screening of the disclosed compounds for potential ability to interact with dopamine receptor D₁ is carried out by method of radioligand binding. For this purpose sDNA of human D₁ receptor is expressed in CHO cells, as it was described in [Dearry A, Gingrich JA, Falardeau P, Fremeau RT Jr, Bates MD and Caron MG. Molecular cloning and expression of the gene for a human D1 dopamine receptor. Nature. 347:72-76, 1990]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D₁ receptor is carried out according to method described in [Zhou Q-Y, Grandy DK, Thambi L, Kushner JA, Van Tol HHM, Cone R, Pribnow D, Salon J, Bunzow JR and Civelli O. Cloning and expression of human and rat D1 dopamine receptors. Nature. 347:76-80, 1990]. In the preferable embodiment the membrane species are incubated together with radioligand (1.4 nM [³H] SCH-23390) in the presence of investigated compounds and without them for 120 minutes at 37°C in the medium consisting of 50mM Tris-HCl, pH 7.4, 1.4mM Ascorbic Acid, 0.001% BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane species with radioligand in the presence of 10 µM of (+)-Butaclamol. R(+)-SCH-23390 is used as positive control. The tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and (+)-Butaclamol.

Test results for some representatives of ligands of general formula **1** presented in Tables 6, 7, confirm their activity towards dopamine receptors D₁.

**7-B.** Activity test for ligands of general formula **1** for binding to dopamine receptors D_{2L}. Screening of the disclosed compounds for potential ability to interact with dopamine receptor D_{2L} is carried out by method of radioligand binding. For this purpose sDNA of human D_{2L} receptor is expressed in CHO cells, as described in [Hayes G, Biden TJ, Selbie LA and Shine J, Structural subtypes of the dopamine D2 receptor are functionally distinct. Expression of the clone D2A and D2B subtypes in heterologous cell line. Mol Endocrinol. 6:920-926, 1992]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D_{2L} receptor is carried out according to method described in [Hayes G, Biden TJ, Selbie LA and Shine J, Structural subtypes of the dopamine D2 receptor are functionally distinct. Expression of the clone D2A and D2B subtypes in a heterologous cell line. Mol Endocrinol. 6:920-926, 1992]. In the preferable embodiment membrane species are incubated with radioligand (0,16 nM [³H] Spiperone) in the presence of investigated compounds and without them for 2 hs at 25°C in the medium consisting of 50mM Tris-HCl, pH 7.4, 1.4mM Ascorbic Acid, 0.001% BSA, 150mM NaCl. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 10 µM of Haloperidol. Spiperone is used as positive control. The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Haloperidol (10 µM).

Test results for some representatives of ligands of general formula 1 presented in Tables 6, 7, confirm their activity towards dopamine receptors D_{2L}.

**7-C**. Activity test for ligands of general formula 1 for binding to dopamine receptors D_{2S}. Screening of the disclosed compounds for potential ability to interact with dopamine receptor D_{2S} is carried out by method of radioligand binding. For this purpose sDNA of human D_{2S} receptor is expressed in CHO cells, as described in [Hayes G, Biden TJ, Selbie LA and Shine J, Structural subtypes of the dopamine D2 receptor are functionally distinct. Expression of the clone D2A and D2B subtypes in heterologous cell line. Mol Endocrinol. 6:920-926, 1992]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D_{2S} receptor is carried out according to method described in [Hayes G, Biden TJ, Selbie LA and Shine J, Structural subtypes of the dopamine D2 receptor are functionally distinct. Expression of the clone D2A and D2B subtypes in a heterologous cell line. Mol Endocrinol. 6:920-926, 1992]. In the preferable embodiment cell membranes are incubated with radioligand (0,16 nM [³H] Spiperone) in the presence of investigated compounds and without them for 2 hs at 25°C in the medium consisting of 50mM Tris-HCl, pH 7.4, 1.4mM Ascorbic Acid, 0.001% BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 10 µM of Haloperidol. Spiperone is used as positive control.

The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Haloperidol (10 µM).

Test results for some representatives of ligands of general formula **1** presented in Tables 6, 7, confirm their activity towards dopamine receptors D_{2S}.

**7-D.** Activity test for ligands of general formula 1 for binding to dopamine receptors D₃. Screening of the disclosed compounds for potential ability to interact with dopamine receptor D₃ is carried out by method of radioligand binding. For this purpose sDNA of human D₃ receptor is expressed in CHO cells, as it was described in [Sokoloff P, Giros B, Martres M-P, Bouthenet M-L and Schwartz J-C. Molecular cloning and characterization of novel dopamine receptor (D3) as target for neuroleptics. Nature. 347:146-151, 1990]. From the cells transfected in this way membrane species are prepared by homogenization of these cells in glass homogenizer with the subsequent separation of plasmatic membranes from cell nucleus, mitochondrias and cell wreckages by differential centrifugation. Determination of investigated compounds' binding to D₃ receptor is carried out according to method described in [Sokoloff P, Giros B, Martres M-P, Bouthenet M-L and Schwartz J-C. Molecular cloning and characterization of novel dopamine receptor (D3) as target for neuroleptics. Nature. 347:146-151, 1990]. In the preferable embodiment membrane species are incubated with radioligand (0,7 nM [³H] Spiperone) in the presence of investigated compounds and without them for 120 min at 37°C in the medium consisting of 50mM Tris-HCl, pH 7.4, 1.4mM Ascorbic Acid, 0.001% BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 25 µM of S(-)-Sulpiride. Spiperone is used as positive control.

The tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and S(-)-Sulpiride (25 µM).

Test results for some representatives of ligands of general formula **1** presented in Tables 6, 7, confirm their activity towards dopamine receptors D₃.

**7-E.** Activity test for ligands of general formula **1** for binding to dopamine receptors D_{4.2}. Screening of the disclosed compounds for potential ability to interact with dopamine receptor D_{4.2} is carried out by method of radioligand binding. For this purpose sDNA of human D_{4.2} receptor is expressed in CHO-K1 cells, as it was described in [Van Tol HHM, Bunzow JR, Guan H-C, Sunahara RK, Seeman P, Niznik HB and Civelli O, Cloning of the gene for a human dopamine D4 receptor with high affinity for the antipsychotic clozapine. Nature. 350:610-614, 1991]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D_{4.2} receptor is carried out according to method described in [Van Tol HHM, Wu CM, Guan H-C, Ohara K, Bunzow JR, Civelli O, Kennedy J, Seeman P, Niznik HB and Jovanovic V, Multiple dopamine D4 receptor variants in the human population. Nature. 358:149-152, 1992]. In the preferable embodiment the membrane species are incubated with radioligand (0,5 nM [³H] Spiperone) in the presence of investigated compounds and without them for 120 min at 25°C in the medium consisting of 50mM Tris-HCl, pH 7.4, 1.4mM Ascorbic Acid, 0.001 % BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane species with radioligand in the presence of 10 µM of Haloperidol.

Spiperone is used as positive control. Tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Haloperidol (10 µM).

Test results for some representatives of ligands of general formula **1** presented in Tables 6, 7, confirm their activity towards dopamine receptors D_{4.2}.

**7-F.** Activity test for ligands of general formula **1** for binding to dopamine receptors D_{4.4}. Screening of the disclosed compounds for potential ability to interact with dopamine receptor D_{4.4} is carried out by method of radioligand binding. For this purpose sDNA of human D_{4.4} receptor is expressed in CHO-K1 cells, as described in [Van Tol HHM, Bunzow JR, Guan H-C, Sunahara RK, Seeman P, Niznik HB and Civelli O, Cloning of the gene for a human dopamine D4 receptor with high affinity for the antipsychotic clozapine. Nature. 350:610-614, 1991]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D_{4.4} receptor is carried out according to method described in [Van Tol HHM, Wu CM, Guan H-C, Ohara K, Bunzow JR, Civelli O, Kennedy J, Seeman P, Niznik HB and Jovanovic V, Multiple dopamine D4 receptor variants in the human population. Nature. 358:149-152, 1992]. In the preferable embodiment the membrane species are incubated with radioligand (1.2 nM [³H] Spiperone) in the presence of investigated compounds and without them for 120 min at 25°C in the medium consisting of 50mM Tris-HCl, pH 7.4, 1.4mM Ascorbic Acid, 0.001% BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 10 µM of Haloperidol.

Spiperone is used as positive control. The tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Haloperidol (10 µM).

Test results for some representatives of ligands of general formula **1** presented in Tables 6, 7, confirm their activity towards dopamine receptors D_{4.4}.

**7-G.** Activity test for ligands of general formula **1** for binding to dopamine receptors D_{4.7}. Screening of the disclosed compounds for potential ability to interact with dopamine receptor D_{4.7} is carried out by method of radioligand binding. For this purpose sDNA of human D_{4.7} receptor is expressed in CHO-K1 cells, as described in [Van Tol HHM, Bunzow JR, Guan H-C, Sunahara RK, Seeman P, Niznik HB and Civelli O, Cloning of the gene for a human dopamine D4 receptor with high affinity for the antipsychotic clozapine. Nature. 350:610-614, 1991]. From the cells transfected in this way membrane species are prepared by homogenization of these cells in glass homogenizer with the subsequent separation of plasmatic membranes from cell nuclei, mitochondrias and cell wreckages by differential centrifugation. Determination of investigated compounds' binding to D_{4.7} receptor is carried out according to method described in [Van Tol HHM, Wu CM, Guan H-C, Ohara K, Bunzow JR, Civelli O, Kennedy J, Seeman P, Niznik HB and Jovanovic V, Multiple dopamine D4 receptor variants in the human population. Nature. 358:149-152, 1992]. In the preferable embodiment the membrane species are incubated with radioligand (1.5 nM [³H] Spiperone) in the presence of investigated compounds and without them for 120 min at 25°C in the medium consisting of 50mM Tris-HCl, pH 7.4, 1.4mM Ascorbic Acid, 0.001% BSA, 150mM NaCl. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 10 µM of Haloperidol.

Spiperone is used as positive control. The tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA* - is overall radioactivity in the presence of radioligand only, *CA* - is radioactivity in the presence of radioligand and tested compound and *NA -* is radioactivity in the presence of radioligand and Haloperidol (10 µM).

Test results for some representatives of ligands of general formula **1** presented in Tables 6, 7, confirm their activity towards dopamine receptors D_{4.7}.

**Table 6. Biological activity of 10 µM ligands of general formula 1 towards dopamine D₁, D_{2S}, D₃, D_{4.2} receptors.**

| **Ligand** | **Dopamine receptors, % inhibition** | | | |
|---|---|---|---|---|
| | D₁ | D_{2S} | D₃ | D_{4.2} |
| **1.1.2(1-2) ·2HCl** | 89 | 97 | 99 | 103 |
| **1.1.2(2-3) ·2HCl** | 70 | 77 (3 µM) | 78 (3 µM) | 71 (100 µM) |
| **1.1.2(4-2) ·2HCl** | 82 | 80 | 64 | 54 |
| **1.6.1(1) ·3HCl** | 59 | 42 | 34 | -6 |
| **1.6.1(2) ·3HCl** | 55 | 31 | 41 | -11 |
| **1.6.2(10) ·3HCl** | 88 | 33 | 25 | 5 |
| **1.6.2(11)** | 95 | 31 | 36 | 6 |
| **1.8.1(3) ·HCl** | 76 | 42 | 50 | 74 |
| **1.9(1)·1/2NDSA** | 77 | 51 | 65 | 77 |

**Table 7. Interaction efficiencies of ligands of general formula 1 with dopamine receptors.**

| **Compound** | **Receptor** | **IC₅₀,** | **Kᵢ** | **n_{H}** |
|---|---|---|---|---|
| | | **µM** | | |
| **1.9(1)·1/2NDSA** | **D₁** | 3.66 | 1.83 | 1.38 |
| **1.1.2(4-2)·2HCl** | | 4.41 | | |
| **1.9(1)·1/2NDSA** | **D_{2S}** | 3,0 | 1.08 | 0.879 |
| **1.1.2(4-2)·2HCl** | | 1.75 | 0.629 | 0.724 |
| **1.9(1)·1/2NDSA** | **D₃** | 1.36 | 0.462 | 1.04 |
| **1.1.2(1-2) ·2HCl** | | 0.256 | 0.868 | 0.797 |

**Example 8**. Activity test for ligands of general formula **1** for binding to histamine receptors.

**8-A.** Activity test for ligands of general formula **1** for binding to histamine receptor H_{1.} Screening of the disclosed compounds for potential ability to interact with histamine receptor H₁ is carried out by method of radioligand binding. For this purpose sDNA of human H1 receptor is expressed in CHO-K1 cells, as it was described in [De Backer MD, Gommeren W, Moereels H, Nobels G, Van Gompel P, Leysen JE and Luyten WH, Genomic cloning, heterologous expression and pharmacological characterization of human histamine H1 receptor. Biochem Biophys Res Comm. 197(3): 1601-1608, 1993]. From the cells transfected in this way membrane species are prepared by homogenization of these cells in glass homogenizer with subsequent separation of plasmatic membranes from cell nuclei, mitochondrias and cell wreckages by differential centrifugation. Determination of investigated compounds' binding to H₁ receptor is carried out according to method described in [De Backer MD, Gommeren W, Moereels H, Nobels G, Van Gompel P, Leysen JE and Luyten WH, Genomic cloning, heterologous expression and pharmacological characterization of a human histamine H1 receptor. Biochem Biophys Res Comm. 197(3): 1601-1608, 1993]. In the preferable embodiment the membrane preparations are incubated with radioligand (1.2 nM [³H] Pyrilamine) in the presence of investigated compounds and without them for 180 min at 25°C in the medium consisting of 50mM Tris-HCl, pH 7.4, 2mM MgCl₂, 100mM NaCl, 250mM sucrose. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 6% of the overall binding is determined by incubation of membrane species with radioligand in the presence of 1 µM of Pyrilamine.

Pyrilamine is used as positive control. Tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Pyrilamine (1 µM).

Test results for some representatives of ligands of general formula **1** presented in Tables 8, 9, confirm their activity towards histamine receptor H₁.

**8-B.** Activity test for ligands of general formula **1** for binding to histamine receptor H₂. Screening of the disclosed compounds for potential ability to interact with histamine receptor H₁ is carried out by method of radioligand binding. For this purpose sDNA of human H₂ receptor is expressed in CHO-K1 cells, as it was described in [Ruat M, Traiffort E, Bouthenet ML, Schwartz JC, Hirschfeld J, Buschauer A and Schunack W, Reversible and irreversible labeling and autoradiographic localization of the cerebral histamine H2 receptor using [125I]iodinated probes. Proc Natl Acad Sci USA. 87(5): 1658-1662, 1990]. From the cells transfected in this way membrane species are prepared by homogenization of these cells in a glass homogenizer with the subsequent separation of plasmatic membranes from cell nucleus, mitochondrias and cell wreckages by differential centrifugation. Determination of investigated compounds' binding to H₂ receptor is carried out according to method described in [Ruat M, Traiffort E, Bouthenet ML, Schwartz JC, Hirschfeld J, Buschauer A and Schunack W, Reversible and irreversible labeling and autoradiographic localization of the cerebral histamine H2 receptor using [125I]iodinated probes. Proc Natl Acad Sci USA. 87(5): 1658-1662, 1990]. In the preferable embodiment the membrane species are incubated with radioligand (0.1 nM [¹²⁵I] Aminopotentidine) in the presence of investigated compounds and without them for 120 min at 25°C in the medium consisting of 50mM Phosphate Buffer, pH 7.4. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane species with radioligand in the presence of 3 µM of Tiotidine. Tiotidine is used as positive control. The tested compounds' binding to the receptor is determined by their ability to displace radioligand and is expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Tiotidine (3 µM).

Test results for some representatives of ligands of general formula **1** presented in Tables 8, 9, confirm their activity towards histamine receptor H₂.

**Table 8. Biological activity of 10 µM ligands of general formula 1 towards histamine receptors.**

| **Ligand** | **Histamine receptors, % inhibition** | |
|---|---|---|
| | **H₁** | **H₂** |
| **1.1.2(4-2)·2HCl** | 100 | 98 |
| **1.1.2(4-4)·2CH₃SO₃H** | 102 | 99 |
| **1.1.2(4-5)·1/2NDSA** | 98 | 100 |
| **1.1.2(1-2)·HCL** | 104 | 102 |
| **1.1.2(2-3)·2HCl** | 98 | 82 |
| **1.6.1(1) ·3HCl** | 100 | 48 |
| **1.6.1(2) ·3HCl** | 102 | 66 |
| **1.6.2(10) ·3HCl** | 103 | 69 |
| **1.6.2(11)** | 100 | 76 |
| **1.8.1(3) ·HCl** | 95 | 82 |
| **1.9(1)·HCl** | 97 | 97 |
| **1.9(1)·1/2NDSA** | 105 | 97 |

**Table 9. Interaction efficiencies of ligands of general formula 1 with histamine H₁ receptors.**

| **Ligand** | **IC₅₀** | **Kᵢ** | **n_{H}** |
|---|---|---|---|
| | µ**M** | | |
| **1.1.2(1-2)·HCl** | <0.01 | | |
| **1.1.2(4-2)·2HCl** | 0,00428 | 0,0016 | 1.01 |
| **1.9(1)·1/2NDSA** | 0.00397 | 0.0019 | 0.789 |

**Example 9.** Activity test for ligands of general formula **1** for binding to serotonin receptors.

**9-A.** Activity test for ligands of general formula **1** for binding to serotonin receptor 5-HT_{1A}. Screening of the disclosed compounds for potential ability to interact with serotonin receptor 5-HT_{1A} is carried out by the method of radioligand binding. For this purpose membrane species are prepared from CHO cells comprising recombinant human 5-HT_{1A} by homogenization of these cells in glass homogenizer with the subsequent separation of plasmatic membranes from cell nuclei, mitochondrias and cell wreckages by differential centrifugation. Determination of investigated compounds' binding to 5-HT_{1A} receptor is carried out according to method described in [May JA, McLaughlin MA, Sharif NA, Hellberg MR and Dean TR. Evaluation of the ocular hypotensive response of serotonin 5-HT1A and 5-HT2 receptor ligands in conscious ocular hypertensive cynomolgus monkeys. J Pharmacol Exp Ther. 306(1): 301-309, 2003]. In the preferable embodiment membrane species are incubated with radioligand (1.5 nM [³H] 8-OH-DPAT) in the presence of investigated compounds and without them for 60 min at 25°C in the medium consisting of 50mM Tris-HCl, pH 7.4, 0.1% Ascorbic Acid, 0.5 mM EDTA, 10mM MgSO₄. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 25% of the overall binding is determined by incubation of membrane species with radioligand in the presence of 10 µM of Metergoline. Metergoline is used as positive control. Tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Metergoline (10 µM).

Test results for some representatives of ligands of general formula **1** presented in Tables 10, 11, confirm their activity towards 5-HT_{1A} receptor.

**9-B.** Activity test for ligands of general formula **1** for binding to serotonin receptor 5-HT_{1B}, Screening of the disclosed compounds for potential ability to interact with serotonin receptor 5-HT_{1B} is carried out by method of radioligand binding. For this purpose membrane species are prepared from rat brains (Wistar) by homogenization of species of rat brain-cortices in glass homogenizer with the subsequent separation of plasmatic membranes from cell nuclei, mitochondrias and cell wreckages by differential centrifugation. Determination of investigated compounds' binding to 5-HT_{1B} receptor is carried out according to method described in [Hoyer D, Engel G and Kalkman HO, Characterization of the 5-HT1B recognition site in rat brain: binding studies with [125I] iodocyanopindolol. Eur J. Pharmacol. 118:1-12, 1985]. In the preferable embodiment the membrane species are incubated with radioligand (10 pM [¹²⁵I] cyanopindolol) in the presence of investigated compounds and without them for 90 min at 37°C in the medium consisting of 50mM Tris-HCl, pH 7.4, 154mM NaCl, 10 µM pargyline, 30 µM isoprenaline. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 30% of the overall binding is determined by incubation of membrane species with radioligand in the presence of 10 µM of Serotonin (5-HT). Serotonin is used as positive control.

Tested compounds' binding to the receptor is determined by their ability to displace radioligand and expressed in percent of displacement (%*I*). Values of %*I* are calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Serotonin (10 µM).

Test results for some representatives of ligands of general formula **1** presented in Tables 10, 11, confirm their activity towards 5-HT_{1B} receptor.

**9-C.** Activity test for ligands of general formula **1** for binding to serotonin receptor 5HT_{2A}. Screening of the disclosed compounds for potential ability to interact with serotonin receptor 5-HT_{2A} is carried out by method of radioligand binding. For this purpose membrane species are prepared from CHO-K1 cells expressing recombinant human 5-HT_{2A} receptor by homogenization of cell suspension in glass homogenizer with the subsequent separation of plasmatic membranes from cell nuclei, mitochondrias and cell wreckages by differential centrifugation. Determination of investigated compounds' binding to 5-HT_{2A} receptor is carried out according to method described in [Saucier C and Albert PR, Identification of an endogenous 5-hydroxytryptamine2A receptor in NIH-3T3 cells: agonist-induced down-regulation involves decreases in receptor RNA and number. J Neurochem. 68: 1998 - 2011, 1997; Bonhaus DW, Bach C, DeSouza A, Rich Salazar FH, Matsuoka BD, Zuppan P, Chan HW and Eglen RM, The pharmacology and distribution of human 5-hydroxytryptamine2B (5-HT2B) receptor gene products: comparison with 5-HT2A and 5-HT2C receptors. Br J Pharmacol. 115: 622 - 628, 1995]. In the preferable embodiment the membrane species are incubated with radioligand (0.5 nM [³H] Ketanserin) in the presence of investigated compounds and without them for 60 min at 25°C in the medium consisting of 50mM Tris-HCl, pH 7.4. After incubation samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 10% of the overall binding is determined by incubation of membrane species together with radioligand in the presence of 1 µM of Mianserin. Ketanserin is used as positive control. Tested compounds' binding to the receptor is determined by their ability to displace radioligand and expressed in percent of displacement (%*I*). Values of %*I* is calculated according to the following equation: $% I = \frac{TA - CA}{TA - NA} * 100 ,$
wherein: *TA -* is overall radioactivity in the presence of radioligand only, *CA -* is radioactivity in the presence of radioligand and tested compound and *NA -* is radioactivity in the presence of radioligand and Mianserin (10 µM).

Test results for some representatives of ligands of general formula **1** presented in Tables 10, 11, confirm their activity towards 5-HT_{2A} receptors.

**9-D.** Activity test for ligands of the general formula **1** for binding to serotonin receptor 5-HT_{2B}. Screening of the disclosed compounds for potential ability to interact with serotonin receptor 5-HT_{2A} is carried out by method of radioligand binding. For this purpose membrane species are prepared from CHO-K1 cells comprising recombinant human 5-HT_{2B} receptor by homogenization of recombinant cells in glass homogenizer with the subsequent separation of plasmatic membranes from cell nuclei, mitochondrias and cell wreckages by differential centrifugation. Determination of the investigated compounds' binding to 5-HT_{2B} receptor is carried out according to the method described in [Bonhaus DW, Bach C, DeSouza A, Salazar FHR, Matsuoka BD, Zuppan P, Chan HW and Eglen RM, The pharmacology and distribution of human 5-hydroxytryptamine 2B (5-HT2B) receptor gene products: comparison with 5-HT2A and 5-HT2C receptors. Br J Pharmacol. 115: 622 - 628, 1995]. In the preferable embodiment the membrane species are incubated with radioligand (1.2 nM [³H] Lysergic acid diethylamide) in the presence of investigated compounds and without them for 60 min at 37°C in the medium consisting of 50mM Tris-HCl, pH 7.4, 4mM CaCl₂, 0.1 % Ascorbic Acid. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 30% of the overall binding is determined by incubation of membrane species with radioligand in the presence of 10 µM of Serotonin (5-HT). Ketanserin is used as positive control. The tested compounds' binding to the receptor is determined by their ability to displace the radioligand and is expressed in percent of displacement (%*I*). Values of %*I* are calculated according to the following equation: $% I = \frac{TA - CA}{TA - NA} * 100 ,$
wherein: *TA -* is the overall radioactivity in the presence of radioligand only, *CA -* is the radioactivity in the presence of radioligand and tested compound and *NA -* is the radioactivity in the presence of radioligand and Serotonin (10 µM).

Test results for some representatives of the ligands of the general formula **1** presented in Tables 10, 11, confirm their activity towards 5-HT_{2B} receptors.

**9-E.** Activity test for ligands of the general formula **1** for binding to serotonin receptor 5-HT_{2C}. Screening of the disclosed compounds for potential ability to interact with serotonin receptor 5-HT_{2C} is carried out by method of radioligand binding. For this purpose membrane species are prepared from CHO-K1 cells expressing recombinant human 5-HT_{2C} receptor by homogenization of these cells in glass homogenizer with the subsequent separation of plasmatic membranes from cell nuclei, mitochondrias and cell wreckages by differential centrifugation. Determination of the investigated compounds' binding with 5-HT_{2C} receptor is carried out according to the method described in [Wolf WA and Schutz JS., The serotonin 5-HT2C receptor is a prominent serotonin receptor in basal ganglia: evidence from functional studies on serotonin-mediated phosphoinositide hydrolysis. J Neurochem. 69: 1449 - 1458, 1997]. In the preferable embodiment the membrane species are incubated with radioligand (1.0 nM [³H] Mesulergine) in the presence of investigated compounds and without them for 60 min at 25°C in the medium consisting of 50mM Tris-HCl, pH 7.4, 0.1 % Ascorbic Acid, 10 µM Pargyline. After incubation the samples are filtered *in vacuo* on glass-microfiber filters G/F (Millipor, USA), filters are washed three times with cold solution of the medium and radioactivity is measured by scintillation counter MicroBeta 340 (Perkin Elmer, USA). Nonspecific binding which makes up 30% of the overall binding is determined by incubation of membrane species with radioligand in the presence of 1 µM Mianserin. SB242084 is used as positive control. The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is the overall radioactivity in the presence of radioligand only, *CA -* is the radioactivity in the presence of radioligand and tested compound and *NA -* is a radioactivity in the presence of radioligand and Mianserin (1 µM).

Test results for some representatives of the ligands of general formula **1** presented in Tables 10, 11, confirm their activity towards 5-HT_{2C} receptors.

**9-F.** Activity test for ligands of the general formula 1 for binding to serotonin receptor 5-HT₆. Screening of the disclosed compounds for potential ability to interact with serotonin receptor 5-HT₆ is carried out by method of radioligand binding. For this purpose membrane species are prepared from HeLa cells expressing recombinant human 5-HT₆ receptor by homogenization of them in glass homogenizer with the subsequent separation of plasmatic membranes from cell nucli, mitochondrias and cell wreckages by differential centrifugation. Determination of investigated compounds' binding to 5-HT₆ receptor is carried out according to the method described in [Monsma FJ Jr, Shen Y, Ward RP, Hamblin MW and Sibley DR, Cloning and expression of a novel serotonin receptor with high affinity for tricyclic psychotropic drugs. Mol Pharmacol. 43:320-327, 1993]. In the preferable embodiment membrane species are incubated with radioligand (1.5 nM [³H] Lysergic acid diethylamide) in the presence of investigated compounds and without them for 120 min at 37°C in the medium consisting of 50mM Tris-HCl, pH 7.4, 150mM NaCl, 2mM Ascorbic Acid, 0.001% BSA. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 30% of overall binding is determined by incubation of membrane species with radioligand in the presence of 5 µM Serotonin (5-HT). Methiothepin is used as positive control. The tested compounds' binding to the receptor is determined by their ability to displace the radioligand and expressed in percent of displacemen (%*I*)t . Values of %*I* are calculated according to the following equation: $% I = \frac{TA - CA}{TA - NA} * 100 ,$
wherein: *TA -* is the overall radioactivity in the presence of radioligand only, *CA -* is the radioactivity in the presence of radioligand and tested compound and *NA -* is a radioactivity in the presence of radioligand and Serotonin (5 µM).

Test results for some representatives of the ligands of the general formula **1** presented in Tables 10, 11, confirm their activity towards 5-HT₆ receptors.

**9-G.** Activity test for ligands of general formula **1** for binding to serotonin receptor 5-HT₇ Screening of the disclosed compounds for potential ability to interact with serotonin receptor 5-HT₇ is carried out by method of radioligand binding. For this purpose sDNA of human 5-HT₇ receptor is expressed in CHO cells as it was described in [Shen Y, Monsma FJ Jr, Metcalf MA, Jose PA, Hamblin MW, and Sibley DR. Molecular cloning and expression of a 5-hydroxytryptamine 7 serotonin receptor subtype. J. Biol. Chem. 1993, 268, 18200 - 18204]. From the cells transfected in this way membrane species are prepared by their homogenization in glass homogenizer with the subsequent separation of plasmatic membranes from cell nuclei, mitochondrias and cell wreckages by differential centrifugation. Determination of investigated compounds' binding to 5-HT₇ receptor is carried out according to method described in [Roth BL, Craigo SC, Choudhary MS, Uluer S, Monsma Jr FJ, Shen Y, Meltzer HY and Sibley DR. Binding of typical and atypical antipsychotic agents to 5-hydroxytryptamine-6 and 5-hydroxytryptamine-7 receptors. J. Pharmacol. Exp. Ther. 1994, 268:1403-1410]. In the preferable embodiment the membrane species are incubated with radioligand (5.5 nM [³H] Lysergic acid diethylamide) in the presence of investigated compounds and without them for 2 hs at 25°C in the medium consisting of 50mM Tris-HCl, pH 7.4, 10mM MgCl₂, 0.5mM EDTA. After incubation the samples are filered *in vacuo* on glass-microfiber filters G/F (Millipor, USA), filters are washed three times with cold solution of medium and radioactivity is measured by scintillation counter MicroBeta 340 (PerkinElmer, USA). Nonspecific binding which makes up 10% of overall binding is determined by incubation of membrane species with radioligand in the presence of 10 µM Serotonin. Methiothepin is used as positive control.

The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is the overall radioactivity in the presence of radioligand only, *CA -* is the radioactivity in the presence of radioligand and tested compound and *NA -* is a radioactivity in the presence of radioligand and Serotonin (10 µM).

Test results for some representatives of ligands of general formula 1 presented in Tables 10, 11, confirm their activity towards serotonin receptors.

**Table 10. Biological activity of 10 µM ligands of the general formula 1 towards serotonin receptors.**

| **Ligand** | 5-HT_{1A} | 5-HT_{1B} | 5-HT_{2A} | 5-HT_{2B} | 5-HT_{2C} | 5-HT₆ | 5-HT₇ |
|---|---|---|---|---|---|---|---|
| | % inhibition | | | | | | |
| **1.1(2)** | 30 | 6 | 99 | 92 | 100 | 95 | 59 |
| **1.1(4)** | 38 | 26 | 99 | 86 | 99 | 97 | 93 |
| **1.1(6)** | 93 | 86 | 100 | 98 | 101 | 104 | 98 |
| **1.1.2(1-2).HCl** | 84 | 82 | 101 | 100 | 101 | 101 | 102 |
| **1.1.2(1-3)** | 68 | 59 | 98 | 95 | 98 | 104 | 98 |
| **1.1.2(1-5)·HCl** | 15 | 6 | 87 | 87 | 94 | 97 | 90 |
| **1.1.2(1-6)·HCl** | 16 | 25 | 99 | 85 | 100 | 98 | 96 |
| **1.1.2(1-7)·HCl** | 25 | 13 | 91 | 89 | 92 | 104 | 94 |
| **1.1.2(1-9)** | -2 | 5 | 97 | 50 | 89 | 89 | 84 |
| **1.1.2(1-10)** | 24 | 41 | 97 | 79 | 96 | 102 | 96 |
| **1.1.2(1-14)·HCl** | 57 | 52 | 101 | 97 | 97 | 108 | 100 |
| **1.1.2(1-16)·HCl** | 37 | 36 | 99 | 91 | 99 | 103 | 95 |
| **1.1.2(1-18)·HCl** | 53 | 45 | 100 | 87 | 99 | 94 | 103 |
| **1.1.2(2-3)·2HCl*** | 53 | 65 | 101 | 89 | 96 | 97 | 100 |
| **1.1.2(4-2)·2HCl** | 9 | 19 | 77 | 47 | 76 | 90 | 96 |
| **1.1.2(5-2)·2HCl** | 17 | 14 | 88 | 80 | 91 | 91 | 98 |
| **1.2.2(1-2)·HCl** | 20 | 15 | 89 | 84 | 96 | 98 | 97 |
| **1.2.2(2-2)** | -5 | 6 | 46 | 10 | 51 | 21 | 74 |
| **1.2.2(2-2)*** | 28 | | | | 94 | 88 | |
| **1.2.2(3-2)·2HCl*** | 6 | | | | 90 | 79 | |
| **1.2.2(4-2)·2HCl** | 31* | 60 | | | 78* | 84* | |
| **1.2.2(5-2)*** | 13 | | | | 92 | 84 | |
| **1.3.3(2)·HCl** | 24 | 31 | 99 | 95 | 98 | 103 | 97 |
| **1.3.3(8)·HCl** | -4 | 12 | 97 | 96 | 97 | 93 | 83 |
| **1.6.1(1)·3HCl *** | 37 | | | | 95 | 84 | |
| **1.6.1(2)·3HCl** | -9 | -6 | -4 | -2 | 4 | -10 | 8 |
| **1.6.1(2)3HCl *** | 32 | | | | 91 | 76 | |
| **1.6.2(10)·3HCl *** | 37 | | | | 99 | 93 | |
| **1.6.2(11)*** | 55 | | | | 95 | 93 | |
| **1.8.1(3)·HCl *** | | | 100 | 101 | 90 | 93 | 99 |
| **1.9(1)·HCl** | | | 68 | 52 | 79 | 79 | 76 |
| | | | (0.1µM) | (0.1µM) | (0.1µM) | (1µM) | (0.3µM) |
| **1.9(1)·1/2NDSA** | 25 | 4 | | 77 | 73 | 78 | |
| | (0.3µM) | (0.3µM) | | (0.1µM) | (0.03µM) | (0.3µM) | |
| **1.9(1)·HCl** | 32 | 13 | 96 | 99 | 97 | 71 | 91 |
| **1.9(3)·HCl** | 37 | 12 | 97 | 95 | 100 | 89 | 81 |
| **1.10(4)·HCl** | 19 | 31 | 91 | 70 | 98 | 69 | 75 |

| | | | | | | | |
|---|---|---|---|---|---|---|---|
| * ligand concentration 1 µM | | | | | | | |

**Table 11. Interaction efficiencies of ligans of general formula 1 to 5-HT receptors.**

| **Ligand** | **5-HT** | **IC₅₀,** | **Kᵢ** | **n_{H}** |
|---|---|---|---|---|
| | | **nM** | | |
| **1.1.2(1-2)·HCl** | 5-HT_{2A} | 5,48 | 1,56 | 0.876 |
| **1.1.2(2-3)·2HCl** | | 61.15 | 17.6 | 1.06 |
| **1.8.1(3)·HCl** | | 22.5 | 6.43 | 1.01 |
| **1.9(1)·HCl** | | 30.1 | 8.6 | 0.765 |
| **1.1.2(1-2)·HCl** | 5-HT_{2B} | 16,6 | 10,6 | 0,868 |
| **1.1.2(2-3)·2HCl** | | 1020 | 647 | 0.913 |
| **1.8.1(3)·HCl** | | 319 | 203 | 0.784 |
| **1.9(1)·HCl** | | 106 | 67.2 | 0.836 |
| **1.9(1)·1/2NDSA** | | 31.4 | 20.0 | 0.552 |
| **1.9(1)·HCl** | 5-HT_{2C} | 33.7 | 17.7 | 1.21 |
| **1.9(1)·1/2NDSA** | | 13.5 | 7.08 | 0.987 |
| **1.1.2(1-2)·HCl** | 5-HT₆ | 6,42 | 2,98 | 1,49 |
| **1.1.2(2-3)·2HCl** | | 109 | 50.5 | 0.744 |
| **1.8.1(3)·HCl** | | 34.8 | 15.9 | 1.07 |
| **1.9(1)·HCl** | | 330 | 153 | 1.04 |
| **1.9(1)·1/2NDSA** | | 103.0 | 47.6 | 1.07 |
| **1.1.2(1-2)·HCl** | | | | |
| **1.1.2(2-3)·2HCl** | | 2.14 | 1.23 | 0.655 |
| **1.8.1(3)·HCl** | | 535 | 307 | 0.665 |
| **1.9(1)·HCl** | | 110 | 63.2 | 1.32 |

**Example 10.** Activity test for ligands of general formula **1** for binding to imidazoline receptor I₂, Screening of the disclosed compounds for potential ability to interact with imidazoline receptor I₂ is carried out by method of radioligand binding. For this purpose membrane species are prepared from rat (Wistar) brain homogenates by homogenization of them in glass homogenizer with the subsequent separation of plasmatic membranes from cell nuclei, mitochondrias and cell wreckages by differential centrifugation. Determination of investigated compounds' binding to imidazoline I₂ receptor is carried out according to the method described in [Brown CM, MacKinnon AC, McGrath JC, Spedding M and Kilpatrick AT, a2-Adrenoceptor subtypes and imidazoline-like binding in the rat brain. Br J Pharmacol. 99:803-809, 1990]. In the preferable embodiment the membrane species are incubated with radioligand (2 nM [³H] Idazoxan) in the presence of investigated compounds and without them for 30 min at 25°C in the medium consisting of 50mM Tris-HCl, pH 7.4, 0.5mM EDTA. After incubation the samples are filtered *in vacuo* on glass-microfiber filters G/F (Millipor, USA), filters are washed three times with cold solution of medium and radioactivity is measured by scintillation counter MicroBeta 340 (Perkin Elmer, USA). Nonspecific binding which makes up 15% of the overall binding is determined by incubation of membrane species with radioligand in the presence of 1 µM Idazoxan. Idazoxan is used as positive control.

The tested compounds' binding to the receptor is determined by their ability to displace the radioligand and expressed in percent of displacement (%*I*). Values of %*I* are calculated according to the following equation: $% I = \frac{TA - CA}{TA - NA} * 100 ,$
wherein: *TA -* is the overall radioactivity in the presence of radioligand only, *CA -* is the radioactivity in the presence of radioligand and tested compound and *NA -* is a radioactivity in the presence of radioligand and Idazoxan (1 µM).

Test results for some representatives of ligands of general formula **1** presented in Tables 12, 13, confirm their activity towards imidazoline I₂ receptors.

**Table 12. Biological activity of 10 µM ligands of general formula 1 towards imidazoline I₂ receptor.**

| **Ligand** | **% inhibition** | IC₅₀ I | Kᵢ | n_{H} |
|---|---|---|---|---|
| | | **nM** | | |
| **1.1.2(1-2)·HCl** | 93 | 268 | 179 | 0.804 |
| **1.1.2(1-3)** | 93 | | | |
| **1.1.2(4-2)·2HCl** | 98 | 310 | 210 | |
| **1.1.2(2-3)·2HCl** | 88 | | | |
| **1.2.2(2-2)** | 80 | | | |
| **1.2.2(3-2)·2HCl** | 63 | | | |
| **1.2.2(4-1)** | 80 | | | |
| **1.1.2(4-2)·2HCl** | 98 | | | |
| **1.2.2(5-2)** | 72 | | | |
| **1.6.1(1)·3HCl** | 80 | | | |
| **1.6.1(2)·3HCl** | 80 | | | |
| **1.6.2(10)·3HCl** | 85 | | | |
| **1.6.2(11)** | 83 | | | |
| **1.8.1(3)·HCl** | 17 | | | |
| **1.9(1)·HCl** | 75 (3 µM) | 994 | 662 | 0.983 |
| **1.9(1)·1/2NDSA** | 87(0.3 µM) | 169 | 110 | 3.31 |

**Example 11.** Preparation of medicaments in form of tablets. Mixture of 1600 mg of starch, 1600 mg of ground lactose, 400 mg of talc and 100 mg of bis-(5-benzyl-2-methyl-2,3,4,5-tetrahydro-1H-pyrido[4,3-b]indole) naphthalene-1,5-disulfonate **1.9(1)·1/2NDSA** is prepared and compressed into bar. The resultant bar is comminuted into granules and sifted through sieve to collect granules of 14-16 mesh. The granules thus obtained are shaped into tablets of suitable form weighing 560mg each. Similarly, according to the invention, pharmaceutical compositions are produced in shape of tablets comprising other ligands of general formula **1** as active ingredient.

**Example 12.** Preparation of medicaments in form of capsules. Carefully mix bis-(5-benzyl-2-methyl-2,3,4,5-tetrahydro-1H-pyrido[4,3-b]indole) naphthalene-1,5-disulfonate **1.9(1)·1/2NDSA** with lactose powder in ratio 2 : 1. The resultant powdery mixture is packed into gelatin capsules of suitable size 300mg to a capsule.

**Example 13.** Injectable compositions for intramuscular, intraperitoneal or subcutaneous injections may be prepared by mixing 500mg of active ingredient with proper solubility, for example, bis-(5-benzyl-2-methyl-2,3,4,5-tetrahydro-1H-pyrido[4,3-b]indole) naphthalene-1,5-disulfonate **1.9(1)·1/2NDSA** with 300mg of chlorobutanol, 2ml of propylene glycol, and 100ml of injectable water.The resultant solution is filtered and placed into 1ml ampoules, and the ampoules are sealed and sterilized in autoclave.

**Example 14.** The nootropic action of active ingredient **1.9(1)·1/2NDSA** (enhancement of memory disturbed by Scopolamine) in test "Passive Avoidance of mice in the Shuttle Chamber". A shuttle chamber (Ugo Basile, Italy) consisted of two sections was used. The walls of one section were opaque while the other section had a transparent cover. The sections were connected through a hole which could be overlapped by a vertical door. The floor was made of transverse metal bars on which DC current impulses could be fed. Experiments were carried out in aged male mice of BALB/c line weighing 20-24 grams.

On the first day of testing 30 minutes before training mice were injected intraintestinally with physiological solution of Scopolamine (0.3 mg/kg) or Scopolamine in combination with active ingredient **1.9(1)·1/2NDSA.** Each group consisted of at least 8 animals. Animal was placed in light section, and latent period of the first entry into dark chamber was registered. Then the vertical door was closed and the animal was punished by 0.6 mA DC current for 3 seconds. After that it was taken back to its homecage. In 22-24 hours the same animal was placed again in light section of the shuttle chamber and latent period of its first entry into dark section, the total time of its stay in light section, and the number of entries into dark section, were registered. Each monitoring lasted for 5 minutes.

Testing was carried out during the day time in isolated laboratory using white noise at level of about 70 decibel above human hearing threshold.

Scopolamine causes the disturbance of training (memory loss) that results in increased latent period for the first entry into dark section, longer stay in light section, and decreased number of entries into dark section.

The fact that active ingredient **1.9(1)·1/2NDSA** can improve the training that has been disturbed by scopolamine is regarded as evidence for its nootropic effect.

The data presented in figures 1, 2 and 3 confirm the nootropic action of active ingredient **1.9(1)·1/2NDSA.**

Analogous testings with other active ingredients such as **1.1(6), 1.1.2(1-2), 1.1.2(2-4), 1.1.2(4-2)** and **1.1.2(5-2)** demonstrate the ability of these compounds to improve memory.

**Example 15.** The nootropic action of active ingredient **1.9(1)·1/2NDSA** (memory enhancement disturbed by MK-801) in the test "Passive Avoidance of mice in the Shuttle Chamber". The testing was carried out as in example 14. On the first day of the testing 30 minutes before training the mice were injected intraintestinally with physiological solution of MK-801 (0.1 mg/kg) or Scopolamine in combination with active ingredient **1.9(1)·1/2NDSA.** Concurrently, physiological solution of MK-801 in combination with active ingredient **1.9 (1) 1/2NDSA** was injected intraintestinally to independent group of mice before training.

The results obtained (fig. 4-6) testify the ability of active ingredient **1.9(1)·1/2NDSA** to act as nootropic.

**Example 16.** Anxiolytic (tranquilizing) action of active ingredient **1.9(1)·1/2NDSA** in the test "Mice Behavior in the Elevated Plus Maze". The length of each arm of the maze was 30cm, width is 5cm, and height of the walls was 15cm. Two opposite arms were closed from both sides and end faces by transparent walls, the other two arms were lit and opened. A mouse was placed in the center of the maze and for the next five minutes the number of entries into opened and closed arms and the time spent in each type of arms was registered. These data were used to calculate the preference indexes for opened arms as ratio of number of the opened arm entries, as well as the total time spent there to the whole number of entries to all arms and the total time spent there. The animals usually avoided opened arms (the preference index is between 0.2 and 0.3). Compounds with tranquilizing action increased this index up to 0.5-0.6 or even more and reduced the number of defecations without changing the overall motion activity of the mice (the total number of their entries to the arms).

The results obtained testify (fig. 7 - 9) that active ingredient **1.9(1)·1/2NDSA** exhibits anxiolytic (tranquilizing) action which is comparable with the activity of Buspirone and Lorasepam.

Some other active ingredients of general formula **1,** for example, active ingredient **1.1.2(4-2)** in doses of 0,05 and 0,1 mg/kg exhibit analogous action.

**Example 17.** Mice Training in Morris Water Maze. A round pool which was filled with water at 20-22°C was used. A round ceramic platform of 14cm height was placed in the pool. Animals's behavior was registered with automated computer videosystem in combination with software package of movement analysis Any-maze (Stoelting Co., US). Before testing mice suitable for training were selected. That was done by placing the platform 1cm above the water level and putting an animal on the platform for 20 seconds. Then the mouse was sunk into the water on the opposite side of the pool, allowed to find the platform and climbed it whithin 60 seconds, where it was left for additional 20 seconds. After that the mouse was repeatedly immersed into water on the opposite side of the pool and allowed to look for platform. If it failed in finding the platform within 60 seconds the experimentator helped it to find the platform and climb it. If the mice could not find the platform itself in two consecutive attempts it was excluded from experiment.

During the next two days the platform was placed 0.5 centimeter lower the water level. Every day the mice were given four attempts for finding the platform within 60 seconds. The time interval between the attempts was 20 seconds, during which the mice stayed on the platform. Every day before the first attempt mouse was placed on the platform for 20 seconds. The time needed for finding and climbing the platform was registered. The animals were sunk in water in 3 different places on the side of the pool opposite to platform. On each day of two-day testing 35-40 minutes before the beginning of training the mice were injected intraintestinally with physiological solution, Scopolamine or Scopolamine in combination with investigated compound. At least 8 animals were used in each group.

On the third day the platform was removed and each animal was placed one time in the pool for a period of 60 seconds. The time each mouse spent in the area where the platform had been located during the previous days was registered. This time interval was regarded as a measure of effectiveness of training fulfilled during the previous two days. The testing was carried out during the day time in isolated laboratory using "white noise" at level of about 70 decibel above the human hearing threshold.

Figures 10 - 13 show test results for active ingredients **1.1.2(4-2), 1.1.2(2-3), 1.1.2(1-2)** and **1.2.2(5-3)** respectively, giving evidence to decreasing memory disorders coused by Scopalomine in mice after unitary introduction of active ingredient. This testifies their therapeutic actions at neurodegenerative diseases and cognitive disorders including Alzheimer's disease.

**Example 18.** Antipsychotic activity of ligands of general formula **1.**

Test of prepulse inhibition of the startle response. Male mice of SHK line were used in the testing. The experiments were carried out during the light period of animal's diurnal. Apomorphine hydrochloride and Haloperidol were received from Sigma Chemicals Company, (USA). Apomorphine hydrochloride was dissolved in 0,1% solution of ascorbic acid prepared with sterilized water. It was introduced subcutaneously (20 mg/kg, volume of injection was 1 ml/kg) 15 minutes before the test. Haloperidol was dissolved in sterilized water using emulsifier Twin 80 and introduced intraintestinally (1 mg/kg, the volume of injection was 10 ml/kg) 1 hour before the test. Compound **1.1.2(4-2)** (CD-008-0307) was dissolved in sterilized water and introduced intraintestinally (0.2 mg/kg and 1 mg/kg, the volume of injection is 10 ml/kg) 1 hour before the test. 0.1% Solution of ascorbic acid prepared with sterilized water and Twin 80 was introduced to control group of animals. The test instrument consisted of a chamber made of transparent plexiglass (manufacturer - Columbia Instruments Company, USA), located on the platform which was placed inside sound insulating chamber. 2Cm away from the platform there was high frequency sound colomn, by means of which acoustic stimuluses were transmitted. Startle of the animal resulted in vibrations of the platform, which were detected by analog converter and registered by computer. Background noise with 65 dB was used. Each animal received 4 stimuli of testing (pulse) stimulus of 20 ms duration and 105 dB or preluse stimulus of 20 ms duration and 85 dB, after which pulse stimulus of 20 ms duration and 105 dB followed in 50 ms. Time interval between repeated pulse or prepulse in combination with pulse stimuli made up 10s. Reduction in the startle in reply to prepulse-plus-pulse stimulus was calculated in percentage in relation to amplitude of startle in response to isolated pulse stimulus.The data obtained show that in mice in normal state prepulse reduction of jerking of 40% was observed. Administration of Apomorphine used in experiments on animals for modelling of psychoto-like conditions caused reduction of prepulse inhibition of startle that reflected the lowering of CNS ability to filter sensory stimulus. Haloperidol (1mg/kg) and compound **1.1.2(4-2)** (CD-008-0307) (0,2 mg/kg and 1 mg/kg) prevented from disturbance of prepulse inhibition of startle at administration of Apomorphine. The results testify that compound **1.1.2(4-2)** in doses of 0.2 mg/kg and 1 mg/kg exhibits antipsychotic properties analogous to those of Haloperidol (fig. 14).

**Example 19.** Mice Behavior in Porsolt's Forced Swim Test. A plastic vessel filled with water at 20-22°C to height of 18cam was used. Each animal was placed in water and the time of motionless hanging in water was registered during 15 minutes - so named behavior of "despair" which was the measure of depressively-like condition. Automated computerized detection of motion with videosystem and Any-maze programm were used in the test. After administration of ligand **1.2.2(5-1)** (Avibon) in dose of 1 mg/kg during 4 days, the time that the test animal spent without moving in water was lowered almost by a factor of 3 (fig/15), that testifies the cutting of period of depressively-like condition.

**Example 20.** Mice behavior in tail suspension test. In the test a mouse was suspended by tail with a sticky tape on the holder over horizontal surface at a height of 40 cm, and during 3 minutes the total time of complete immobility was recorded. Automated computerized detection of motion with videosystem and Any-maze programm were used. After administration of ligand **1.1.2(1-2)** (CD-008-0045) in dose of 0.05 mg/kg during 4 days the time of complete immobility of mouse was lowered almost by factor of 2.5 (fig.16), that testifies the cutting of period of depressively-like condition.

### Commercial Applicability

This invention may be used in medicine, veterinary, biochemistry.

## Claims

1. Ligands exhibiting biological activity simultaneously towards α-adrenergic, dopamine, histamine, imidazoline, serotonin receptors, which are compounds of general formula **1,** in the form of bases, geometrical isomers, racemic mixtures or individual optical isomers, and pharmaceutically acceptable salts and/or hydrates thereof, 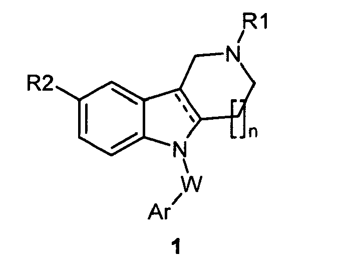 wherein:
**R¹** is selected from H, optionally substituted C₁-C₄ alkyl, acyl, heterocyclyl, alkoxycarbonyl, substituted sulfonyl;
**R²** is a substituent of cyclic system selected from hydrogen, halogen, optionally substituted C₁-C₄ alkyl, CF₃, CN, alkoxy, alkoxycarbonyl, carboxyl, aromatic heterocyclyl or substituted sulfonyl;
**Ar** represents optionally substituted aryl not necessarily annelated with heterocyclyl, or optionally substituted aromatic heterocyclyl;
**W** represents optionally substituted (CH₂)ₘ group, optionally substituted ethenyl group -CH=CH-, optionally substituted propenyl group -CH₂-CH=CH-, ethynyl group -C≡C-, or SO₂ group;
n = 1 or 2; and m=1, 2 or 3,
Solid line accompanied by dotted line, i.e. (---) may represent single or double bond.

2. Ligands according to claim **1** exhibiting biological activity simultaneously towards α_{1A}-, α_{1B}-,α_{1D}- and α_{2A}-adrenergic receptors.

3. Ligands according to claim **1,** exhibiting biological activity simultaneously towards dopamine D₁, D_{2S}, D₃ and D_{4.2} receptors.

4. Ligands according to claim **1**, exhibiting biological activity simultaneously towards histamine H₁ and H₂ receptors.

5. Ligands according to claim **1,** exhibiting biological activity towards imidazoline I₂ receptors.

6. Ligands according to claim **1,** exhibiting biological activity towards serotonin 5-HT₇ receptors.

7. Ligands according to claim **1,** exhibiting biological activity simultaneously towards serotonin 5-HT_{1A}, 5-HT_{1B}, 5-HT_{2A}, 5-HT_{2B}, 5-HT_{2C}, 5-HT₆ and 5-HT₇ receptors.

8. Ligands according to claims **1-7,** representing substituted 1,2,3,4-tetrahydro-1H-pyrido[4,3-b]indoles of general formula **1.1** and 1,2,3,4,5,6-hexahydroazepino[4,3-b]indoles of general formula **1.2,** and pharmaceutically acceptable salts thereof, 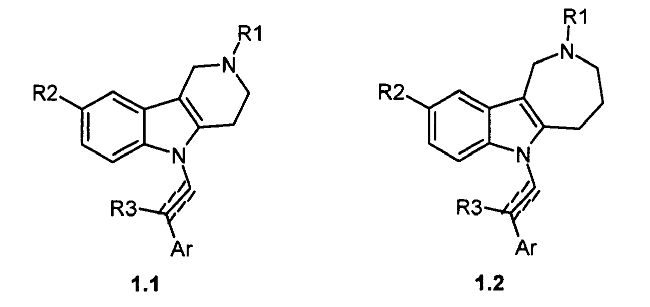
wherein: **R¹**, **R²** and **Ar** are all as defined above;
**R³** represents hydrogen atom, hydroxyl group or alkyl;
Solid line accompanied by two dotted lines, i.e. ( 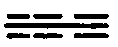 ) may represent single, double or triple bond.

9. Ligands according to claim **8,** representing substituted 1,2,3,4-tetrahydro-1H-pyrido[4,3-b]indoles of general formula **1.1.1** and 1,2,3,4,5,6-hexahydroazepino[4,3-b]indoles of general formula **1.2.1,** and pharmaceutically acceptable salts thereof, 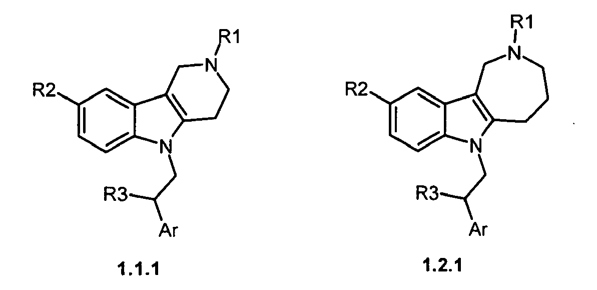 wherein: **R¹, R², R³** and **Ar** are all as defined above.

10. Ligands according to claim **9,** which are substituted 1,2,3,4-tetrahydro-1H-pyrido[4,3-b]indoles of general formula **1.1.2** and 1,2,3,4,5,6-hexahydroazepino[4,3-b]indoles of general formula **1.2.2,** and pharmaceutically acceptable salts thereof, 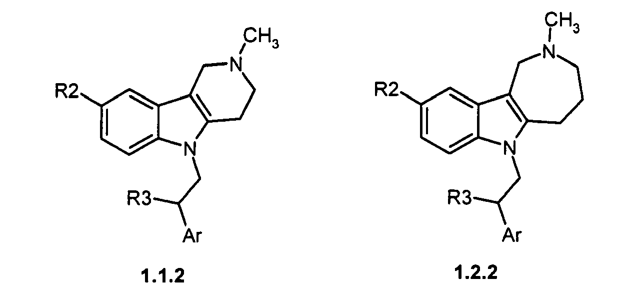 wherein: **R², R³** and **Ar** are all as defined above.

11. Ligands according to claim **10,** representing substituted 1,2,3,4-tetrahydro-1H-pyrido[4,3-b]indoles of general formulas **1.1.2(1), 1.1.2(2), 1.1.2(3), 1.1.2(4), 1.1.2(5), 1.1.2(6),** and pharmaceutically acceptable salts thereof, 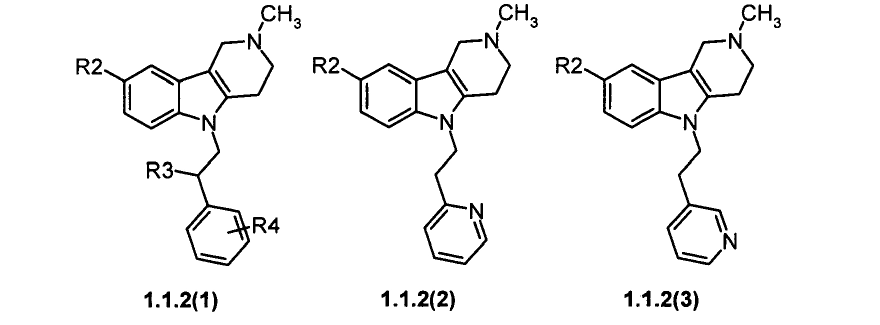 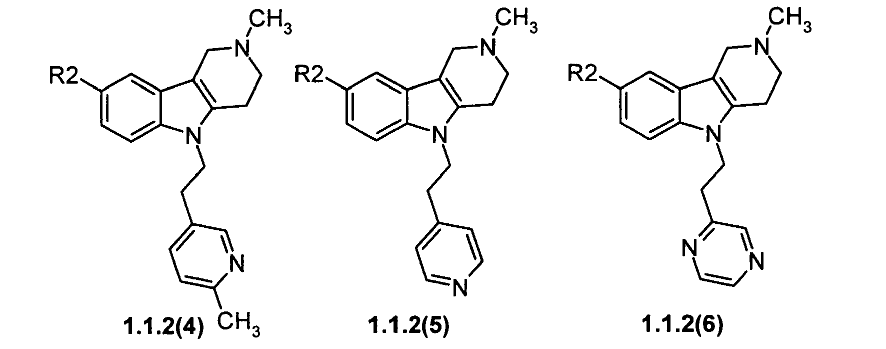
wherein: **R²** and **R³** are all as defined above,
**R⁴** represents a substituent of cyclic system selected from hydrogen, halogen, optionally substituted C₁-C₄ alkyl, optionally substituted C₁-C₄ alkoxy, CF₃, CN, and substituted amino group.

12. Ligands according to claim **11,** selected from the group consisting of:
2-methyl-5-phenethyl-1,2,3,4-tetrahydro-1H-pyrido[4,3-b]indole **1.1.2(1-1),** 2,8-dimethyl-5-phenethyl-1,2,3,4-tetrahydro-1H-pyrido[4,3-b]indole **1.1.2(1-2),**
2,8-dimethyl-5-[2-(4-methylphenyl)ethyl]-1,2,3,4-tetrahydro-1H-pyrido [4,3-b]indole**1.1.2(1-3),**
2-methyl-5-phenethyl-8-methoxy-1,2,3,4-tetrahydro-1H-pyrido[4,3-b]indole **1.1.2(1-4),**
2-methyl-5-(2-hydroxy-2-phenylethyl)-8-fluoro-1,2,3,4-tetrahydro-1H-pyrido[4,3-b]indole **1.1.2(1-6),**
2-methyl-5-phenethyl-8-trifluoromethyl-1,2,3,4-tetrahydro-1H-pyrido[4,3-b]indole **1.1.2(1-7),**
2,8-dimethyl-5-(2-phenyl-2-hydroxyethyl)-1,2,3,4-tetrahydro-1H-pyrido[4,3-b]indole **1.1.2(1-10),**
2,8-dimethyl-5-[(2-(4-dimethylaminophenyl)ethyl)-1,2,3,4-tetrahydro-1H-pyrido[4,3-b]indole **1.1.2(1-11),**
2,8-dimethyl-5-[2-(4-methoxyphenyl)ethyl]-1,2,3,4-tetrahydro-1H-pyrido[4,3-b]indole **1.1.2(1-13),**
2,8-dimethyl-5-[2-(4-fluorophenyl)ethyl]-1,2,3,4-tetrahydro-1H-pyrido[4,3-b]indole **1.1.2(1-15),**
2,8-dimethyl-5-[2-(4-trifluoromethylphenyl)ethyl]-1,2,3,4-tetrahydro-1H-pyrido[4,3-b]indole **1.1.2(1-17),**
2-methyl-5-[2-(4-methylphenyl)ethyl]-8-fluoro-1,2,3,4-tetrahydro-1H-pyrido[4,3-b]indole **1.1.2(1-20),**
2,8-dimethyl-5-[2-(pyridin-2-yl)ethyl]-1,2,3,4-tetrahydro-1H-pyrido[4,3-b]indole **1.1.2(2-2),**
2,8-dimethyl-5-[2-(pyridin-3-yl)ethyl]-1,2,3,4-tetrahydro-1H-pyrido[4,3-b]indole **1.1.2(3-2),**
2,8-dimethyl-5-[2-(4-methylpyridin-3-yl)ethyl]-1,2,3,4-tetrahydro-1H-pyrido[4,3-b]indole **1.1.2(4-1),**
2,8-dimethyl-5-[2-(pyridin-4-yl)ethyl]-1,2,3,4-tetrahydro-1H-pyrido[4,3-b]indole **1.1.2(5-1),** as free bases and/or pharmacologically acceptable salts thereof, and
2,8-dimethyl-5-[2-(4-methylpyridin-3-yl)ethyl]-1,2,3,4-tetrahydro-1H-pyrido[4,3-b]indole bis-methylsulfonate **1.1.2(4-4)**·2CH₃SO₃H and
2,8-dimethyl-5-[2-(4-methylpyridin-3-yl)ethyl]-1,2,3,4-tetrahydro-1H-pyrido[4,3-b]indole naphthalene-1,5-disulfonate **1.1.2(4-5)·**1/2NDSA

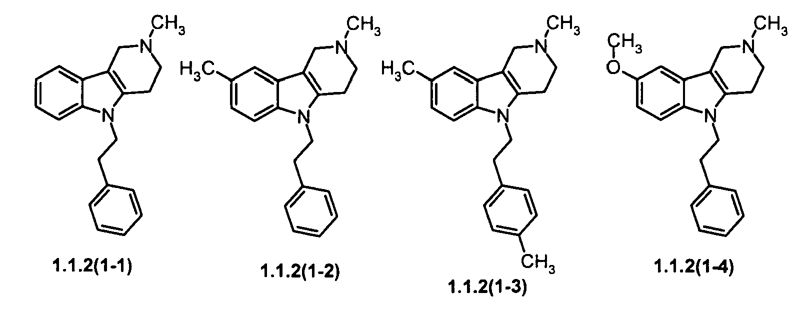

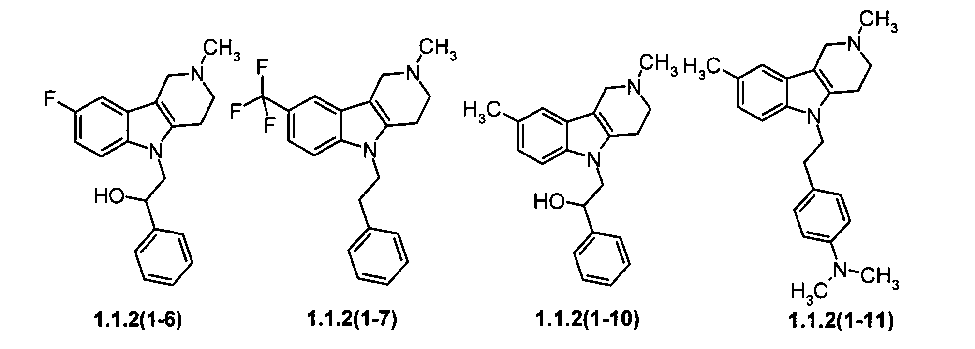

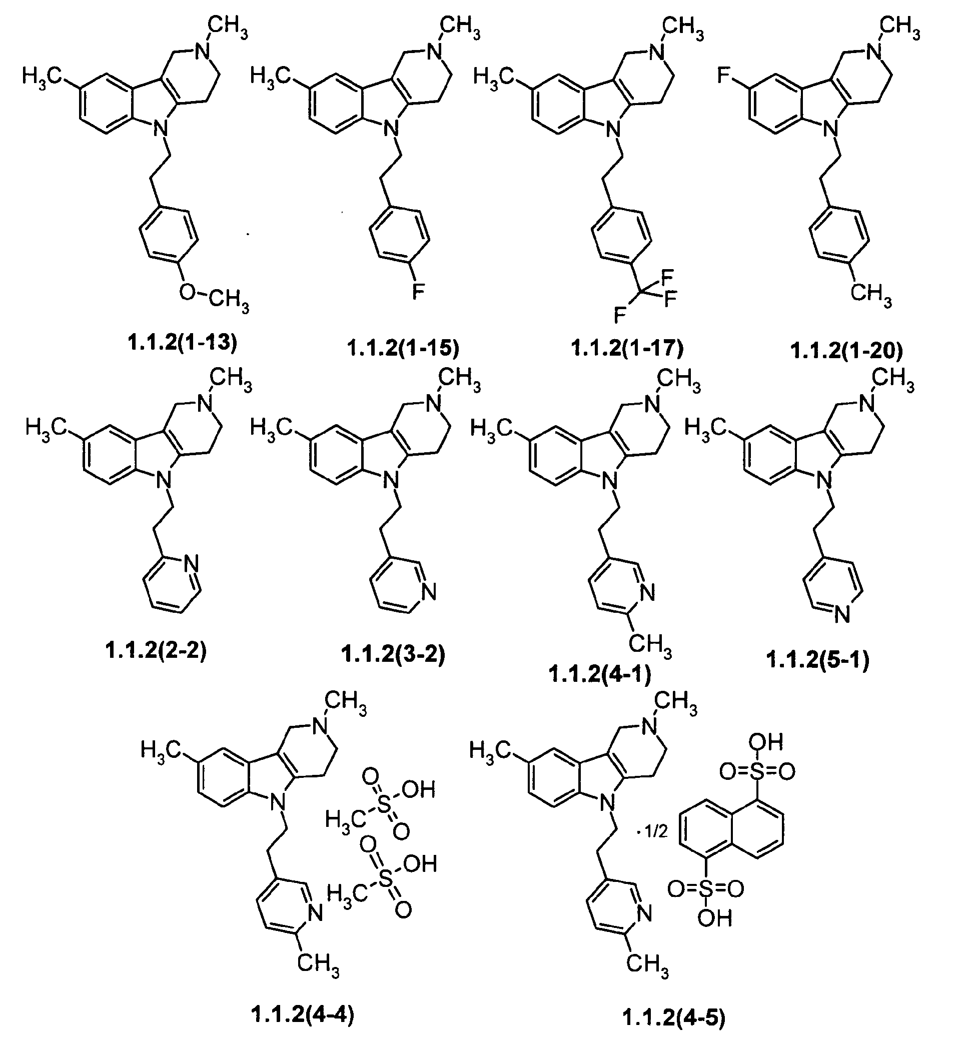

13. Ligands according to claim **10,** representing substituted 1,2,3,4,5,6-hexahydroazepino[4,3-b]indoles of general formulas **1.2.2(1), 1.2.2(2), 1.2.2(3), 1.2.2(4), 1.2.2(5), 1.2.2(6)** and pharmaceutically acceptable salts thereof, 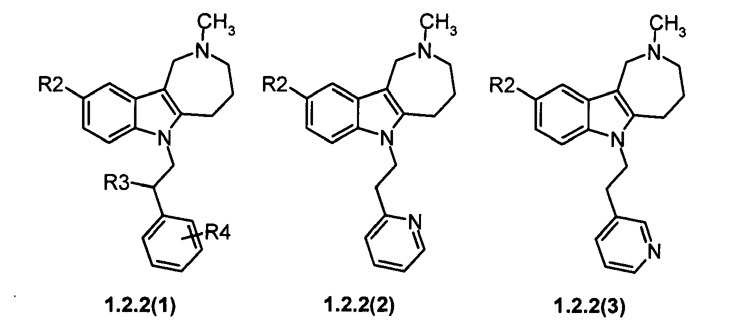 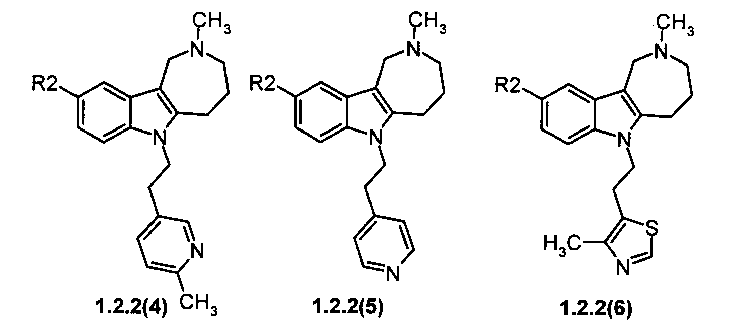 wherein: **R², R³** and **R⁴** are all as defined above.

14. Ligands according to claim **13,** which are selected from the group consisting of:
2-methyl-6-phenethyl-1,2,3,4,5,6-hexahydroazepino[4,3-b]indole **1.2.2(1-1),** 2,9-dimethyl-6-phenethyl-1,2,3,4,5,6-hexahydroazepino[4,3-b]indole **1.2.2(1-2),**
2-methyl-6-phenethyl-9-methoxy-1,2,3,4,5,6-hexahydroazepino[4,3-b]indole **1.2.2(1-4),**
2-methyl-6-phenethyl-9-fluoro-1,2,3,4,5,6-hexahydroazepino[4,3-b]indole **1.2.2(1-5),**
2-methyl-6-phenethyl-9-trifluoromethyl-1,2,3,4,5,6-hexahydroazepino[4,3-b]indole **1.2.2(1-7),**
2,9-dimethyl-6-(2-hydroxy-2-phenylethyl)-1,2,3,4,5,6-hexahydroazepino[4,3-b]indole **1.2.2(1-8)**,
2,9-dimethyl-6-[2-(4-methylphenyl)ethyl)-1,2,3,4,5,6-hexahydroazepino-[4,3-b]indole **1.2.2(1-9),**
2,9-dimethyl-6-[2-(4-methoxyphenyl)ethyl)-1,2,3,4,5,6-hexahydroazepino[4,3-b]indole **1.2.2(1-10),**
2,9-dimethyl-6-[2-(4-fluorophenyl)ethyl)-1,2,3,4,5,6-hexahydroazepino-[4,3-b]indole **1.2.2(1-12),**
2,9-dimethyl-6-[2-(4-trifluoromethylphenyl)ethyl)-1,2,3,4,5,6-hexahydroazepino[4,3-b]indole **1.2.2(1-13),**
2-methyl-6-[2-(4-methylphenyl)ethyl)-9-fluoro-1,2,3,4,5,6-hexahydroazepino-[4,3-b]indole **1.2.2(1-15),** and pharmaceutically acceptable salts thereof.

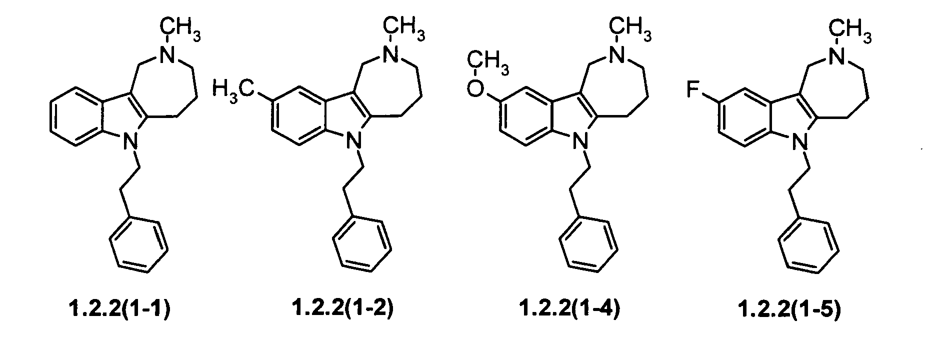

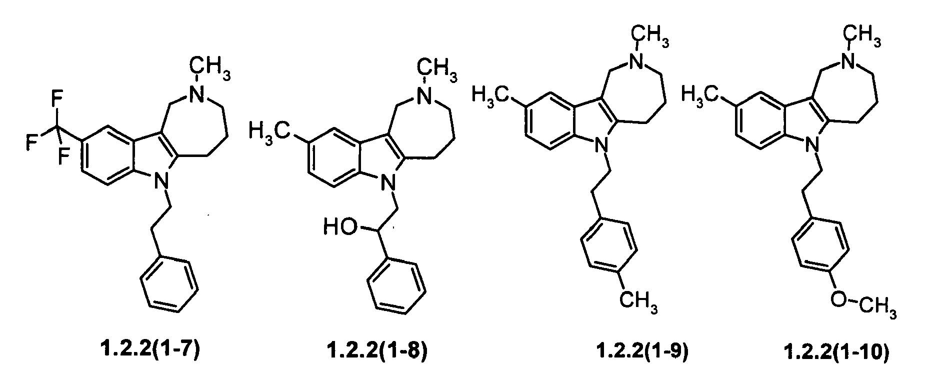

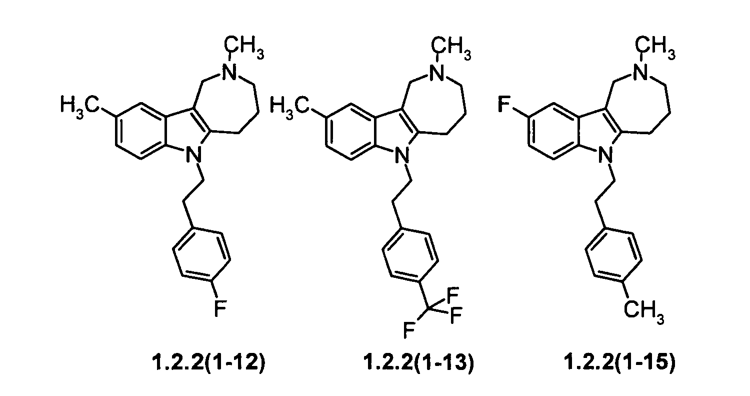

15. Ligands according to claims **1-7,** representing substituted 1,2,3,4-tetrahydro-1H-pyrido[4,3-b]indoles of general formula **1.3** and 1,2,3,4,5,6-hexahydroazepino[4,3-b]indoles of general formula **1.4,** and pharmaceutically acceptable salts thereof, 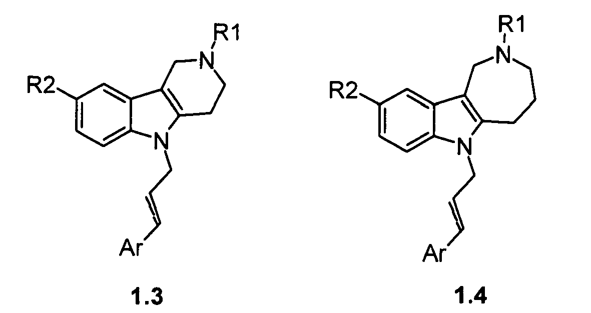 wherein: **R¹, R², Ar** and solid line accompanied by dotted line (---) are all as defined above.

16. Ligands according to claim **15,** representing substituted 1,2,3,4-tetrahydro-1H-pyrido[4,3-b]indoles of general formulas **1.3.1, 1.3.2, 1.3.3** and 1,2,3,4,5,6-hexahydroazepino[4,3-b]indoles of general formula **1.4.3,** and pharmaceutically acceptable salts thereof, 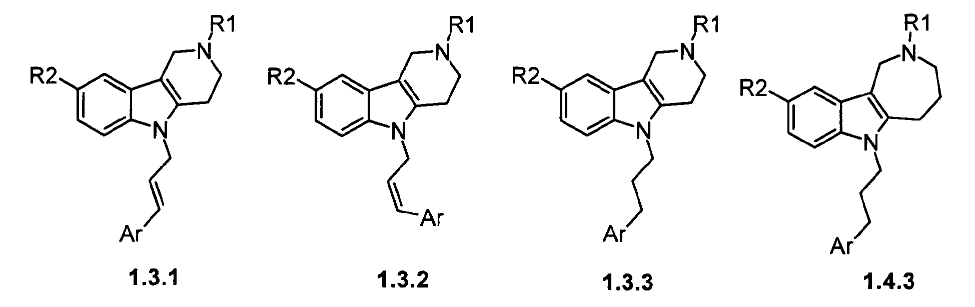 wherein: **R¹, R²,** and **Ar** are all as defined above;

17. Ligands according to claims **1-7,** representing substituted 2,3,4,4a,5,9b-hexahydro-1H-pyrido[4,3-b]indoles of general formula **1.5** and 1,2,3,4,5,5a,6,10b-octahydroazepino[4,3-b]indoles of general formula **1.6,** and pharmaceutically acceptable salts thereof, 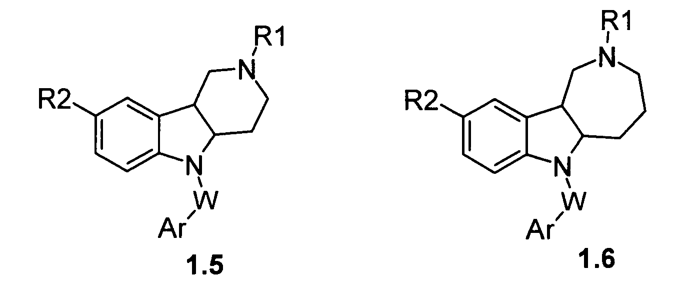 wherein: **R¹, R², Ar** and W are all as defined above;

18. Ligands according to claim **17,** representing substituted cis-2,3,4,4a,5,9b-hexahydro-1H-pyrido[4,3-b]indoles of general formula **1.5.1** and *cis*-1,2,3,4,5,5a,6,10b-octahydroazepino[4,3-b]indoles of general formula **1.6.1,** and pharmaceutically acceptable salts thereof, 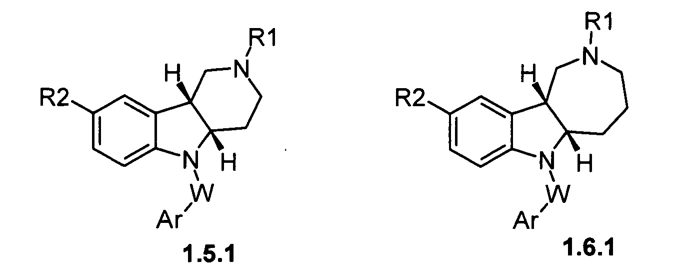 wherein: **R¹, R², Ar** and **W** are all as defined above;

19. Ligands according to claim **17,** representing substituted *trans*-2,3,4,4a,5,9b-hexahydro-1H-pyrido[4,3-b]indoles of general formula **1.5.2** and *trans-*1,2,3,4,5,5a,6,10b-octahydroazepino[4,3-b]indoles of general formula **1.6.2,** and pharmaceutically acceptable salts thereof, 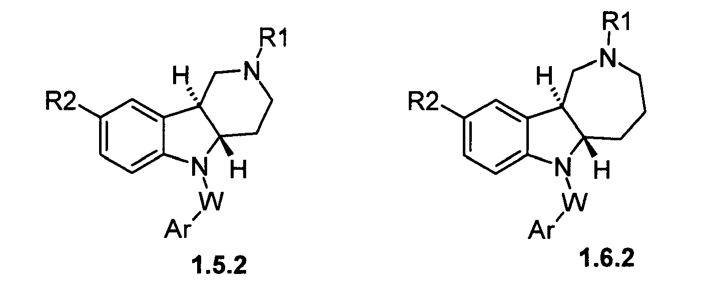 wherein: **R¹, R², Ar** and **W** are all as defined above;

20. Ligands according to claims **1-7,** representing substituted hydrogenated 1H-pyrido[4,3-b]indoles of general formula **1.7** and hydrogenated azepino[4,3-b]indoles of general formula **1.8,** and pharmaceutically acceptable salts thereof, 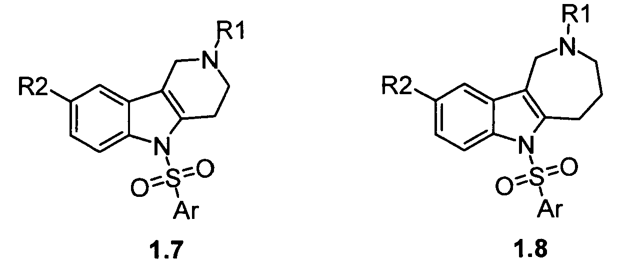 wherein **R¹, R²,** and **Ar** are all as defined above.

21. Ligands according to claims **1-7,** representing substituted hydrogenated 1H-pyrido[4,3-b]indoles of general formula **1.9** and hydrogenated azepino[4,3-b]indoles of general formula **1.10,** and pharmaceutically acceptable salts thereof, 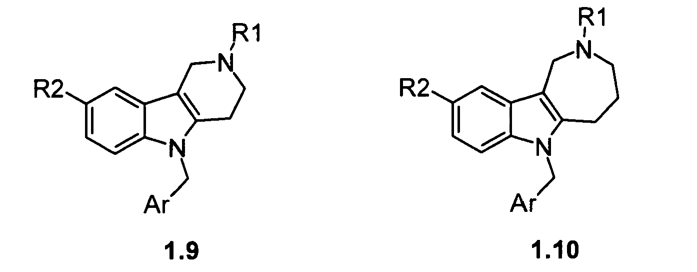 wherein: **R¹, R²,** and **Ar** are all as defined above.

22. Ligands according to claim 21, representing: 5-benzyl-2-methyl-1,2,3,4-tetrahydro-1H-pyrido[4,3-b]indole hydrochloride **1.9(1)·**HCl, 5-benzyl-2-methyl-1,2,3,4-tetrahydro-1H-pyrido[4,3-b]indole methanesulfonate **1.9(1)·**CH₃SO₃H, 5-benzyl-2-methyl-1,2,3,4-tetrahydro-1H-pyrido[4,3-b]indole naphthalene-1,5-disulfonate **1.9(1)·**1/2NDSA.

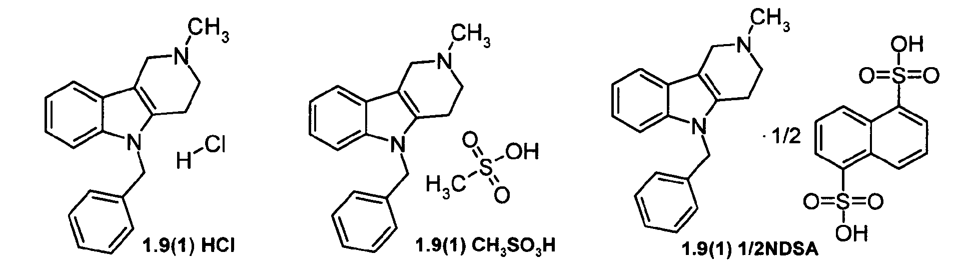

23. Active ingredient for pharmaceutical compositions and medicaments intended for treatment and prevention of CNS diseases and neurological disorders associated with hyper- or hypoactivation of α-adrenergic, dopamine, histamine, imidazoline, serotonin receptors, which is a ligand of general formulas **1, 1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10,** in the form of free bases and pharmaceutically acceptable salts, hydrates, solvates, geometrical isomers, racemic mixtures or individual optical isomers and pharmaceutically acceptable salts thereof.

24. Active ingredient according to claim 23, which is a ligand of general formulas **1.1.2(1-1), 1.1.2(1-2), 1.1.2(1-3), 1.1.2(1-4), 1.1.2(1-6), 1.1.2(1-7), 1.1.2(1-10), 1.1.2(1-11), 1.1.2(1-13), 1.1.2(1-15), 1.1.2(1-17), 1.1.2(1-20), 1.1.2(2-2), 1.1.2(3-2), 1.1.2(4-1), 1.1.2(4-4), 1.1.2(4-5), 1.1.2(5-1), 1.2.2(1-1), 1.2.2(1-2), 1.2.2(1-4), 1.2.2(1-5), 1.2.2(1-7), 1.2.2(1-8), 1.2.2(1-9), 1.2.2(1-10), 1.2.2(1-12), 1.2.2(1-13), 1.2.2(1-15), 1.9(1)** and pharmaceutically acceptable salts thereof.

25. Active ingredient according to claims **23** and **24,** selected from the group consisting of:
2,8-dimethyl-5-[2-(4-methylpyridin-3-yl)ethyl]-1,2,3,4-tetrahydro-1H-pyrido[4,3-b]indole bis-methanesulfonate **1.1.2(4-4),**
2,8-dimethyl-5-[2-(4-methylpyridin-3-yl)ethyl]-1,2,3,4-tetrahydro-1H-pyrido[4,3-b]indole naphthalene-1,5-disulfonate **1.1.2(4-5),**
5-benzyl-2-methyl-1,2,3,4-tetrahydro-1H-pyrido[4,3-b]indole hydrochloride **1.9(1)·**HCl, 5-benzyl-2-methyl-1,2,3,4-tetrahydro-1H-pyrido[4,3-b]indole methanesulfonate **1.9(1)·**CH₃SO₃H,
5-benzyl-2-methyl-1,2,3,4-tetrahydro-1H-pyrido[4,3-b]indole naphthalene-1,5-disulfonate **1.9(1)·**1/2NDSA.

26. Pharmaceutical composition with wide spectrum of receptor specific activity, including α-adrenergic, dopamine, histamine, imidazoline, serotonin receptors, comprising pharmaceutically effective amount of active ingredient of general formulas **1, 1.1, 1.1.1, 1.1.2, 1.1.2(1), 1.1.2(2), 1.1.2(3), 1.1.2(4), 1.1.2(5), 1.1.2(6), 1.2, 1.2.1, 1.2.2, 1.2.2(1), 1.2.2(2), 1.2.2(3), 1.2.2(4), 1.2.2(5), 1.2.2(6), 1.3, 1.4, 1.5, 1.5.1, 1.5.2, 1.6, 1.6.1, 1.6.2, 1.7, 1.8, 1.9, 1.10** in the form of free bases, geometrical isomers, racemic mixtures or individual optical isomers, pharmaceutically acceptable salts and/or hydrates thereof.

27. Pharmaceutical composition according to claim **26,** comprising pharmaceutically effective amount of active ingredient of general formulas **1.1.2(1-1), 1.1.2(1-2), 1.1.2(1-3), 1.1.2(1-4), 1.1.2(1-6), 1.1.2(1-7), 1.1.2(1-10), 1.1.2(1-11), 1.1.2(1-13), 1.1.2(1-15), 1.1.2(1-17), 1.1.2(1-20), 1.1.2(2-2), 1.1.2(3-2), 1.1.2(4-1), 1.1.2(4-4), 1.1.2(4-5), 1.1.2(5-1), 1.2.2(1-1), 1.2.2(1-2), 1.2.2(1-4), 1.2.2(1-5), 1.2.2(1-7), 1.2.2(1-8), 1.2.2(1-9), 1.2.2(1-10), 1.2.2(1-12), 1.2.2(1-13), 1.2.2(1-15)** and pharmaceutically acceptable salts and/or hydrates thereof.

28. Pharmaceutical composition according to claim **26,** comprising pharmaceutically effective amount of active ingredient of general formulas 1.1.2(4-4), 1.1.2(4-5), **1.9(1)·**HCl, **1.9(1)**·CH₃SO₃H,**1.9(1)·**1/2NDSA.

29. Process for preparation of pharmaceutical composition as defined in claims **26-28** which consists in mixing together of at least one active ingredient according to claims **23-25** with pharmaceutically acceptable carrier, diluents or excipient.

30. Medicament intended for treatment and prevention of CNS diseases and neurological disorders associated with hyperactivation (or hypoactivation) of alpha-adrenergic, dopamine, histamine, imidazoline and serotonin receptors, which comprises an effective amount of active ingredient according to claims **23-25** or pharmaceutical composition according to claims **26-28** in the form of tablet, capsule, intravenous, intranasal and transdermal formulation, muscular injection, syrup, suppository, aerosol, or pessary placed in pharmaceutically acceptable packing.

31. Method for treating and prophylaxis of various CNS diseases and conditions, associated with hyperactivation (or hypoactivation) of α-adrenergic, dopamine, histamine, imidazoline and serotonin receptors which consists in administering of effective amount of active ingredient according to claims **23-25** or pharmaceutical composition according to claims **26-28,** or medicament according to claim **30** to human or warm-blooded animal.

32. 1-Aryl-2-(1,2,3,4-tetrahydro-1H-pyrido[4,3-b]indol-5-yl)ethanols of general formula **1.1.1** and 1-aryl-2-(2,3,4,5-tetrahydro-1H-azepino[4,3-b]indol-5-yl)ethanols of general formula **1.2.1** and pharmaceutically acceptable salts thereof, 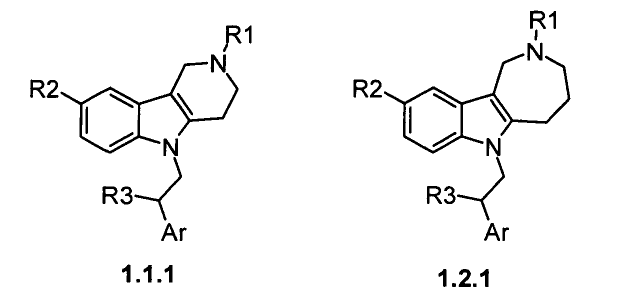 wherein: **R¹, R²,** and **Ar** are all as defined above, **R³** = OH.

33. 2,8-Dimethyl-5-[2-(4-methylpyridin-3-yl)ethyl]-1,2,3,4-tetrahydro-1H-pyrido-[4,3-b]indole bis-methylsulfonate **1.1.2(4-4)·**2CH₃SO₃H and
2,8-dimethyl-5-[2-(4-methylpyridin-3-yl)ethyl]-1,2,3,4-tetrahydro-1H-pyrido[4,3-b]indole naphthalene-1,5-disulfonate **1.1.2(4-5)·**1/2NDSA

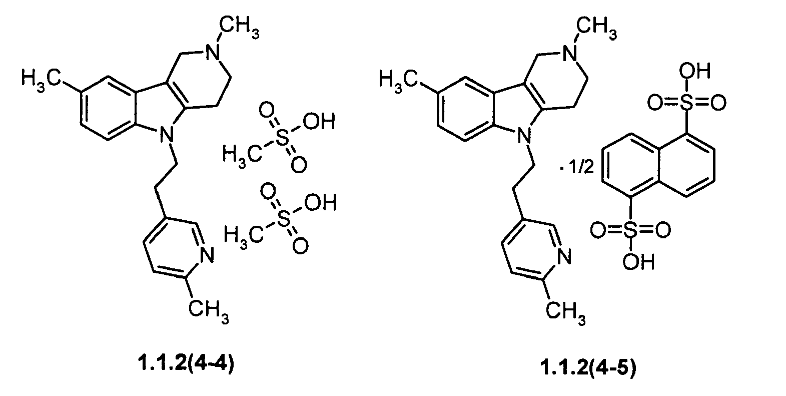

34. Substituted 1,2,3,4-tetrahydro-1H-pyrido[4,3-b]indoles of general formula **1.3** and 1,2,3,4,5,6-hexahydroazepino[4,3-b]indoles of general formula **1.4,** and pharmaceutically acceptable salts thereof, 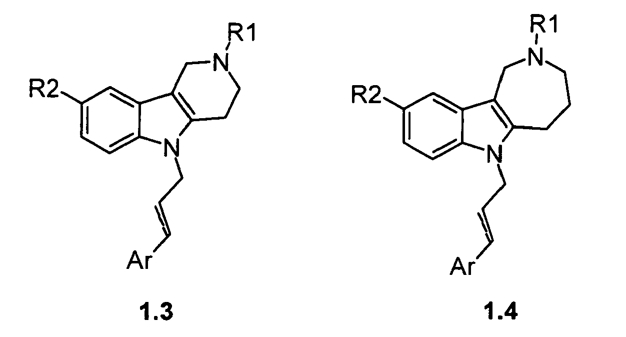 wherein: **R¹, R², Ar** and solid line accompanied by dotted line (---) are all as defined above.

35. Compounds according to claim **34,** which are substituted 1,2,3,4-tetrahydro-1H-pyrido[4,3-b]indoles of general formulas **1.3.1, 1.3.2, 1.3.3** and 1,2,3,4,5,6-hexahydroazepino[4,3-b]indoles of general formula **1.4.3,** and pharmaceutically acceptable salts thereof, 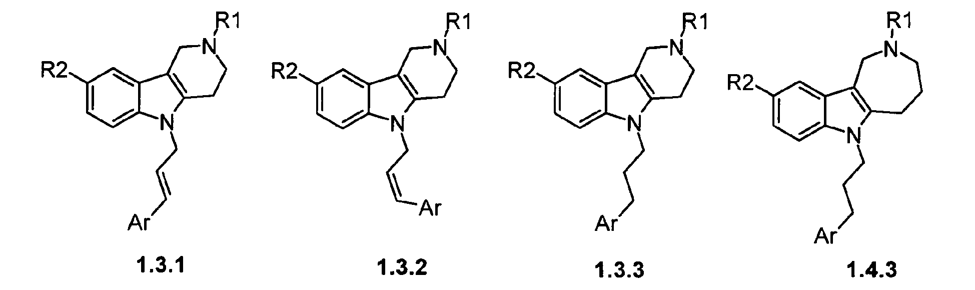 wherein: **R¹, R²,** and **Ar** are all as defined above;

36. Substituted hydrogenated azepino[4,3-b]indoles of general formula **1.8,** and pharmaceutically acceptable salts thereof, 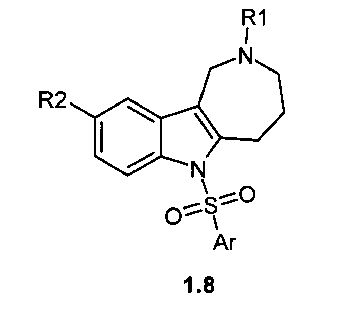 wherein: **R¹, R²,** and **Ar** are all as defined above.

37. 5-Benzyl-2-methyl-1,2,3,4-tetrahydro-1H-pyrido[4,3-b]indole methanesulfonate **1.9(1)·**CH₃SO₃H

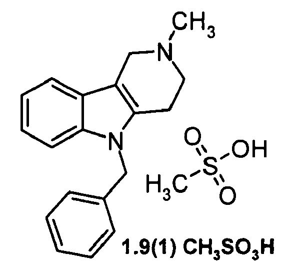

38. Method for preparation of 1-aryl-2-(1,2,3,4-tetrahydro-1H-pyrido[4,3-b]indol-5-yl)ethanols of general formula **1.1.1** and 1-aryl-2-(2,3,4,5-tetrahydro-1H-azepino[4,3-b]indol-5-yl)-ethanols of general formula **1.2.1** according to claim **32,** by interaction of compounds of formula **3** with corresponding epoxides of formula **4** in the presence of alkali, 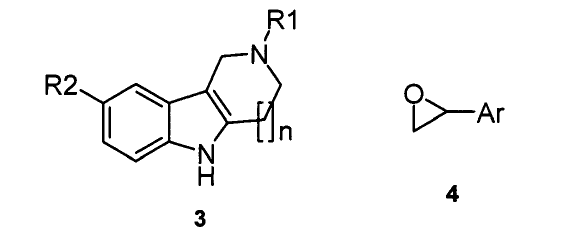 wherein: **R¹, R²,** and **Ar** are all as defined above.

39. Method for preparation of 2,8-dimethyl-5-[2-(4-methylpyridin-3-yl)ethyl]-1,2,3,4-tetrahydro-1H-pyrido[4,3-b]indole bis-methylsulfonate **1.1.2(4-4)·**2CH₃SO₃H according to claim **33,** by interaction of 2,8-dimethyl-5-[2-(4-methylpyridin-3-yl)-ethyl]-1,2,3,4-tetrahydro-1H-pyrido[4,3-b]indole of formula **1.1.2(4-1)** with methanesulfonic acid 5.

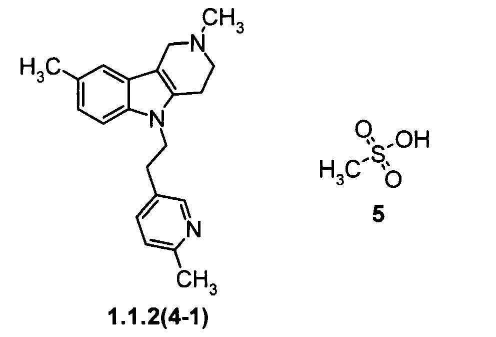

40. Method for preparation of 2,8-dimethyl-5-[2-(4-methylpyridin-3-yl)ethyl]-1,2,3,4-tetrahydro-1H-pyrido[4,3-b]indole naphthalene-1,5-disulfonate **1.1.2(4-5)·**1/2NDSA according to claim **33,** by interaction of 2,8-dimethyl-5-[2-(4-methylpyridin-3-yl)-ethyl]-1,2,3,4-tetrahydro-1H-pyrido[4,3-b]indole dihydrochloride of formula **1.1.2(4-1)**·2HCl with naphthalene-1,5-disulfonic acid **6.**

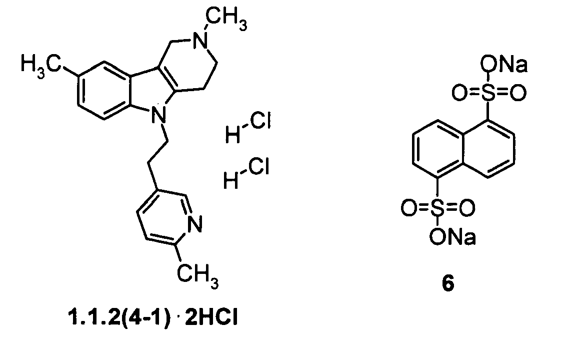

41. Method for preparation of 1,2,3,4-tetrahydro-1H-pyrido[4,3-b]indoles of general formulas **1.3.1, 1.3.2, 1.3.3** and 1,2,3,4,5,6-hexahydroazepino[4,3-b]indoles of general formulas **1.4.1, 1.4.2, 1.4.3** according to claims **34-35,** by interaction of compounds of formula **3** with alkenylchlorides **7** and subsequent reduction of obtained propylenes **1.3.1, 1.3.2, 1.4.1** and **1.4.2** that gave corresponding substituted propanes **1.3.3** and **1.4.3.** 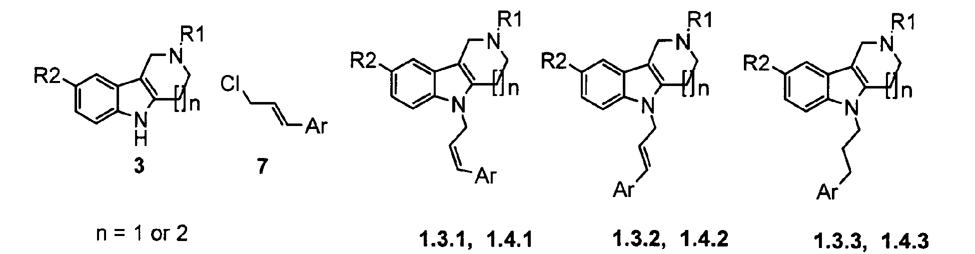 wherein: **R¹, R²,** and **Ar** are all as defined above.

42. Method for preparation of substituted hydrogenated 6-sulfonyl-azepino[4,3-b]indoles of general formula **1.8** according to claim **36,** by interaction of compounds of formula **3** with corresponding sulfonyl chlorides **8,** 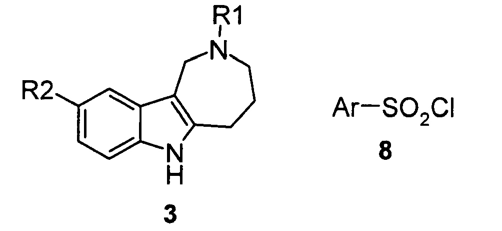 wherein: **R¹, R²,** and **Ar** are all as defined above.

43. Method for preparation of 5-benzyl-2-methyl-1,2,3,4-tetrahydro-1H-pyrido[4,3-b]indole methanesulfonate **1.9(1)·**CH₃SO₃H according to claim **37,** by interaction of 5-benzyl-2-methyl-1,2,3,4-tetrahydro-1H-pyrido[4,3-b]indole **1.9(1)·**with methanesulfonic acid **5.**
